(19)

**Europäisches Patentamt**
**European Patent Office**
**Office européen des brevets**

(11) **EP 4 244 252 B1**

(12) **EUROPEAN PATENT SPECIFICATION**

(45) Date of publication and mention
of the grant of the patent:
**29.05.2024 Bulletin 2024/22**

(21) Application number: **22769624.2**

(22) Date of filing: **26.08.2022**

(51) International Patent Classification (IPC):
**C07K 16/24** *(2006.01)* **A61K 39/00** *(2006.01)*
**A61P 37/06** *(2006.01)*

(52) Cooperative Patent Classification (CPC):
**C07K 16/244; A61P 37/06;** A61K 2039/545;
C07K 2317/21

(86) International application number:
**PCT/EP2022/073766**

(87) International publication number:
**WO 2023/025932 (02.03.2023 Gazette 2023/09)**

(54) **TREATMENT OF CHRONIC OBSTRUCTIVE PULMONARY DISEASE WITH AN ANTI-INTERLEUKIN-33 ANTIBODY**

BEHANDLUNG VON CHRONISCH OBSTRUKTIVER LUNGENERKRANKUNG MIT EINEM ANTI-INTERLEUKIN-33-ANTIKÖRPER

TRAITEMENT DE LA BRONCHO-PNEUMOPATHIE CHRONIQUE OBSTRUCTIVE AVEC UN ANTICORPS ANTI-INTERLEUKINE-33

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **27.08.2021 US 202163237630 P**
**16.05.2022 US 202263364734 P**

(43) Date of publication of application:
**20.09.2023 Bulletin 2023/38**

(73) Proprietor: **MedImmune Limited**
**Cambridge CB2 0AA (GB)**

(72) Inventors:
• **SADIQ, Muhammad Waqas**
**151 85 Södertälje (SE)**
• **LOZANO, Eulalia Jimenez**
**28033 Madrid (ES)**

(74) Representative: **AstraZeneca Intellectual Property**
**Eastbrook House**
**Shaftesbury Road**
**Cambridge CB2 8BF (GB)**

(56) References cited:
**WO-A1-2016/156440    WO-A1-2021/089563**
**WO-A1-2021/183849    WO-A1-2021/228760**

• **CHUAN QIU ET AL: "Anti-interleukin-33 inhibits cigarette smoke-induced lung inflammation in mice", CANCER RESEARCH, WILEY-BLACKWELL PUBLISHING LTD, GB, vol. 138, no. 1, 13 December 2012 (2012-12-13), pages 76-82, XP071276272, ISSN: 0019-2805, DOI: 10.1111/IMM.12020**
• **ALLINNE JEANNE ET AL: "IL-33 blockade affects mediators of persistence and exacerbation in a model of chronic airway inflammation", JOURNAL OF ALLERGY AND CLINICAL IMMUNOLOGY, ELSEVIER, AMSTERDAM, NL, vol. 144, no. 6, 25 September 2019 (2019-09-25), page 1624, XP085935196, ISSN: 0091-6749, DOI: 10.1016/J.JACI.2019.08.039 [retrieved on 2019-09-25] cited in the application**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 244 252 B1

- Anonymous: "Proof-of-Concept Study to Assess the Efficacy, Safety and Tolerability of SAR440340 (Anti-IL-33 mAb) in Patients With Moderate-to-severe Chronic Obstructive Pulmonary Disease (COPD) - Full Text View - ClinicalTrials.gov", , 12 October 2020 (2020-10-12), XP055787787, Retrieved from the Internet: URL:https://clinicaltrials.gov/ct2/show/NC T03546907 [retrieved on 2021-03-19]
- AGATA GABRYELSKA ET AL: "IL-33 blockade affects mediators of persistence and exacerbation in a model of chronic airway inflammation", FRONTIERS IN IMMUNOLOGY, vol. 10, 1 January 2019 (2019-01-01), XP055724152, DOI: 10.3389/fimmu.2019.00692
- SCOTT I. ET AL: "Proof of Mechanism for Anti-Interleukin-33 Antibody Tozorakimab in a Phase 1 Study in Healthy Adults and Patients with Chronic Obstructive Pulmonary Disease", B21. BASIC, CLINICAL, AND TRANSLATIONAL COPD STUDIES: THE ONGOING HUNT FOR UNDERLYING MECHANISMS AND THERAPEUTIC TARGETS, 1 May 2022 (2022-05-01), pages A2397-A2397, XP055982451, DOI: 10.1164/ajrccm-conference.2022.205.1_Meeti ngAbstracts.A2397 Retrieved from the Internet: URL:http://dx.doi.org/10.1164/ajrccm-confe rence.2022.205.1_MeetingAbstracts.A2397>
- Anonymous: "Efficacy and Safety of Tozorakimab in Symptomatic Chronic Obstructive Pulmonary Disease With a History of Exacerbations - Full Text View - ClinicalTrials.gov", , 22 December 2021 (2021-12-22), XP055982456, Retrieved from the Internet: URL:https://clinicaltrials.gov/ct2/show/NC T05166889 [retrieved on 2022-11-17]

Remarks:

The complete document including Reference Table(s) and the Sequence Listing(s) can be downloaded from the EPO website

**Description**

**TECHNICAL FIELD**

The present disclosure relates to methods of treating COPD, particularly by administering an anti-IL-33 antibody or antibody variant thereof.

**BACKGROUND**

Chronic obstructive pulmonary disease is the fourth leading cause of death in the world and is projected to be the third leading cause of death worldwide by 2030 (Adeloye et al 2015). Chronic obstructive pulmonary disease is characterised by persistent respiratory symptoms and airflow limitation (post-bronchodilator [BD] forced expiratory volume in one second [FEV1]/forced vital capacity [FVC] < 0.70) that is due to airway and/or alveolar abnormalities, usually caused by significant exposure to noxious particles or gases, and influenced by host factors including abnormal lung development. Significant comorbidities may have an impact on morbidity and mortality (GOLD 2020). Chronic obstructive pulmonary disease is a life-threatening respiratory condition, and the disease course is characterised by a long period of disability, which results in considerable loss of health related quality of life (van Manen et al 2003).

Chronic obstructive pulmonary disease is not fully reversible, usually progressive, and associated with an enhanced chronic inflammatory response in the lung. There is an increasing evidence base demonstrating that the overall symptomatic burden has a substantial detrimental impact on health related quality of life and also contributes to increased risk of exacerbations and a worse disease prognosis (Miravitlles and Ribera 2017).

Acute exacerbations of COPD are episodes of symptom worsening that have significant adverse consequences for patients (Wedzicha and Seemungal 2007). Greater frequency of exacerbations is associated with accelerated lung function decline, health-related quality of life impairment, and increased mortality (Donaldson et al 2002, Seemungal et al 1998, Soler-Cataluna et al 2005). Furthermore, as the incidence of COPD increases, exacerbations place a greater burden on health care systems, accounting for more than 10 million unscheduled attendances per year in the United States (Mannino and Braman 2007). The direct costs of COPD treatment in the United States are greater than $32 billion per year, with exacerbations estimated to account for 50% to 75% of these healthcare costs (Celli et al 2004, Guarascio et al 2013, Toy et al 2010). Exacerbations are also important outcome measures in COPD, with acute treatment targeting accelerated recovery, whereas long-term maintenance inhaled therapy is designed to prevent and reduce their frequency and severity (Ritchie and Wedzicha 2020). WO2021/089563 relates to an IL-33 antagonist for use in the prevention or treatment of abnormal epithelium physiology or EGFR-mediated diseases, and corresponding methods of prevention or treatment comprising administering an IL-33 antagonist to a patient in need thereof.

Despite adequate treatment with optimised maintenance inhaled therapy, approximately 30% to 40% of patients continue to have moderate or severe exacerbations (Müllerová et al 2017, Vestbo et al 2017). Even maximal triple therapy (LABA + LAMA + ICS) may still be insufficient (Rabe et al 2020); hence, a substantial unmet medical need remains.

**SUMMARY OF THE DISCLOSURE**

The invention is as described in the claims. The present disclosure describes methods for treating COPD. The methods disclosed herein comprise administration of anti-IL-33 antibodies or antibody variants thereof.

Interleukin-33 expression is increased in COPD (Byers et al 2013), and inversely correlates with lung function (Byers et al 2013, Kearley et al 2015). Neutralisation of IL-33 activity with MEDI3506 has potential to disrupt the cycle of inflammatory structural damage in lungs of patients with COPD, and thereby provide therapeutic benefit to patients with COPD.

The invention provides an IL-33 antibody or an antibody variant thereof for use in a method of treating chronic obstructive pulmonary disease (COPD) in a subject, wherein the method comprises administering a therapeutically effective amount of an anti-IL-33 antibody or antibody variant thereof in a dose of about 300 mg at an interval of every 2 weeks (Q2W), 4 weeks (Q4W) or 8 weeks (Q8W), or in a dose of about 600 mg at an interval of every 4 weeks (Q4W), wherein the anti-IL-33 antibody comprises: a VH domain having the sequence set forth in SEQ ID NO: 4 and a VL domain having the sequence set forth in SEQ ID NO: 8.

Also disclosed herein is a method of treating COPD in a subject comprising administering a therapeutically effective amount of an anti-IL-33 antibody or antibody variant thereof in a dose effective to achieve at least 80% inhibition of IL-33 in the lung or epithelial lining fluid (ELF), wherein the anti-IL-33 antibody comprises: a heavy chain variable region comprising a HCDR1 having the sequence as set forth in SEQ ID NO: 1, a VHCDR2 having the sequence of SEQ ID NO: 2, a VHCDR3 having the sequence of SEQ ID NO: 3; and a light chain variable region a VLCDR1 having the sequence of SEQ ID NO: 5, a VLCDR2 having the sequence of SEQ ID NO: 6, and a VLCDR3 having the sequence of SEQ ID NO: 7.

In some instances, the dose may be effective to achieve at least about 90%, optionally at least 95%, inhibition of IL-33 in the lung.

In some instances, the dose is about 300 mg at an interval of every 4 weeks (Q4W) or 8 weeks (Q8W). In some instances, the dose is about 300 mg Q8W. In some instances, the dose is about 300 mg Q4W.

In some instances, the COPD is associated with chronic bronchitis in the subject.

In some instances, the COPD is moderate COPD, moderate-to-severe COPD or severe COPD.

In some instances, the subject to be treated has a history of at least one, optionally at least two moderate, or at least one severe, acute exacerbations of COPD (aeCOPD) in the 12 months prior to treatment.

In some instances, prior to treatment, the subject has a post bronchodilator forced expiratory volume in 1 second ($FEV_1$) to forced vital capacity (FVC) ratio (post-BD-FEVi/FVC) of less than (<) 0.70. In some instances, prior to treatment the subject has a post-BD FEV1 > 20% of predicted normal value.

In some instances, the subject is a current smoker or a former smoker. In some instances, the subject is a former smoker. In some instances, the subject has a smoking history of at least 10 pack-years.

In some instances, the subject to be treated is receiving COPD inhaled maintenance therapy comprising a long acting Beta 2 agonist (LABA), a long activating muscarinic receptor antagonist (LAMA), and/or a inhaled corticosteroid (ICS). In some instances, the inhaled maintenance therapy comprises LABA and LAMA, ICS and LABA, or ICS, LABA and LAMA.

In some instances, the anti-IL-33 antibody or antibody variant thereof is selected from: human antibody, a humanized antibody, a chimeric antibody, a monoclonal antibody, a recombinant antibody, an antigen-binding antibody fragment, a single chain antibody, a monomeric antibody, a diabody, a triabody, tetrabody, a Fab fragment, an IgG1 antibody, an IgG2 antibody, an IgG3 antibody, and an IgG4 antibody.

In some instances, the anti-IL-33 antibody or antibody variant thereof is an IgG1.

In some instances, the anti-IL-33 antibody or antibody variant thereof is a human antibody.

The anti-IL-33 antibody or antibody variant thereof comprises a VH domain at least 95%, 90% or 85% identical to the sequence set forth in SEQ ID NO: 4 and a VL domain at least 95%, 90% or 85% identical to the sequence set forth in SEQ ID NO: 8.

In some instances, the anti-IL-33 antibody comprises a VH domain sequence as set forth in SEQ ID NO:4 and a VL domain sequence as set forth in SEQ ID NO: 8.

In some instances, the anti-IL-33 antibody comprises a light chain sequence as set forth in SEQ ID NO:9 and a heavy chain sequence as set forth in SEQ ID NO: 10.

In some instances, the anti-IL-33 antibody variant has the same pharmacokinetic (pK) characteristics as 33_670087_7B (MEDI3506/tozorakimab) in humans.

In some instances, the anti-IL-33 antibody is tozorakimab.

In some instances, the administration is subcutaneous.

Disclosed herein is a method of improving a marker of chronic obstructive pulmonary disease (COPD) in a subject, comprising: administering a therapeutically effective amount of an anti-IL-33 antibody or antibody variant thereof in a dose of from about 300 to about 600 mg at an interval of every 4 weeks (Q4W) or 8 weeks (Q8W), wherein the anti-IL-33 antibody comprises: a heavy chain variable region comprising a HCDR1 having the sequence as set forth in SEQ ID NO:1, a VHCDR2 having the sequence of SEQ ID NO: 2, a VHCDR3 having the sequence of SEQ ID NO: 3; and a light chain variable region a VLCDR1 having the sequence of SEQ ID NO: 5, a VLCDR2 having the sequence of SEQ ID NO: 6, and a VLCDR3 having the sequence of SEQ ID NO: 7. The marker is selected from: annualised rate of moderate to severe COPD exacerbations, time to first moderate to severe COPD exacerbation, FEV1, forced expiratory volume in 1 second (FEV1), FEV1 to Forced Vital Capacity (FVC) ratio (FEV1/FVC), or breathlessness, cough and sputum scale (BCSS) score, COPD Assessment Test (CAT) score and St. George's respiratory Questionnaire (SGRQ) score.

In some instances, for any of the preceding aspects, the anti-IL-33 antibody or antibody variant thereof is administered for a period of at least 12 weeks. In some instances, for any of the preceding aspects, the anti-IL-33 antibody or antibody variant thereof is administered for a period of at least 24 weeks. In some instances, for any of the preceding aspects, the anti-IL-33 antibody or antibody variant thereof is administered for a period of at least 52 weeks.

Disclosed herein is a method of reducing the annualised rate of moderate to severe COPD exacerbations in a subject comprising administering a therapeutically effective amount of an anti-IL-33 antibody or antibody variant thereof in a dose of from about 300 to about 600 mg at an interval of every 4 weeks (Q4W) or 8 weeks (Q8W), wherein the anti-IL-33 antibody comprises: a heavy chain variable region comprising a HCDR1 having the sequence as set forth in SEQ ID NO: 1, a VHCDR2 having the sequence of SEQ ID NO: 2, a VHCDR3 having the sequence of SEQ ID NO: 3; and a light chain variable region a VLCDR1 having the sequence of SEQ ID NO: 5, a VLCDR2 having the sequence of SEQ ID NO: 6, and a VLCDR3 having the sequence of SEQ ID NO: 7.

Also disclosed herein is a method of improving pre-bronchodilator FEV1 in a subject with COPD comprising administering a therapeutically effective amount of an anti-IL-33 antibody or antibody variant thereof in a dose of from about 300 to about 600 mg at an interval of every 4 weeks (Q4W) or 8 weeks (Q8W), wherein the anti-IL-33 antibody comprises: a heavy chain variable region comprising a HCDR1 having the sequence as set forth in SEQ ID NO: 1, a VHCDR2 having the sequence of SEQ ID NO: 2, a VHCDR3 having the sequence of SEQ ID NO: 3; and a light chain variable region a VLCDR1 having the sequence of SEQ ID NO: 5, a VLCDR2 having the sequence of SEQ ID NO: 6, and a VLCDR3 having the sequence of SEQ ID NO: 7.

Also disclosed herein is a method of improving the E-RS: COPD score in a subject with COPD comprising administering a therapeutically effective amount of an anti-IL-33 antibody or antibody variant thereof in a dose of from about 300 to about 600 mg at an interval of every 4 weeks (Q4W) or 8 weeks (Q8W), wherein the anti-IL-33 antibody comprises: a heavy chain variable region comprising a HCDR1 having the sequence as set forth in SEQ ID NO: 1, a VHCDR2 having the sequence of SEQ ID NO: 2, a VHCDR3 having the sequence of SEQ ID NO: 3; and a light chain variable region a VLCDR1 having the sequence of SEQ ID NO: 5, a VLCDR2 having the sequence of SEQ ID NO: 6, and a VLCDR3 having the sequence of SEQ ID NO: 7.

Also disclosed herein is a method of improving the SGRQ score in a subject with COPD comprising administering a therapeutically effective amount of an anti-IL-33 antibody or antibody variant thereof in a dose of from about 300 to about 600 mg at an interval of every 4 weeks (Q4W) or 8 weeks (Q8W), wherein the anti-IL-33 antibody comprises: a heavy chain variable region comprising a HCDR1 having the sequence as set forth in SEQ ID NO:1, a VHCDR2 having the sequence of SEQ ID NO: 2, a VHCDR3 having the sequence of SEQ ID NO: 3; and a light chain variable region a VLCDR1 having the sequence of SEQ ID NO: 5, a VLCDR2 having the sequence of SEQ ID NO: 6, and a VLCDR3 having the sequence of SEQ ID NO: 7.

Also disclosed herein is a method of improving the CAT score in a subject with COPD comprising administering a therapeutically effective amount of an anti-IL-33 antibody or antibody variant thereof in a dose of from about 300 to about 600 mg at an interval of every 4 weeks (Q4W) or 8 weeks (Q8W), wherein the anti-IL-33 antibody comprises: a heavy chain variable region comprising a HCDR1 having the sequence as set forth in SEQ ID NO:1, a VHCDR2 having the sequence of SEQ ID NO: 2, a VHCDR3 having the sequence of SEQ ID NO: 3; and a light chain variable region a VLCDR1 having the sequence of SEQ ID NO: 5, a VLCDR2 having the sequence of SEQ ID NO: 6, and a VLCDR3 having the sequence of SEQ ID NO: 7.

**BRIEF DESCRIPTION OF DRAWINGS**

Figure 1A shows IL-33/tozorakimab complex amount measured in serum of healthy participants from Part I of NCT03096795

Figure 1B shows IL-33/sST2 complex amount measured in serum of healthy participants from Part I of NCT03096795

Figure 1C shows IL-33/tozorakimab complex amount measured in serum of participants with COPD from Part II of NCT03096795

Figure 1D shows IL-33/sST2 complex amount measured in serum of participants with COPD from Part II of NCT03096795

Figure 2A shows nasal mucosal lining fluid levels of free IL-33$^{red}$ plus IL-33$^{red}$/tozorakimab measured at day 29 in the 300 mg MAD cohort

Figure 2B shows nasal mucosal lining fluid levels of free IL-33$^{red}$ measured at day 29 in the 300 mg MAD cohort

Figure 2C shows nasal mucosal lining fluid levels of free IL-33$^{ox}$ measured at day 29 in the 300 mg MAD cohort

Figure 3 shows tozorakimab inhibits ex vivo IL-33 challenge in whole blood from healthy participants

Figure 4A shows serum levels of IL-5 at days 1, 14 and 28 in participants in the 300 mg MAD cohort (Placebo, n=6; tozorakimab (n=6). The plot shows mean ± SEM. A mixed-effect longitudinal model was used to generate p values comparing the trajectories of the biomarkers between tozorakimab and placebo. (p = 0.0037)

Figure 4B shows serum levels of IL-13 at days 1, 14 and 28 in participants in the 300 mg MAD cohort (Placebo, n=6; tozorakimab (n=6). The plot shows mean ± SEM. A mixed-effect longitudinal model was used to generate p values comparing the trajectories of the biomarkers between tozorakimab and placebo. (p = 0.034)

**Figure 4C** shows serum levels of eosinophils at days 1, 14 and 28 in participants in the 300 mg MAD cohort (Placebo, n=6; tozorakimab (n=6). The plot shows mean ± SEM. A mixed-effect longitudinal model was used to generate p values comparing the trajectories of the biomarkers between tozorakimab and placebo. (p = 0.0023)

**Figure 5** shows that *Alternaria alternata* induces rapid IL-33 release in the bronchoalveolar lavage fluid (BALF) in humanised IL-33 mice

**Figure 6** shows tozorakimab inhibits ALT-induced BALF IL-5 in humanized IL-33 mice. Test substances were dosed intranasally at -24 hours prior to challenge with ALT. BALF was harvested at 24 hours post ALT challenge and analysed for presence of IL-5. Significant effect of test substances was determined using one-way ANOVA with Bonferroni's multiple comparisons test. ***p<0.001, **p< 0.01 (n=4)

**Figure 7A** shows scratch wound repair in normal human bronchial epithelial cells upon treatment with wildtype IL-33 (IL-33), oxIL-33, and oxIL-33 + anti-ST2 antibody

**Figure 7B** shows quantification of the % wound closure from scratch wound assays described in Figure 3A

**Figure 8** shows scratch wound impairment was also seen in bronchial epithelial cells obtained from COPD subjects

**Figure 9** shows % scratch closure in A549 cells with increasing concentrations of tozorakimab and anti-TSLP antibody

**Figure 10** is the PK/PD target engagement model description

**Figure 11** shows the prediction of tozorakimab systemic concentrations vs the observed tozorakimab systemic concentrations from Ph1 dose cohorts

**Figure 12** shows the prediction of tozorakimab:IL-33 complex formation vs the observed tozorakimab:IL-33 complex formation from Ph1 dose cohorts

**Figure 13** shows the prediction ofIL-33:sST2 complex reduction vs the observed IL-33 :sST2 complex reduction from Ph1 dose cohorts

**Figure 14** shows the IL-33 inhibition predicted from dose response for IL33/sST2 complex inhibition in blood (Q2W - top line; Q4W - middle line; Q6W - bottom line)

**Figure 15** shows the predicted suppression of IL-33 in the lung at trough with tozorakimab (Q4W - top line; Q8W - bottom line)

**Figure 16** shows the predicted tozorakimab serum concentration following 300mg Q4W (higher line) and 300mg Q8W (lower line). The serum concentrations required for 60%, 80% and 90% identified by Alternaria mouse model are indicated by dashed lines

**Figure 17** shows the predicted tozorakimab serum concentration following 300mg Q4W (higher line) and 150mg Q4W (lower line). The serum concentration threshold for response in the scratch wound closure assay in shown as a dashed line

**Figure 18** also shows the prediction of tozorakimab systemic concentrations vs the observed tozorakimab systemic concentrations from Ph1 dose cohorts

**Figure 19** also shows the prediction of IL-33:sST2 complex reduction vs the observed IL-33:sST2 complex reduction from Ph1 dose cohorts

## DETAILED DESCRIPTION

The term "about" or "approximately" means an acceptable error for a particular value as determined by one of ordinary skill in the art, which depends in part on how the value is measured or determined. In certain embodiments, the term "about" or "approximately" means within 1, 2, 3, or 4 standard deviations. In certain embodiments, the term "about" or "approximately" means within 30%, 25%, 20%, 15%, 1 0%, 9%, 8%, 7%, 6%, 5%, 4%, 3%, 2%, 1%, 0.5%, or 0.05% of a given value or range. Whenever the term "about" or "approximately" precedes the first numerical value in a series of two or more numerical values, it is understood that the term "about" or "approximately" applies to each one of the numerical values in that series.

## *Treatment of COPD*

Chronic obstructive pulmonary disease (COPD) is a chronic inflammatory lung disease that causes obstructed airflow from the lungs. Symptoms include breathing difficulty, cough, mucus (sputum) production and wheezing.

COPD may be characterised by persistent respiratory symptoms and airflow limitation, as reported through airway spirometry, preferably post-bronchodilator (post-BD) airway spirometry. As used herein, "post-bronchodilator (post-BD) airway spirometry" refers to airway spirometry performed following the administration of a bronchodilator, typically administered via an inhaler or nebuliser. In some embodiments, the bronchodilator is selected from albuterol or salbutamol. Post-BD spirometry results may be expressed as forced expiratory volume in one second (FEV1), or forced vital capacity (FVC).

The disclosure provides for treatments of COPD. In some instances, the COPD is moderate-to-severe COPD. Moderate-to-severe COPD is generally characterised by the subject having a post-BD forced expiratory volume in one second (FEV1) < 80% of the predicted normal value, i.e. that predicted for healthy patients. In some instances, the subject has a post-BD $FEV_1$ of < 80%, < 75%, < 70%, < 65%, < 60%, < 55%, < 50%, <45%, < 40% or < 35% of the predicted normal value. In some instances, the subject has a post-BD FEV1 which is < 70% and > 30% of the predicted normal value. In some instances, the subject has a post-BD $FEV_1$ which is < 80% and >30%, < 75% and >30%, < 70% and >30%, < 65% and >30%, < 60% and > 30% of the predicted normal value, < 55% and > 30% of the predicted normal value, < 50% and > 30% of the predicted normal value, < 45% and > 30% of the predicted normal value, < 40% and > 30% of the predicted normal value. In some instances, the subject has a post-BD FEV1 which is < 80% and >35%, < 75% and >35%, < 70% and >35%, < 65% and >35%, < 60% and > 35% of the predicted normal value, < 55% and > 35% of the predicted normal value, < 50% and > 35% of the predicted normal value, < 45% and > 35% of the predicted normal value, or < 40% and > 35% of the predicted normal value. In some instances, the subject has a post-BD FEV1 which is < 60% and > 40% of the predicted normal value, < 55% and > 40% of the predicted normal value, < 50% and > 40% of the predicted normal value, or < 45% and > 40% of the predicted normal value.

In some instances, the subject has a post-BD FEV1 which is > 20% of the predicted normal value. In some instances, the subject has a post-DB $FEV_1$ which >21%, >22%, >23%, >24%, >25%, >26%, >27%, >28%, >29% or >30% of the predicted normal value. In some instances, the subject has a post-BD FEV1 which is > 30% of the predicted normal value.

In some instances, the COPD is characterised by the presence of a post-BD FEV1/forced vital capacity (FVC) of < 0.70, < 0.65, < 0.60 < 0.55, < 0.50, < 0.45, < 0.40, < 0.35 or < 0.30. In some instances, the COPD is characterised by the presence of a post-BD FEV1/ FVC of < 0.70.

COPD is a chronic condition, the severity of which may wax and wane. A subject with COPD may therefore experience one or more acute exacerbation of COPD (AECOPD, also referred to herein as a "COPD Exacerbation"), which may be separated by a period with relatively few symptoms.

As used herein, an "AECOPD" or" aeCOPD" is a change in the subject's usual COPD symptoms that lasts 2 or more days, is beyond normal day-to-day variation, is acute in onset, and may warrant a change in regular medication. The change in symptoms may include at least one major or minor COPD symptom from the list below:

- Major COPD symptoms: dyspnea, increase in sputum volume, and change in sputum color

- Minor COPD symptoms: cough, wheeze, sore throat, cold symptoms (rhinorrhea or nasal congestion), and fever without other cause.

In some instances, the change in symptoms includes at least two COPD symptoms from the list above. In some instances, the change in symptoms includes at least one major COPD symptom from the list above. In some instances, the change in symptoms includes at least one major COPD symptom and at least one other major or minor symptom from the list above.

An AECOPD may be classified as mild, moderate, or severe. As used herein, a "severe AECOPD" is one which results in an inpatient COPD-related hospitalization (for example, a subject being hospitalized for the COPD exacerbation or admitted for ≥ 24 hours to an observation area, the emergency department, or other equivalent healthcare facility depending on the country and healthcare system). Severe AECOPD may result in a COPD-related death. A "moderate AECOPD" is one which does not meet the criteria for "severe" (i.e. hospitalization). Moderate and Severe AECOPD result in use of systemic corticosteroids and/or antibiotics, or a single depot injectable dose of corticosteroids. In some instances, AECOPDs are confirmed to have occurred while a subject was on a stable dual or triple maintenance therapy for COPD and not as a result of a gap or step down in the treatment. Finally, a "mild AECOPD" is an exacerbation which does not meet the criteria for "severe" or "moderate".

For severe AECOPD, the start date of the AECOPD may be the earlier of the date of hospitalisation, or, for moderate AECOPD, the start date of systemic corticosteroid or antibiotic treatment, and the end date may be the latest of the end date of the systemic corticosteroid or antibiotic treatment, or the date of hospital discharge.

In some instances, the subject to be treated may have a history of at least one moderate or severe AECOPD in the 12 months prior to treatment (i.e. prior to administration of the first dose). In some instances, the subject may have a history of at least one optionally at least two, moderate, or at least one severe AECOPD in the 11, 10, 9, 8, 7, 6, 5, 4, 3, 2, or 1 months prior to treatment (i.e. prior to administration of the first dose). In some instances, the subject may have a history or at least two moderate AECOPD in the 12 months prior to treatment. In some instances, the subject may have a history or at least two moderate AECOPD within 52 weeks prior to treatment. In some instances, the subject may have a history or at least one severe AECOPD in the 12 months prior to treatment. In some instances, the subject may have a history or at least one severe AECOPD within 52 weeks prior to treatment.

An alternate method of classifying AECOPD is through using the COPD composite exacerbations (COPDCompEx) algorithm, as outlined in "COPDCompEx: A novel composite endpoint for COPD exacerbations to enable faster clinical development" (Vogelmeier et al, Respiratory Medicine, volume 173 November 2020, 106175). The COPDCompEx is a composite endpoint for exacerbations in COPD, combining exacerbations with events defined from participant e-Diaries and PEF. The definitions for both types of exacerbation are as follows: COPDCompEx defined exacerbations: episodes leading to one or more of the following: hospitalization, emergency room visit, treatment with OCS, or treatment with antibiotics. Diary events: defined by threshold and slope criteria using the following diary and home spirometry variables: overall symptom rating, nighttime awakenings due to symptoms, reliever medication use, PEF. Advantageously, COPDCompEx events tend to be more frequent, and offer diagnostic power for the severity of COPD over a shorter timeframe than with AECOPD events as described above.

In some instances, the subject to be treated may have a history of at least one COPDCompEx events in the 12 months prior to treatment (i.e. prior to administration of the first dose). A subject to be treated may have a history of at least one COPDCompEx event in the 11, 10, 9, 8, 7, 6, 5, 4, 3, 2, or 1 months prior to treatment (i.e. prior to administration of the first dose).

COPD may also be classified through the Breathlessness, Cough and Sputum Scale (BCSS) score relative to baseline. As used herein, "baseline", with regard to any COPD marker disclosed herein, such as BCSS score, means the numerical value of that parameter for a patient prior to or at the time of the first administration of the IL-33 therapy.

The BCSS is a 3-item daily diary (Leidy et al 2003) that assesses the severity of the 3 symptoms: breathlessness, sputum, and cough, each on a 5-point scale. Item scores can be reported as domains scores and are summed to yield a total score. In some instances, the subject has a total BCSS score of 4 or more, 5 or more, 6 or more, 7 or more, 8 or more, 9 or more, 10 or more, 11 or more, 12 or more, 13 or more, or 14 or more prior to treatment with the anti-IL33 antibody or fragment thereof. In some instances, the subject has a score of 2 or more, 3 or more, 4 or more, or 5 in the cough domain prior to treatment with the anti-IL33 antibody or fragment thereof. In some embodiments, the subject has a score of 2 or more, 3 or more, 4 or more, or 5 in the sputum domain prior to treatment with the anti-IL33 antibody or fragment thereof. In some instances, the BSCC score "prior to treatment" is an average of the daily scores recorded over the 4 weeks prior to treatment with anti-IL33 antibody or fragment thereof as described herein.

In some instances, COPD is classified using Exacerbations of Chronic Pulmonary Disease Tool - Patient-reported Outcome (EXACT-PRO) score. The EXACT-PRO is a 14-item ePRO instrument developed to assess the frequency, severity and duration of COPD exacerbations (Jones et al 2011; Leidy et al 2011). The instrument was developed for daily, at home, administration using a handheld electronic device. Respondents are instructed to complete the diary each evening just prior to bedtime and to answer the questions while considering their experiences "today". The daily EXACT-PRO total score has a range of 0 to 100 with higher scores indicative of greater severity. Total score changes are used to identify the onset and recovery from an EXACT-PRO defined exacerbation event. In identifying event onset and recovery, the EXACT-PRO can provide information on event frequency and duration as well as event severity. In some instances, the subject to be treated has an EXACT-PRO score of at least 50, at least 60, at least 70, or at least 80 prior to treatment with anti-IL33 antibody or fragment thereof as described herein.

In some instances, the COPD may be classified using the St George's Respiratory Questionnaire (SGRQ). The SGRQ is a 50-item questionnaire developed to measure health status (quality of life) in patients with diseases of airways obstruction. A global score ranges from 0 to 100. Scores by dimension are calculated for three domains: Symptoms, Activity and Impacts (Psycho-social) as well as a total score. Lower score indicates better quality of life (QoL). The first part ("symptoms") evaluates symptomatology, including frequency of cough, sputum production, wheeze, breathlessness and the duration and frequency of attacks of breathlessness or wheeze. The second part has two components: "activity" and "impacts." The "activity" section addresses activities that cause breathlessness or are limited because of breathlessness. The "impacts" section covers a range of factors including influence on employment, being in control of health, panic, stigmatization, the need for medication, side effects of prescribed therapies, expectations for health and disturbances of daily life. The recall period of the questionnaire is over the past 4 weeks. Psychometric testing has demonstrated its repeatability, reliability and validation. Sensitivity has been demonstrated in clinical trials. A minimum change in 4 units (the "Minimum Clinically Important Difference") was established as clinically relevant after patient and clinician testing (Jones COPD 2005 2(1):75-9).

In some instances, COPD may be classified by the COPD Assessment Test (CAT) score. The CAT is a questionnaire that is designed for patients with COPD to measure the effects of the disease on their quality of lives. The CAT is an 8 item patient-completed questionnaire assessing globally the impact of COPD (cough, sputum, dyspnea, chest tightness) on health status. The CAT ranges in scope from 0 to 40. Higher scores denote a more severe impact of COPD on a subject's life. In some instances, the subject to be treated has a CAT score prior to therapy of at least 10.

In some instances, the COPD may be classified through E-RS™:COPD, an 11-item ePRO developed to evaluate the severity of respiratory symptoms of COPD (Leidy et al 2014a; Leidy et al 2014b). The E-RS™:COPD is a subset of items from the EXACT-PRO. The E-RS™:COPD was designed to be captured as part of the daily EXACT-PRO assessment. Summation of E-RS™:COPD item responses produces a total score ranging from 0 to 40, with higher scores indicating greater severity. In addition to the total score, symptom domain scores can be calculated for breathlessness (5 items; score range: 0 to 17), cough and sputum (3 items; score range: 0 to 11) and chest symptoms (3 items; score range: 0 to 12) by summing the responses of items within a respective domain. In some instances, the subject to be treated has an E-RS™:COPD score of at least 20, at least 25, at least 30, or at least 35. In some instances, the subject to be treated has an E-RS™:COPD score in the cough and sputum domain of at least 6, at least 7, at least 8, at least 9, at least 10, or 11. In some instances, the subject to be treated has an E-RS™:COPD score in the chest symptom domain of at least 7, at least 8, at least 9, at least 10, at least 11, or 12. In some instances, the subject to be treated has an E-RS™:COPD score in the breathlessness domain of at least 9, at least 10, at least 11, at least 12, at least 13, at least 14, at least 15, at least 16, or 17.

In some instances, COPD is associated with chronic bronchitis in the subject. A subject with chronic bronchitis may have suffered from the symptoms of bronchitis (cough, production of mucus/sputum, fatigue, shortness of breath, fever, chills, and/or chest discomfort) for a period of more than 8 weeks, more than 16 weeks, more than 32 weeks, or more than 52 weeks.

In some instances, the subject to be treated is a current smoker or former smoker. In some instances, the subject to be treated has a smoking history of 10 or more pack-years. Pack-years are calculated as average number of cigarettes per day × number of years / 20. For example, 1 pack-year = 20 cigarettes smoked per day for 1 year or 10 cigarettes per day for 2 years.

In some instances, the subject is a current smoker.

In some instances, the subject is a former smoker. A "former smoker" may be defined as a subject who is not smoking at the onset of therapy and with smoking cessation for a minimum of 6 months prior to therapy onset with an intention to quit permanently.

In some instances, the subject may have a history of inadequate response or intolerance to other medications for COPD, such as inhaled corticosteroids (ICS), long-acting beta agonists (LABA), and/or long-acting muscarinic antagonists (LAMA), or these other medications for COPD may otherwise be medically inadvisable. As used herein an "inadequate response" to a treatment if administration of said treatment does not result in the short- and/or long-term amelioration of one of more symptoms of COPD as described herein. Alternatively, an inadequate response may result in a return of the condition to moderate-to-severe levels following secession of said treatment. In some instances, COPD may have been previously treated with an ICS and a LABA, an ICS and a LAMA, a LABA and a LAMA, or an ICS, a LABA and a LAMA, and has been shown to be unresponsive to said treatment. COPD may be classified as having an inadequate response to a treatment if the COPD remains moderate to severe despite the treatment, or if the subject experiences a moderate or severe AECOPD event following secession or completion of the course of therapy.

In some instances, the subject to be treated may have received or is receiving a course of ICS, LAMA and/or LABA therapy (e.g. ICS-LAMA, ICS-LABA, LAMA-LABA or ICS-LAMA-LABA therapy, collectively referred to as "COPD inhaled maintenance therapies") prior to treatment with the anti-IL-33 antibody or antibody variant thereof as described herein. In some instances, the course of therapy may have begun at least 3 months prior to the administration of the first dose of the anti-IL-33 antibody or antibody variant thereof as described herein, and may be at least 3 months long. In some instances, the course of therapy began at least 3 months prior to the administration of the first dose of the anti-IL-33 antibody or antibody variant thereof as described herein, and may be ongoing at the start of and may be continued during the treatment window with said anti-IL-33 antibody or antibody variant thereof.

As used herein, "inhaled corticosteroids (ICS)" refers to any corticosteroid treatment administered for the treatment of COPD through use of a nebuliser, inhaler, or vaporiser. The ICS may be selected from fluticasone propionate, budesonide, and/or beclometasone dipropionate.

As used herein, "long-acting Beta-2 adrenergic receptor agonists (LABA)" refers to any beta-adrenoceptor agonist with a duration of action of approximately 12 hours or more. This is in contrast to short-acting beta agonists (SABA) such as salbutamol, which have a duration of action of approximately 4-6 hours. Exemplary LABAs include arformoterol, bambuterol, clenbuterol, formoterol, salmeterol, protokylol. A LABA may be an "ultra-LABA" with a duration of action of 24 hours or more, for example indacaterol, olodaterol, or vilanterol. A LABA may be administered through any suitable route, for example through use of a nebuliser, inhaler, or vaporiser.

As used herein, long-acting muscarinic antagonists (LAMA) re anticholinergic agents that block the activity of the muscarinic acetylcholine receptor. Exemplary LAMA include tiotropium bromide, glycopyrronium bromide, and aclidinium bromide. A LAMA may be administered through any suitable route, for example through use of a nebuliser, inhaler, or vaporiser.

A subject with an "intolerance" to a treatment is one for whom the treatment provokes one or more side-effects which make continuation of the treatment inadvisable. For example, allergic reactions to treatment may be indicative of an intolerance.

In some instances, the COPD to be treated is moderate to severe COPD. In some instances, the moderate to severe COPD is characterised by:

- Association with chronic bronchitis in the subject,

- A history of at least one, optionally at least two, moderate or at least one severe acute exacerbation of COPD in the 12 months prior to treatment,

- A post-bronchodilator forced expiratory volume in 1 second (FEV1) to forced vital capacity (FVC) ratio (post-BD FEV1/FVC) of <0.70, and/or a post-BD FEV1 > 30% and < 80% predicted normal value.

- An average breathlessness, cough and sputum scale (BCSS) score of > 2 in cough and/or > 2 in sputum domains.

In some instances, the COPD to be treated is characterised by:

- A history of at least two moderate or at least one severe acute exacerbation of COPD within 52 weeks prior to treatment,

- A post-bronchodilator forced expiratory volume in 1 second (FEV1) to forced vital capacity (FVC) ratio (post-BD FEV1/FVC) of <0.70

- A post-BD FEV1 > 20% of predicted normal value.

- A CAT score of greater than or equal to 10, and each of the phlegm and cough items greater than or equal to 2, prior to treatment.

In certain instances, the subject has a blood eosinophil count of greater than or equal to about 300 cells per μl or less than 300 cells per μl prior to treatment. In some instances, the subject has a blood eosinophil count of greater than or equal to 300 cells per μl prior to treatment.

*Administration Regimens*

The present disclosure relates to dosage regimens of an anti-IL-33 antibody or antibody variant, which finds particular effectiveness in the treatment of COPD. A dosage regimen is made up of one or more doses of a controlled size, administered throughout a treatment window. Where there is more than one dose, the doses are separated by a dosing interval. The anti-IL-33 antibody or antibody variant is administered in a therapeutically effective amount. As used herein, an "effective amount" or "therapeutically effective amount" of an agent, e.g., a pharmaceutical formulation comprising an IL-33 antibody, refers to an amount effective, at dosages and for periods of time necessary, to achieve the desired therapeutic or prophylactic result.

The size of the dose of anti-IL-33 antibody or antibody variant thereof may be expressed in terms of weight of the anti-IL-33 antibody or antibody variant. In certain instances, the anti-IL-33 antibody or antibody variant thereof is administered in a dose of about 600 mg.

In some instances, the dose is 600 mg. In some instances, the anti-IL-33 antibody or antibody variant thereof may be formulated for subcutaneous injection at 150 mg/mL, such that a 600 mg dose is administered as a 4 mL treatment. A 600 mg dose of the anti-IL-33 antibody or antibody variant thereof may be administered as two concurrent 300 mg dosages. As used herein, "concurrent" doses refer to doses which are administered simultaneously, or sequentially with no or only a minimal time period (e.g. less than 1 hour, less than 30 minutes, less than 15 minutes, less than 5 minutes) separating them.

In some instances, the anti-IL-33 antibody or antibody variant thereof is administered in a dose of about 300 mg.

In some instances, the dose is 300 mg. In some instances, the anti-IL-33 antibody or antibody variant thereof may be formulated for subcutaneous injection at 150 mg/mL, such that a 300 mg dose is administered as a 2 mL treatment. In some instances, a 300 mg dose of the anti-IL-33 antibody or antibody variant thereof may be administered as two concurrent 150 mg dosages. As used herein, "concurrent" doses refer to doses which are administered simultaneously, or sequentially with no or only a minimal time period (e.g. less than 1 hour, less than 30 minutes, less than 15 minutes, less than 5 minutes) separating them.

The size of the dose of an anti-IL-33 antibody or antibody variant thereof may be expressed in terms of the plasma drug concentration provided by the dose, as the amount of active compound manipulated so as to provide a plasma drug concentration of a certain level. By varying the amount, bioavailability, or timing/frequency of the antibody or variant administered, the skilled person can control the plasma concentration in the subject. As plasma concentrations vary across time with drug uptake and clearance, they may be expressed in various standardised ways - for example as a maximum, minimum (trough) or across time.

In some instances, the dose may be selected so as to provide a $C_{max,ss}$ (the observed maximum concentration at steady state) of between about 20 and about 50 $\mu$g/mL, between about 25 and about 45 $\mu$g/mL, between about 30 and about 40 $\mu$g/mL, between about 35 and about 40 $\mu$g/mL, or about 37 $\mu$g/mL In some instances, the $C_{maz,ss}$ is that observed during the dosing period. In this context, the "dosing period" refers to the time between two consecutive doses.

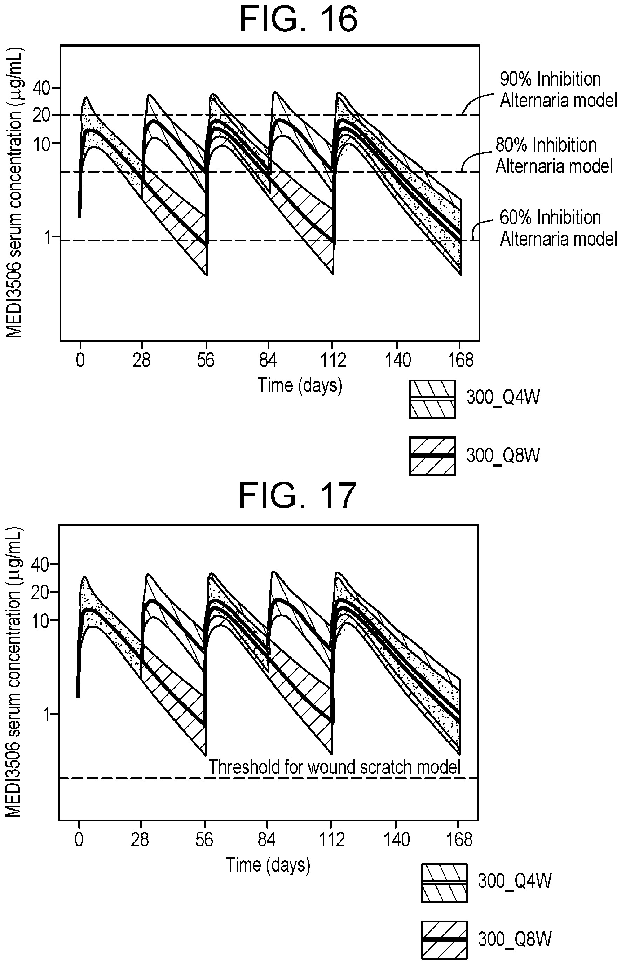

The examples show that a 300 mg Q4W or Q8W dosing regimen of MEDI3 506 are predicted to achieve serum concentrations necessary for inhibition of both the redIL-33:ST2 signalling axis and the oxIL-33:RAGE/EGFR signalling axis, achieving sustained, dual pathway inhibition (Figures 16 and 17). The MEDI3506 serum concentration can be measured (and therefore used to determine $C_{max,ss}$) anti-drug antibody reagents in a suitable assay format to capture and detect MEDI3506 from a biological sample (e.g. blood). In some instances, the assay may use an anti-IgG1 capture mAb and a stabilised MEDI3506 antigen labelled with a detectable marker. The detectable marker can be quantified in order to determine the concentration of MEDI3506. In some instances, the MEDI3506 antigen (IL-33) may stabilised in a reduced form, for example, by mutating one or more cysteine residues to serine to prevent redIL-33 conversion to the oxidised form (oxIL-33) through disulphide bond formation. Assays and platforms suitable for the detection of serum biomarkers are well known to the skilled person.

In some instances, the dose is selected so as to provide a $C_{max,ss}$ of between about 10 and about 35 $\mu$g/mL, between about 15 and about 30 $\mu$g/mL, between about 15 and about 30 $\mu$g/mL, between about 15 and about 25 $\mu$g/mL, about 15 to about 20 $\mu$g/mL, or about 18.6 $\mu$g/mL.

In some instances, the anti-IL-33 antibody or antibody variant thereof may be administered in a dose selected so as to provide an area under the plasma concentration-time curve throughout a dosing period (AUC).

In some instances, the dose may be selected so as to provide an AUC of between about 400 and about 800 $\mu$g · day/mL, between about 500 and about 750 $\mu$g · day/mL, between about 600 and about 700 $\mu$g · day/mL, between about 600 and about 650 $\mu$g · day/mL, between about 600 and about 620 $\mu$g · day/mL, between about 610 and about 620 $\mu$g · day/mL, or about 616 $\mu$g · day/mL over the dosing period.

In some instances, the dose may be selected so as to provide an AUC of between about 200 and about 515 $\mu$g · day/mL, between about 250 and about 500 $\mu$g · day/mL, between about 300 and about 450 $\mu$g · day/mL, between about 300 and about 350 $\mu$g · day/mL, or about 323 $\mu$g · day/mL over the dosing period.

In some instances, the dose may be selected so as to provide an AUC of between about 100 and about 300 $\mu$g · day/mL, between about 100 and about 250 $\mu$g · day/mL, between about 100 and about 200 $\mu$g · day/mL, between about 150 and about 200 $\mu$g · day/mL, or about 161.5 $\mu$g · day/mL over the dosing period.

Administration of the anti-IL-33 antibody or antibody variant thereof is performed as multiple doses separated by a dosing interval. In some instances, the dosing interval is 2 weeks (14 days) or 4 weeks (28 days). In some embodiments, the dosing interval is 4 weeks (28 days). In some instances, the dosing interval is about 4 weeks (i.e. 28 $\pm$ 4 days). In some instances, the dosing interval is about 8 weeks (i.e. 56$\pm$ 4 days).

In some instances, a dose may be administered across multiple days, for example, as two or more sub-doses. As used herein, a "sub-dose" is a fractional quantity of a dose of therapeutic, such that the total quantity of therapeutic administered in sub-doses is equal to that in the dose. Any fractional quantity may be used, for example such that two, three, four, five or more sub-doses comprise a single dose. In some instances, a dose may be administered as two or more sub-doses separated by a period of 1, 2, 3, 4, 5, or 6 days. In some instances, a dose may be administered as two or more sub-doses separated by a period of 1, 2, or 3 weeks. Sub-doses may be administered on two, three, four or more consecutive days. Sub-doses which make up a dose may be of equal size, or may differ in size, so long as their total is equal to the dose.

Therefore, as used herein, a dose of 600 mg with a 4 week dosing window (Q4W) may be substituted for 150 mg administered weekly (Q1W), 300 mg administered every 2 weeks (Q2W), or 450 mg administered every 3 weeks (Q3W), all of which provide a dosing regimen equivalent to 600 mg every 4 weeks. A dose of 300 mg with a 4 week dosing window (Q4W) may be substituted for 150 mg administered every two weeks (Q2W) or 75 mg administered weekly(Q1W). A dose of 300 mg with an 8 week dosing window (Q8W) may be substituted for 150 mg administered every four weeks (Q4W) or 75 mg administered every two weeks (Q2W), or 37.5 mg administered every week (Q1W).

When the dosing interval is expressed as a number of weeks, a margin of error is permissible such that a week may be expressed as 7 days ± 1 day. In some embodiments, a week may be expressed as 7 days ± 0.5 days, 7 days ± 0.25 days, or exactly 7 days. Where the dosing interval is multiple weeks, the margins of error in each week may be combined. For example, in some instances, the dosing interval is 4 weeks ± 4 days. In some instances, the dosing interval may be 4 weeks ± 3 days. In some embodiments, the dosing interval may be 4 weeks ± 2 days. In some embodiments, the dosing interval may be 4 weeks ± 1 day. In some instances, the dosing interval may be exactly 4 weeks. In some instances, the dosing interval may be 8 weeks ± 4 days. In some instances, the dosing interval may be 8 weeks ± 3 days. In some instances, the dosing interval may be 8 weeks ± 2 days. In some instances, the dosing interval may be 8 weeks ± 1 day. In some instances, the dosing interval may be exactly 8 weeks.

In some instances, the anti-IL-33 antibody or antibody variant thereof is administered during a "treatment window", which as used herein refers to a period commencing at the administration of the first dose and running until the final dose of the anti-IL-33 antibody or antibody variant thereof is administered. The date the first dose is administered is referred to as "Day 1" of "Week 0", with Week 1 commencing 7 days later, Week 2 commencing 7 days after that, and so on. In some embodiments, the treatment window may be 12 weeks long (i.e. running from Week 0 to Week 12). In some embodiments, the treatment window may be 16 weeks long (i.e. running from Week 0 to Week 15) and the dosing interval is 4 weeks, such that a total of 4 doses are administered (on Week 0, 4, 8 and 12 respectively). In some embodiments, the treatment window may be 12 weeks long, and the dosing interval is 4 weeks, such that doses are administered Days 1 (Week 0), 29 ± 4 (Week 4), 57 ± 4 (Week 8), and 85 ± 4 (Week 12).

In some instances, the treatment window may be 12 weeks, 14 weeks, 16 weeks, 18 weeks, 20 weeks, 22 weeks, 24 weeks, 26 weeks, 28 weeks, 30 weeks, 32 weeks, 34 weeks, 36 weeks, 38 weeks, 40 weeks, 42 weeks, 44 weeks, 46 weeks, 48 weeks, 50 weeks, 52 weeks or more. In some instances, the treatment window is 52 weeks or more. In some instances, the treatment window may be 48 weeks or more.

In some instances, the anti-IL-33 antibody or antibody variant thereof is administered at about 300 mg Q4W. The examples show that administration of MEDI3506 300 mg Q4W is predicted to achieve approximately 94% depletion (target engagement) in the lung. This level of target engagement is potentially greater than that predicted to be achieved for the anti-IL-33 antibody itepekimab, in which a recent Phase II study in COPD resulted in a marked reduction of COPD exacerbations as well as a significant improvement of FEV1 in former smokers (Rabe et al 2021). Itepekimab has a significantly longer reported half-life compared to MEDI3506 (The itepekimab $t_{1/2}$, which also known as SAR440340 and REGN3500, is reported as 30 days in US2021/0000949 - see [411] therein).

In some instances, the anti-IL-33 antibody or antibody variant thereof is administered at about 300 mg Q8W. The examples show that administration of MEDI3506 300 mg Q8W (i.e., with a twice as long dosing window) is predicted to provide approximately 83% inhibition of IL 33 at trough in lung tissue; therefore, it could provide an adequate efficacy in COPD with a more convenient dose frequency for patients compared with Q4W.

In some instances, therefore, the IL-33 antibody or antibody variant thereof may be administered at a dose that achieves at least 80%, 85% or 90% target engagement in the lung. In some instances, the dose may achieve at least 90% target engagement in the lung. In some instances, the dose may achieve at least 91%, 92%, 93% or 94% target engagement in the lung. In some instances, the % target engagement may be achieved at trough concentration.

### Markers of COPD

In some instances, the methods disclosed herein improve one or more markers of COPD (also referred to as "COPD markers").

COPD markers include: annualised rate of moderate to severe COPD exacerbations, time to first moderate to severe COPD exacerbation, time to first severe COPD exacerbation, FEV1, FVC, E-RS COPD total score, SGRQ score, CAT score.

In some instances, an improvement in E-RS COPD score means a subject has experienced a decrease in CAT score of more than or equal to 2 (the "minimum clinically important difference", or "MCID") compared to baseline.

In some instances, an improvement in CAT score means a subject has experienced a decrease in CAT score of more than or equal to 2 (the "minimum clinically important difference", or "MCID") compared to baseline.

In some instances, an improvement in SGRQ score means a subject has experienced a decrease in SGRQ total score of more than or equal to 4 (the "minimum clinically important difference", or "MCID") compared to baseline.

In some instances, the change in a marker may be observed following the treatment window. In some instances, the observation may occur immediately after the treatment window. In some instances, the observation may be made after an additional period running immediately from the treatment window. The additional period may be 1, 2, 3, 4, 5, 6, or more days. The additional period may be 1, 2, 3, 4 or more weeks. The additional period may be 1, 2, 3, 4 or more months. The additional period may be the same length as the dosing interval, such that the additional period follows the final dose and runs until the point where a further dose would be due. The treatment window plus any additional period may be referred to as the "intervention window". Certain treatment outcomes may be measured after the intervention window, for example change in pre- or post-BD FEV1 or FEV1 i/FVC.

In some instances, the additional period may be 4 weeks and the treatment window may be 8 weeks, such that the total length of the treatment window plus the additional period is 12 weeks. In some embodiments, the additional period may be 4 weeks and the treatment window may be 12 weeks, such that the total length of the treatment window plus the additional period is 16 weeks. In other embodiments, the additional period may be 4 weeks and the treatment window may be 24 weeks, such that the total length of the treatment window plus the additional period is 28 weeks.

Alternatively or additionally, the change in marker may be observed during the treatment window. A marker may be observed at the end of a dosing period, for example the first, second, third, fourth, fifth, sixth, seventh, or further dosing period. Alternatively, a marker may be observed or measured one day after the end of a dosing period, for example the first, second, third, fourth, fifth, sixth, seventh, or further dosing period. A marker may be observed or measured one day before the end of a dosing period, for example the first, second, third, fourth, fifth, sixth, seventh, or further dosing period. A marker may be observed two days after the end of a dosing period, for example the first, second, third, fourth, fifth, sixth, seventh, or further dosing period. A marker may be observed two days before the end of a dosing period, for example the first, second, third, fourth, fifth, sixth, seventh, or further dosing period. A marker may be observed three days after the end of a dosing period, for example the first, second, third, fourth, fifth, sixth, seventh, or further dosing period. A marker may be observed three days before the end of a dosing period, for example the first, second, third, fourth, fifth, sixth, seventh, or further dosing period. A marker may be observed four days after the end of a dosing period, for example the first, second, third, fourth, fifth, sixth, seventh, or further dosing period. A marker may be observed four days before the end of a dosing period, for example the first, second, third, fourth, fifth, sixth, seventh, or further dosing period. In some embodiments, the treatment window may be 24 weeks, and the change in symptom is observed during week 12.

Certain treatment outcomes may be measured at the 4, 12, 24, or 28, 36 or 52-week time point, for example change in pre-BD FEV1 or FEV1 i/FVC scores.

Treatment with the anti-IL-33 antibody or variant thereof, as described herein, may result in an increase in forced expiratory volume in 1 second (FEV1) as defined herein, in particular an increase observed following the treatment window (and any additional period as defined herein) as relative to baseline. In some instances, the increase in FEV1 is observed at weeks 2, 4, 8, 12, 16, 20, 24, 28, 32, 36 or 52 relative to baseline. In some instances, the increase in $FEV_1$ may be observed at week 4, 12, 24, 36 or 52 relative to baseline. In some instances, the increase in $FEV_1$ may be by 5%, 10%, 15%, 20%, 30%, 40%, 50%, 60%, 70%, 80%, 90%, 100%, or more than 100% of the FEV1 observed prior to treatment. In some instances, the increase in $FEV_1$ may be to > 70 %, > 75% or >80% of the predicted normal value.

In some instances, treatment with the anti-IL-33 antibody or variant thereof, as described herein, may result in an increase in FEV1/FVC relative to baseline. In some instances, the increase in FEV1/FVC may be of 5%, 10%, 15%, 20%, 30%, 40%, 50%, 60%, 70%, 80%, 90%, 100%, or more than 100% of the post-BD FEV1/FVC relative to baseline. In some instances, the increase in FEV1/FVC may be an increase of at least 0.1, 0.2, 0.3, 0.4, 0.5, 0.6 or 0.7. In some instances, the increase in FEV1/FVC may be to > 0.70. In some instances, the increase in FEV1/FVC may be to > 0.75, > 0.80, > 0.85, > 0.90, > 0.95 or > 0.99.

In some instances, the one or more markers may be selected from a decrease in the frequency, duration, or severity of AECOPD, optionally the frequency, duration or severity of moderate or severe AECOPD, relative to baseline. In some instances, the decrease in the frequency, duration, or severity of AECOPD may be observed at week 4, 8, 12, 16, 29, 24, 28, 32, 36, 40, 44, 48 or 52 relative to baseline. In some instances, the decrease in the frequency, duration, or severity of AECOPD may be observed at week 52 relative to baseline.

In some instances, treatment with the anti-IL-33 antibody or variant thereof, as described herein, may result in a decrease in the frequency of AECOPD. In some instances, the decrease in the frequency of AECOPD may be a 5%, 10%, 15%, 20%, 30%, 40%, 50%, 60%, 70%, 80%, 90%, or 95% decrease in the frequency of AECOPD relative to baseline. In some instances, the decrease in frequency of AECOPD may be a decrease over 1, 2, 3, 4, 5, 6, 7, 8, 9, 10, 11 or 12 months. In some instances, the decrease in frequency of AECOPD may be a decrease over 12 months. In some instances, the decrease in frequency of AECOPD may be a decrease in annual AECOPD frequency. In some instances, the decrease in frequency of AECOPD may be a decrease to an average of one AECOPD in every 8 weeks, one AECOPD in every 16 weeks, one AECOPD in every 32 weeks, one AECOPD in every 52 weeks, or less than one AECOPD in every 52 weeks.

In some instances, treatment with the anti-IL-33 antibody or variant thereof, as described herein, may result in a decrease in the average duration of AECOPD. In some instances, the decrease in average duration of AECOPD may be a 5%, 10%, 15%, 20%, 30%, 40%, 50%, 60%, 70%, 80%, 90%, or 95% decrease in the average duration of AECOPD relative to baseline. In some instances, the decrease in average duration of AECOPD may be a decrease to an average duration of 24hrs or less.

In some instances, treatment with the anti-IL-33 antibody or variant thereof, as described herein, may result in a decrease in the severity of AECOPD. A decrease in severity may result in a decrease in the frequency or duration of moderate and/or severe AECOPD relative to baseline, and this may be accompanied by an increase in the frequency or duration of mild AECOPD. A decrease in severity may result in a decrease in the frequency or duration of severe AECOPD relative to baseline, and this may be accompanied by an increase in the frequency or duration of moderate or mild AECOPD.

In some instances, the one or more symptoms may be selected from a decrease in the frequency or duration of COPDCompEx events, relative to baseline. In some instances, the decrease in the frequency or duration COPDCompEx may be observed at week 4, 8, 12, 16, 29, 24, 28, 32, 36, 40, 44, 48 or 52 relative to baseline.

In some instances, treatment with the anti-IL-33 antibody or variant thereof, as described herein, may result in a decrease in the frequency of COPDCompEx events. In some instances, the decrease in the frequency of COPDCompEx events may be a 5%, 10%, 15%, 20%, 30%, 40%, 50%, 60%, 70%, 80%, 90%, or 95% decrease in the frequency of COPDCompEx events relative to baseline. In some instances, the decrease in frequency of COPDCompEx events may be a decrease over 1, 2, 3, 4, 5, 6, 7, 8, 9, 10, or 11 months. In some instances, the decrease in frequency of COPDCompEx events may be a decrease in annual COPDCompEx events frequency. In some embodiments, the decrease in frequency of COPDCompEx events may be a decrease to an average of one COPDCompEx event in every 8 weeks, one COPDCompEx event in every 16 weeks, one COPDCompEx event in every 32 weeks, one COPDCompEx event in every 52 weeks, or less than one COPDCompEx event in every 52 weeks.

In some instances, treatment with the anti-IL-33 antibody or variant thereof, as described herein, may result in a decrease in the average duration of COPDCompEx events. In some instances, the decrease in average duration of AECOPD may be a 5%, 10%, 15%, 20%, 30%, 40%, 50%, 60%, 70%, 80%, 90%, or 95% decrease in the average duration of COPDCompEx events relative to baseline. In some instances, the decrease in average duration of COPDCompEx events may be a decrease to an average duration of 24 hrs or less.

In some instances, the treatment may result in a decrease in objective cough frequency over 24 hours, relative to baseline. Objective cough frequency over 24 hours may be measured using an automated cough monitor (ACM), for example the VitaloJAK™ (Vitalograph, Buckinghamshire, UK), which is fitted and worn by the subject for approximately 24 hours and records cough frequency. Alternatively, objective cough frequency may be recorded through alternative means, for example recording or direct observation of the subject, followed by construction of a tally or count. In some embodiments, the decrease in objective cough frequency may be a 5%, 10%, 15%, 20%, 30%, 40%, 50%, 60%, 70%, 80%, 90%, 95% or more than 95% decrease relative to baseline.

In some instances, the treatment may result in a decrease in reliever medication usage, including reliever inhaler usage, relative to baseline. Reliever usage may be expressed as the number of puffs (i.e. the sum of different relievers, if applicable) used by the subject in a 24 hour period. In some instances, reliever usage may be expressed as the average usage in a 24 hour period, i.e. the total count of reliever usage during a period and averaged over 24 hours, wherein the period is more or less than a 24 hours. In some instances, the decrease in reliever medication usage may be a 5%, 10%, 15%, 20%, 30%, 40%, 50%, 60%, 70%, 80%, 90%, 95% or more than 95% decrease relative to baseline.

In some instances, the treatment results in a decrease in EXACT-PRO score relative to baseline. In some embodiments, the decrease may be of 5, 10, 15, 20, 25, 30, 35, 40, 45, 50, 55, 60, 65, 70, 75, 80, 95 or more points on the EXACT-PRO scale, relative to baseline. In some instances, the decrease may be from a baseline score of 50 or more to a post-treatment score of less than 50. In some instances, the decrease may be from a baseline score of 60 or more, of 70 or more, or of 80 or more, to a post-treatment score of less than 50. In some instances, the decrease may be from a baseline score of 50 or more, 60 or more, of 70 or more, or of 80 or more, to a post-treatment score of less than 40. In some instances, the decrease may be from a baseline score of 40 or more, 60 or more, of 70 or more, or of 80 or more, to a post-treatment score of less than 30.

In some instances, the treatment results in a decrease in E-RS™:COPD score relative to baseline. In some instances, the decrease may be of 5, 10, 15, 20, 25, 30, 35 or more points on the E-RS™:COPD scale, relative to baseline. In some instances, the decrease may be from a baseline score of 9 or more to a post-treatment score of less than 9 in the breathlessness domain. In some instances, the decrease may be from a baseline score of 6 or more to a post-treatment score of less than 6 in the cough and sputum domain. In some instances, the decrease may be from a baseline score of 7 or more to a post-treatment score of less than 7 in the chest symptom domain.

In some instances, the treatment may result in an improvement in Cough visual analogue scale (Cough VAS) relative to baseline. Cough VAS, or cough severity VAS, comprises a 100 mm linear scale marked with a horizontal line by the subject, with 0 mm representing "no cough" and 100 mm representing "worst cough", measuring subjective assessment by the subject of the prior 24 hrs for severity of cough symptoms (Smith et al 2006). In some instances, the improvement may be a 5%, 10%, 15%, 20%, 30%, 40%, 50%, 60%, 70%, 80%, 90%, 95% or more than 95% decrease in Cough VAS relative to baseline.

In some instances, the treatment results in an improvement in Breathlessness, Cough and Sputum Scale (BCSS) score relative to baseline. In some instances, the improvement may be a decrease in the total score relative to baseline. In some instances, the improvement may be a 1, 2, 3, 4, 5, 6, 7, 8, 9, 10, or more than 10 point improvement in total score. In some instances, the improvement may comprise a decrease in the sputum and/or cough domain score relative to baseline. In some embodiments, the improvement may be a 2, 3, 4 or 5 point decrease in sputum, and/or cough domain score. In some instances, the improvement in BCSS score may comprise a reduction in sputum and/or cough domain score from >2 to < 2. In some instances, the improvement in BCSS score may comprise a reduction in sputum and/or cough domain score from >2 to <2.

In some instances, the treatment may result in an improvement in Cough and Sputum Assessment Questionnaire (CASA-Q) score relative to baseline. The CASA-Q is a self-administered questionnaire that assesses cough and sputum based on their frequency, severity, and impact on daily activities in the previous 7 days (Crawford et al 2008; Monz et al 2010). The CASA-Q contains four domains: cough symptoms, cough impact, sputum symptoms, and sputum impact. Each domain contains three to eight items, each of which is answered in five categories from "never" to "always" for frequency and from "not at all" to "a lot/extremely" for intensity. For each domain, the items are summed and rescaled to obtain a score ranging from 0 to 100, with higher scores associated with fewer symptoms or less impact. In some instances, the improvement may be an increase in the scores in one or more of the four domains relative to baseline. In some instances, the domain's score may increase by 10, 20, 30, 40, 50, 60, 70, 80 or more points relative to baseline.

In some instances, the treatment results in an improvement in St George's Respiratory Questionnaire (SGRQ) score relative to baseline. The SGRQ is a 50-item ePRO instrument developed to measure the health status of participants with airway obstruction diseases (Jones et al 1991). The questionnaire is divided into 2 parts: Part 1 consists of 8 items pertaining to the severity of respiratory symptoms in the preceding 4 weeks; Part 2 consists of 42 items related to the daily activity and psychosocial impacts of the individual's respiratory condition. The SGRQ yields a total score and 3 domain scores (symptoms, activity, and impacts). The total score indicates the impact of disease on overall health status. This total score is expressed as a percentage of overall impairment, in which 100 represents the worst possible health status and 0 indicates the best possible health status. Likewise, the domain scores range from 0 to 100, with higher scores indicative of greater impairment. Based on empirical data and interviews with patients, a change of 4 units is associated with a minimum clinically important difference. Specific details on the scoring algorithms are provided by the developer in a user manual (Jones and Forde 2009). In some instances, the improvement may be a decrease of SGRQ score by 4 or more units relative to baseline. In some embodiments, the improvement may be a decrease of SGRQ score by 8, 12, 16, 20 or more units relative to baseline. In some instances, the improvement may be a decrease of SGRQ score by 5, 10, 20, 30, 40, 50, 60, 70, 80 or more units relative to baseline.

In some instances, the treatment may result in a decrease in the frequency, duration, or severity of a symptom of COPD selected from dyspnea, increase in sputum volume, change in sputum colour, cough, wheeze, sore throat, cold symptoms (rhinorrhea or nasal congestion), and fever without other cause. In some instances, the treatment may result in a decrease in the frequency, duration, or severity of a symptom selected from dyspnea, increase in sputum volume, and change in sputum colour. In some embodiments, the treatment may result in a decrease in the frequency, duration, or severity of a symptom selected from cough, wheeze, sore throat, cold symptoms (rhinorrhea or nasal congestion), and fever without other cause.

In some instances, the treatment may result in a decrease in the frequency, duration of one or more of the symptoms of chronic bronchitis in the subject, selected from cough, production of mucus/sputum, fatigue, shortness of breath, fever, chills, and/or chest discomfort. In some instances, the treatment may result in a decrease in the frequency, duration, or severity of a symptom of chronic bronchitis to an average of one case in 8 weeks, one case in 16 weeks, one case in 32 weeks, one case in 52 weeks, or less than one case in 52 weeks.

*Anti-IL-33 antibodies*

The therapies described herein relate to anti-IL-33 antibodies, and variants and fragments thereof.

Interleukin-33 (IL-33) is a member of the interleukin-1 (IL-1) cytokine family that is encoded by the IL33 gene. IL-33 is constitutively expressed in multiple cell types, including structural cells, such as smooth muscle, epithelial, and endothelial cells. It has been reported that IL-33 expression can also be induced by inflammatory factors in macrophages and dendritic cells. Cellular stress caused by environmental triggers, such as allergens, toxins, and pathogens, and mechanistic insult can lead to IL-33 release. Free IL-33 associates with a heterodimeric IL-33 receptor complex composed of suppression of tumorigenicity 2 (ST2) protein and interleukin-1 receptor accessory protein (IL-1 RAcP) to activate the AP-1 and NF-κB pathways through the adaptor protein myeloid differentiation primary response 88 (MyD88) and possibly MyD88-adapter-like (Mal) protein. IL-33 stimulates numerous cell types, including innate lymphoid type II cells (ILC2), mast cells, basophils, eosinophils, and dendritic cells, to promote an immune response.

The terms "interleukin 1 receptor-like 1 (IL1RL1)" and "ST2," used interchangeably herein, refer to any native ST2 from any vertebrate source, including mammals such as primates (e.g., humans) and rodents (e.g., mice and rats), unless otherwise indicated. ST2 is also referred to in the art as DER4, T1, and FIT-1. The term encompasses "full-length," unprocessed ST2, as well as any form of ST2 that results from processing in the cell. At least four isoforms of ST2 are known in the art, including soluble (sST2, also known as IL 1 RL 1-a) and transmembrane (ST2L, also known as IL 1 RL 1-b), which arise from differential mRNA expression from a dual promoter system, and ST2V and ST2LV, which arise from alternative splicing. The domain structure of ST2L includes three extracellular immunoglobulin-like C2 domains, a transmembrane domain, and a cytoplasmic Toll/Interleukin-1 receptor (TIR) domain. sST2 lacks the transmembrane and cytoplasmic domains contained within ST2L and includes a unique 9 amino acid (a.a.) C-terminal sequence (see, e.g., Kakkar et al. Nat. Rev. Drug Disc.40 7: 827-840, 2008). sST2 can function as a decoy receptor to inhibit soluble IL-33. The term also encompasses naturally occurring variants of ST2, e.g., splice variants (e.g., ST2V, which lacks the third immunoglobulin motif and has a unique hydrophobic tail, and ST2LV, which lacks the transmembrane domain of ST2L) or allelic variants (e.g., variants that are protective against COPD risk or that confer COPD risk as described herein). The amino acid sequence of an exemplary human ST2 can be found, for example, under UniProtKB accession number 001638. ST2 is a part of the IL-33 receptor along with the co-receptor protein IL-1 RAcP. Binding of IL-33 to ST2 and the co-receptor interleukin-1 receptor accessory protein (IL-1 RAcP) forms a 1:1:1 ternary signaling complex to promote downstream signal transduction (Lingel et al. Structure 17(10): 1398-1410,2009, and Liu et al. Proc. Nat. Acad. Sci. 11 0(37): 14918-14924, 2013).

It is contemplated that antibodies or antibody variants that specifically bind to and inhibit components of the IL-33/ST2 signaling axis may be useful for the treatment of COPD.

"Antibody" is used in the broadest sense and encompasses various antibody structures, including but not limited to monoclonal antibodies, polyclonal antibodies, multispecific antibodies (e.g., bispecific antibodies), and antibody fragments so long as they exhibit the desired antigen-binding activity.

It is particularly contemplated that anti-IL33 antibodies or antibody variants, i.e., antibodies that bind specifically to and inhibit/neutralize IL-33, are effective in the treatment of COPD. In some instances, the antibody may be monoclonal (MAbs); recombinant; chimeric; humanized, such as complementarity-determining region (CDR)-grafted; human; antibody variants, including single chain; and/or bispecific; as well as fragments; variants; or derivatives thereof. Antibody fragments include those portions of the antibody that bind to an epitope on the polypeptide of interest. Examples of such fragments include Fab and F(ab') fragments generated by enzymatic cleavage of full-length antibodies. Other binding fragments include those generated by recombinant DNA techniques, such as the expression of recombinant plasmids containing nucleic acid sequences encoding antibody variable regions.

Monoclonal antibodies may be modified for use as therapeutics or diagnostics. "Monoclonal antibody" or "monoclonal antibody composition" as used herein refers to polypeptides, including antibodies, bispecific antibodies, etc., that have substantially identical amino acid sequence or are derived from the same genetic source. This term also includes preparations of antibody molecules of single molecular composition. A monoclonal antibody composition displays a single binding specificity and affinity for a particular epitope.

One instance is a "chimeric" antibody in which a portion of the heavy (H) and/or light (L) chain is identical with or homologous to a corresponding sequence in antibodies derived from a particular species or belonging to a particular antibody class or subclass, while the remainder of the chain(s) is/are identical with or homologous to a corresponding sequence in antibodies derived from another species or belonging to another antibody class or subclass. Also included are fragments of such antibodies, so long as they exhibit the desired biological activity. See U.S. Pat. No. 4,816,567; Morrison et al., 1985, Proc. Natl. Acad. Sci. 81:6851-55.

In another instance, a monoclonal antibody is a "humanized" antibody. Methods for humanizing non-human antibodies are well known in the art. See U.S. Pat. Nos. 5,585,089 and 5,693,762. Generally, a humanized antibody has one or more amino acid residues introduced into it from a source that is non-human. Humanization can be performed, for example, using methods described in the art (Jones et al., 1986, Nature 321 :522-25; Riechmann et al., 1998, Nature 332:323-27; Verhoeyen et al., 1988, Science 239:1534-36), by substituting at least a portion of a rodent complementarity-determining region for the corresponding regions of a human antibody.

Also contemplated are human antibodies and antibody variants (including antibody fragments) that bind to IL-33. Using transgenic animals (e.g., mice) that are capable of producing a repertoire of human antibodies in the absence of endogenous immunoglobulin production such antibodies are produced by immunization with a polypeptide antigen (i.e., having at least 6 contiguous amino acids), optionally conjugated to a carrier. See, e.g., Jakobovits et al., 1993, Proc. Natl. Acad. Sci. 90:2551-55; Jakobovits et al., 1993, Nature 362:255-58; Bruggermann et al., 1993, Year in Immuno. 7:33. See also PCT App. Nos. PCT/US96/05928 and PCT/US93/06926. Additional methods are described in U.S. Pat. No. 5,545,807, PCT App. Nos. PCT/US91/245 and PCT/GB89/01207, and in European Patent Nos. 54607381 and 546073A1. Human antibodies can also be produced by the expression of recombinant DNA in host cells or by expression in hybridoma cells as described herein.

Chimeric, CDR grafted, and humanized antibodies and/or antibody variants are typically produced by recombinant methods. Nucleic acids encoding the antibodies are introduced into host cells and expressed using materials and procedures described herein. In one instance, the antibodies may be produced in mammalian host cells, such as CHO cells. Monoclonal (e.g., human) antibodies may be produced by the expression of recombinant DNA in host cells or by expression in hybridoma cells as described herein.

Antibodies and antibody variants (including antibody fragments) useful in the disclosed methods may comprise:

(a) a heavy chain variable region comprising a HCDR1 having the sequence as set forth in SEQ ID NO: 1, a VHCDR2 having the sequence of SEQ ID NO: 2, a VHCDR3 having the sequence of SEQ ID NO: 3; and (b) a light chain variable region a VLCDR1 having the sequence of SEQ ID NO: 5, a VLCDR2 having the sequence of SEQ ID NO: 6, and a VLCDR3 having the sequence of SEQ ID NO: 7.

The anti-IL-33 antibody or antibody variant thereof for use in the invention comprises a heavy chain variable region (VH) domain having the sequence set forth in SEQ ID NO: 4 and a light chain variable region (VL) domain having the sequence set forth in SEQ ID NO: 8.

In some instances, the anti-IL-33 antibody is 33_640087_7B, as disclosed in WO2016/156440. 33_640087_7B, also referred to in the art as MEDI3506, is an anti-IL-33 antibody that binds to the reduced form of IL-33 (redIL-33) with high affinity. 33_640087_7B also inhibits the conversion of redIL-33 to the oxidised form (oxIL-33), which has been shown to induce signalling via RAGE and induce epithelial cell proliferation.

33_640087_7B is an exemplary anti-IL-33 antibody having : (a) a heavy chain variable region comprising a HCDR1 having the sequence as set forth in SEQ ID NO: 1, a VHCDR2 having the sequence of SEQ ID NO: 2, a VHCDR3 having the sequence of SEQ ID NO: 3; and (b) a light chain variable region a VLCDR1 having the sequence of SEQ ID NO: 5, a VLCDR2 having the sequence of SEQ ID NO: 6, and a VLCDR3 having the sequence of SEQ ID NO: 7.

33_640087_7B also comprises a VH domain having the amino acid sequence as set forth in SEQ ID NO: 4 and a VL domain having the amino acid sequence as set forth in SEQ ID NO: 8.

33_640087_7B is an IgG1 antibody, the sequence of the full length light chain and heavy chain of 33_640087_7B, including the IgG1 chain, is set forth in SEQ ID NOs: 9 and 10, respectively.

In some instances, the anti-IL-33 antibody or antibody variant thereof may have similar, or the same pharmacokinetic (pK) characteristics as 33_670087_7B in humans.

In particular, the anti-IL-33 antibody or antibody variant may have a similar, or the same, half-life in humans as 33_670087_7B. The anti-IL-33 antibody or antibody variant having a similar, or the same, half-life in humans as 33_670087_7B, when administered at a dose of 30 mg Q2W, may have a half-life of about 10 to about 20 days, about 12 to about 15 days, or of about 12.7 days. The anti-IL-33 antibody or antibody variant having a similar, or the same, half-life in humans as 33_670087_7B, when administered at a dose of 100 mg Q2W, may have a half-life of about 10 to about 20 days, about 12 to about 15 days, or of about 13.2 days. The anti-IL-33 antibody or antibody variant having a similar, or the same, half-life in humans as 33_670087_7B, when administered at a dose of 300 mg Q2W, may have a half-life of about 10 to about 20 days, about 12 to about 15 days, or of about 14.8 days.

In some instances, the IL-33 antibody or variant thereof may competitively inhibit binding of IL-33 to 33_640087-7B (as described in WO2016/156440). WO2016/156440 discloses that 33_640087_7B binds to redIL-33 with particularly high affinity and attenuates both ST-2 and RAGE-dependent IL-33 signaling. An antibody or variant thereof is said to competitively inhibit binding of a reference antibody to a given epitope if it specifically binds to that epitope to the extent that it blocks, to some degree, binding of the reference antibody to the epitope. Competitive inhibition may be determined by any method known in the art, for example, solid phase assays such as competition ELISA assays, Dissociation-Enhanced Lanthanide Fluorescent Immunoassays (DELFIA®, Perkin Elmer), and radioligand binding assays. For example, the skilled person could determine whether an antibody or variant thereof competes for binding to IL-33 by using an in vitro competitive binding assay, such as the HTRF assay described in WO2016/156440, paragraphs 881-886. For example, the skilled person could label 33_640087-7B with a donor fluorophore and mix multiple concentrations with fixed concentration samples of acceptor fluorophore labelled-redIL-33. Subsequently, the fluorescence resonance energy transfer between the donor and acceptor fluorophore within each sample can be measured to ascertain binding characteristics. To elucidate competitive binding antibody molecules, the skilled person could first mix various concentrations of a test binding molecule with a fixed concentration of the labelled 33_640087-7B antibody. A reduction in the FRET signal when the mixture is incubated with labelled IL-33 in comparison with a labelled antibody-only positive control would indicate competitive binding to IL-33. An antibody or variant thereof may be said to competitively inhibit binding of the reference antibody to a given epitope by at least 90%, at least 80%, at least 70%, at least 60%, or at least 50%.

In various instances, the anti-IL-33 antibody or antibody variant thereof is selected from human antibody, a humanized antibody, a chimeric antibody, a monoclonal antibody, a recombinant antibody, an antigen-binding antibody fragment, a single chain antibody, a monomeric antibody, a diabody, a triabody, tetrabody, a Fab fragment, an IgG1 antibody, an IgG2 antibody, an IgG3 antibody, and an IgG4 antibody. In some instances, the anti-IL-33 antibody variant may be selected from the group consisting of a diabody, a triabody, a tetrabody, a Fab fragment, single domain antibody, scFv, wherein the dose is adjusted such that the binding sites to be equimolar to those dosed by bivalent antibodies.

In some instances, the anti-IL-33 antibody or antibody variant thereof may bind to IL-33 comprising an amino acid sequence of SEQ ID NO: 11. In various instances, the anti-IL-33 antibody or antibody variant thereof may be capable of binding to a mature form of the full-length IL-33 protein comprising an amino acid sequence of SEQ ID NO: 11. In various instances, the anti-IL-33 antibody or antibody variant thereof may be capable of binding to an IL-33 protein fragment comprising amino acids 72-270, 79-270, 95-270, 99-270, 107-270, 109-270, 111-270, or 112-270 of SEQ ID NO:11.

In various instances, the anti-IL-33 antibody or antibody variant thereof may be capable of binding to the reduced (red-IL-33) and/or the oxidised (ox-IL-33) form of IL-33. In some instances, the anti-IL-33 antibody or antibody variant thereof may be capable of preferentially binding to the reduced (red-IL-33) and/or the oxidised (ox-IL-33) form of IL-33.

In various instances, the anti-IL-33 antibody or antibody variant thereof may be an inhibitory antibody, capable of inhibiting IL-33 or a fragment thereof as defined herein. In various instances, an inhibitory antibody may be capable of inhibiting the association of IL-33 or a fragment thereof with an IL-33 receptor.

## EXAMPLES

### Example 1 - Proof of mechanism for anti-interleukin-33 antibody tozorakimab: results of a phase 1 study in healthy adults and participants with chronic obstructive pulmonary disease

The alarmin cytokine interleukin (IL)-33 orchestrates inflammatory and remodelling responses following tissue damage (Scott IC et al. Sci Rep 2018;8:3363; Cohen E et al. Nat Commun 2015;6:8327; Murdaca G et al. Int J Mol Sci 2019;20:5856). Excess IL-33 plays a key role in initiating and driving chronic obstructive pulmonary disease in COPD (Allinne J et al. J Allergy Clin Immunol 2019;144:1624-37.e10; Schmitz J et al. Immunity 2005;23:479-90). Tozorakimab (MEDI3506) is a human immunoglobulin G1 monoclonal antibody that specifically and potently targets IL-33. This first-in-human study (NCT03096795) evaluated the safety, tolerability, pharmacokinetics and immunogenicity of tozorakimab. This report details proof of mechanism for tozorakimab from this study.

### Methods

The three-part, phase 1, randomized, blinded, placebo-controlled study was conducted between May 15, 2017 and September 30, 2019 across two centres in the UK. In all cohorts, participants were randomized 3:1 to receive tozorakimab:placebo. This report presents data from parts 1 and 2.

Part 1 eligible participants with a history of mild atopy and sensitivity to house dust mites (HDM), received single ascending doses (SADs) of either 300 mg intravenous (IV) or 1 mg, 3 mg, 10 mg, 30 mg, 100 mg or 300 mg subcutaneous (SC) tozorakimab or placebo. Part 2 eligible participants with Global Initiative for Chronic Obstructive Lung Disease (GOLD) grade I-II COPD received multiple ascending doses (MADs) of 30 mg, 100 mg or 300 mg SC tozorakimab or placebo.

Pharmacodynamics (PD) were assessed as exploratory outcomes. Target engagement was measured with ultra-selective assays for IL-33 forms in serum (all cohorts), and in local airway nasal mucosal lining fluid (MLF) samples by non-invasive nasosorption (MAD cohort). Serum levels of sST2 were also measured. After IL-33 challenge, interferon gamma (IFN-$\gamma$) was measured ex vivo with whole blood assays (SAD cohorts). Multiplex immunoassays (Meso Scale Discovery) were used to explore the PD effects of tozorakimab on inflammatory mediators (MAD cohort). Eosinophil levels were measured in whole blood (MAD cohort).

### Results

Patient baseline demographics were as follows:

| | SAD cohorts (n = 56) | | MAD cohorts (n = 24) | | Japanese cohort (n = 8) | |
|---|---|---|---|---|---|---|
| | Tozo n = 42 | Placebo n = 14 | Tozo n = 18 | Placebo n = 6 | Tozo n = 6 | Placebo n = 2 |
| Male sex, n (%) | 41 (97.6) | 14 (100.0) | 10 (55.6) | 5 (83.3) | 6 (100.0) | 2 (100.0) |
| Age, years, mean (SD) | 37.2 (8.9) | 37.6 (9.7) | 64.2 (5.6) | 66.2 (4.1) | 32.2 (9.6) | 31.5 (9.2) |
| Race, n (%) | | | | | | |
| Asian | 6 (14.3) | 3 (21.4) | 0 (0.0) | 0 (0.0) | 6 (100.0) | 2 (100.0) |
| Black or African American | 1 (2.4) | 1 (7.1) | 0 (0.0) | 0 (0.0) | 0 (0.0) | 0 (0.0) |
| White | 30 (71.4) | 8 (57.1) | 18 (100.0) | 6 (100.0) | 0 (0.0) | 0 (0.0) |
| Other | 5 (11.9) | 1 (7.1) | 0 (0.0) | 0 (0.0) | 0 (0.0) | 0 (0.0) |
| Body mass index, kg/m$^2$, mean (SD) | 24.7 (2.7) | 26.9 (2.4) | 28.0 (4.8) | 25.6 (4.0) | 23.8 (2.4) | 21.0 (1.2) |

In total, 56 participants were enrolled and randomized in the SAD cohorts (healthy adults with mild atopy and sensitivity to HDM): 42 in the tozorakimab-treated group and 14 in the placebo-treated group. 24 patients were enrolled and randomised in the MAD cohorts (adults with GOLD grade I-II COPD): 18 in the tozorakimab-treated group and six in the placebo-treated group).

*Target engagement biomarker studies (exploratory endpoint)*

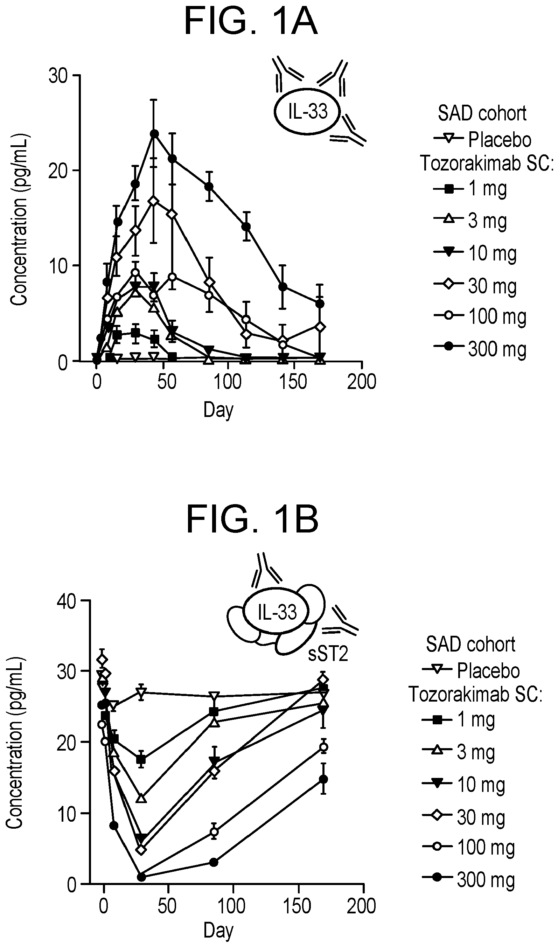

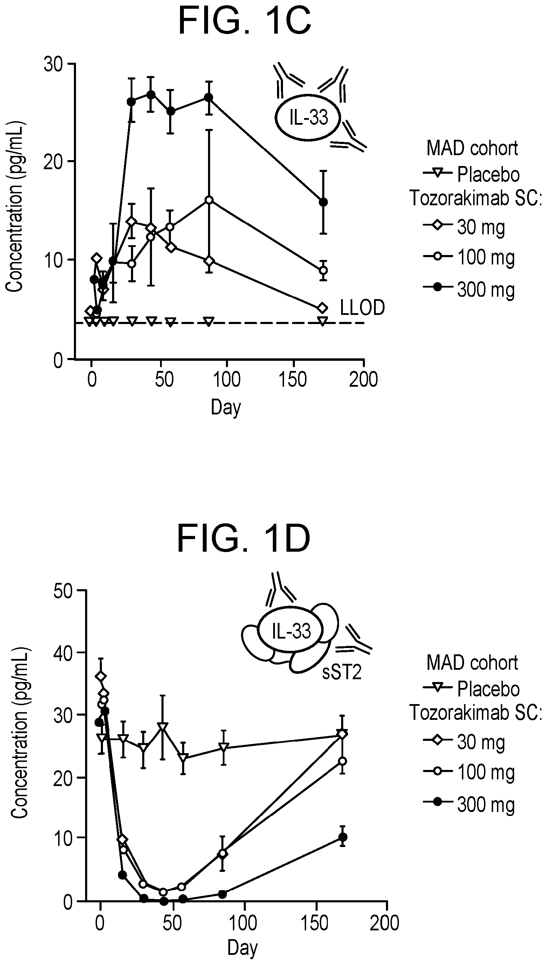

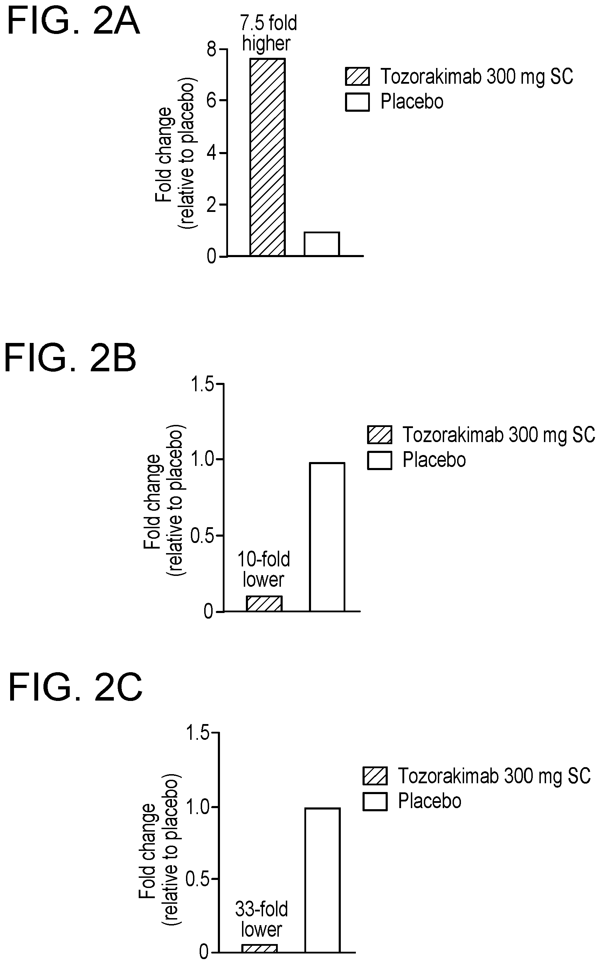

Tozorakimab target engagement was demonstrated in serum (Figure 1) and local airway nasal MLF (Figure 2). In serum, tozorakimab increased levels of IL-33/tozorakimab complex compared with placebo across all cohorts (Figure 1A [SAD cohort] and 1C [MAD cohort]), whereas levels of the endogenous IL-33/sST2 complex were decreased in all cohorts (Figure 1B [SAD cohort] and 1D [MAD cohort]). Tozorakimab did not significantly impact serum levels of total sST2 compared with placebo, at any dose level.

In local airway nasal mucosal lining fluid (MLF), tozorakimab increased levels of IL-33/tozorakimab complex compared with placebo (MAD cohort) (Figure 2A) and decreased levels of both reduced and oxidized forms of IL-33 (Figure 2B and 2C).

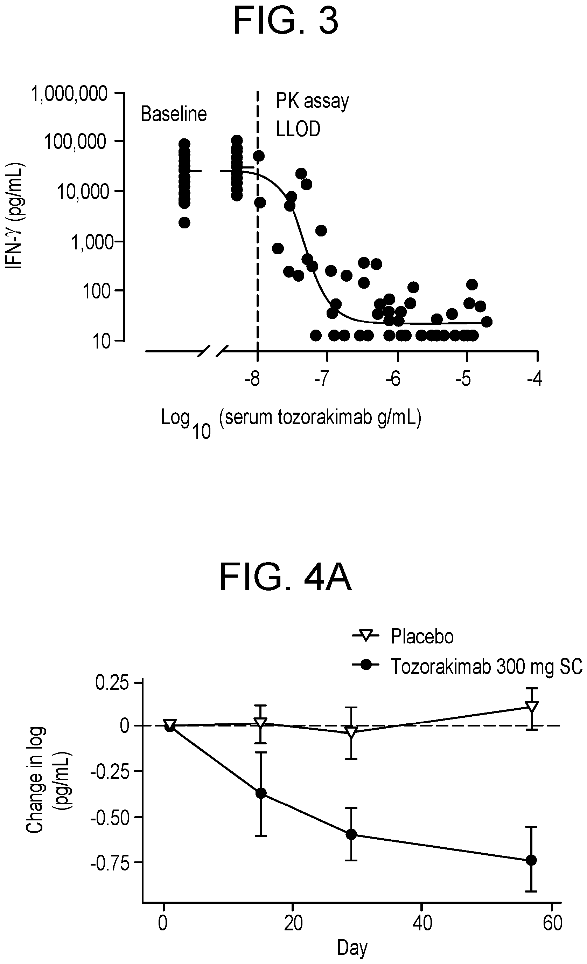

Higher levels of tozorakimab in circulation correlate with lover levels of induced IFN-$\gamma$ (Figure 3).

*Pharmacodynamic biomarker studies (exploratory endpoint)*

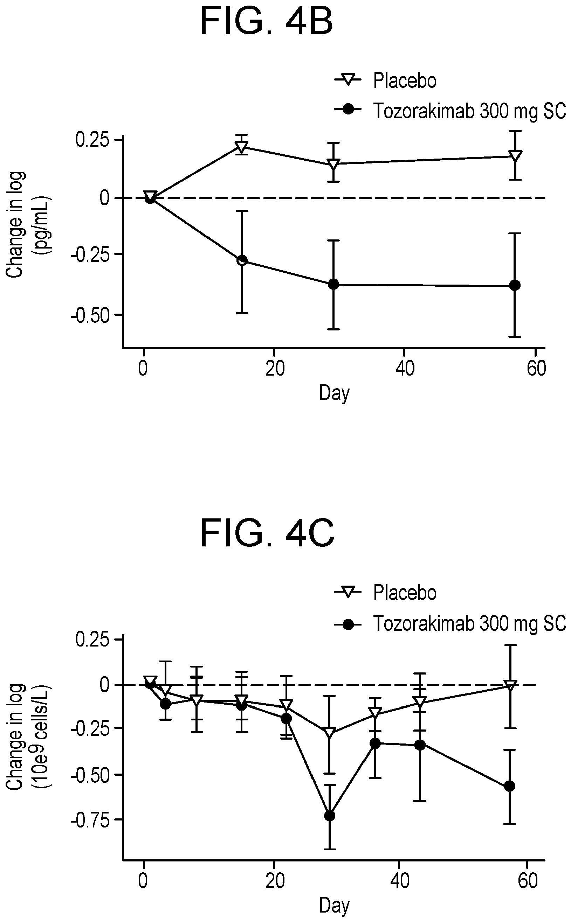

Tozorakimab (300 mg SC) significantly reduced serum IL-5 and IL-13 levels compared with placebo (Figure 4A and 4B). Moreover, tozorakimab significantly reduced blood eosinophil levels (Figure 4C) and these reductions correlated with reductions in serum levels of IL-5 (repeated measures correlation [r] = 0.64; 95% confidence interval [CI]: 0.23-0.86, p = 0.0034) and IL-13 (r = 0.75; 95% CI: 0.43-0.91, p = 0.00019).

*Conclusions*

These data show proof of mechanism through target engagement and identification of PD biomarkers for tozorakimab in a first-in-human study (NCT03096795) in patients with COPD. Target engagement was demonstrated in the circulation and locally in the airway using pioneering nasosorption sampling. The results support the entry of tozorakimab into phase 2 and phase 3 studies. Phase 2 (NCT04631016) and phase 3 studies (NCT05166889 and NCT05158387) are currently underway to investigate the safety and efficacy of tozorakimab for the treatment of COPD.

**Example 2 - A Phase II, Randomized, Double-blind, Placebo-controlled Study to Assess the Efficacy, Safety and Tolerability of MEDI3506 in Participants with Moderate to Severe Chronic Obstructive Pulmonary Disease and Chronic Bronchitis (FRONTIER 4)**

The present example describes a Phase 2 randomized, double-blinded, placebo-controlled, parallel-group, proof-of-concept study to evaluate the efficacy, safety, PK, and immunogenicity of MEDI3506 in adult subjects with moderate or severe COPD receiving Standard of Care (dual or triple therapy) as maintenance therapy. Participants also have a history of $\geq$ 1 moderate or severe acute exacerbation in the previous 12 months while on stable background treatment, and moderate to severe chronic bronchitis, with active sputum and cough symptoms

MEDI3506 (also referred to herein as 33_640087_7B) is a human IgG1 mAb that binds to human IL-33. MEDI3506 binds full length and mature forms of human IL-33 with exceptionally high affinity and prevents IL-33 binding to soluble (sST2) and membrane-bound forms of ST2 (also known as IL-1RL1) receptor.

Several clinical and non-clinical studies point to the IL-33/ST2 signalling axis playing a key role in the pathogenesis of COPD. Thus, blocking this signalling pathway could be of therapeutic benefit in COPD.

Participants must be on stable doses of dual therapy (ICS + LABA, or LABA + LAMA) or triple therapy (ICS + LABA + LAMA) for $\geq$ 3 months prior to enrolment and should remain so during the study. There should have been no change in maintenance COPD treatment after a previous exacerbation prior to entering into the study.

Participants will be randomised into treatment groups which will receive 600 mg MEDI3506 SC (20 mM L-histidine/L-histidine-hydrochloride, 220 mM L-arginine-hydrochloride, 0.03% (w/v) polysorbate 80, pH 5.5), or volume-matched placebo SC (referred to collectively "investigational products"); in a 1:1 ratio overall every 4 weeks (Q4W) for a total of 7 doses with the final dose at Week 24.

Participants will be enrolled in this study for at least a 4-week screening/run-in period, a 24-week intervention period (or "treatment window") during which they receive 7 doses SC Q4W, a 4 week additional period, and an 8-week follow-up period. The investigational schema laid out in Table 2

The primary estimand is as follows: The difference in mean change from baseline in FEV1 at Week 12 (MEDI3506 - placebo) will be estimated using a repeated measures mixed effects analysis of covariance model, for the ITT population.

This will include all available data from all visits up to and including Week 12, irrespective of whether the participant discontinued study intervention or received reliever therapy. The model will include fixed effects for baseline, eosinophil strata, background medication strata, visit, study intervention, and the baseline by visit, and study intervention by visit interactions. An unstructured covariance matrix will be used to describe the correlations between observations on a participant between visits.

A similar approach will be taken for the analysis of cough VAS, BCSS, CASA-Q, SGRQ, and reliever medication. Data may be log-transformed prior to analysis where appropriate. Change from baseline in objective cough parameters and oscillometry parameters at Week 12 will be analyzed using analysis of covariance. Analysis of time to event and annualized rate of event data will include available data (up to Week 28 where this is available) for all participants. Time to event endpoints will be analysed

**Screening procedures**

Participants should meet the following criteria:

1. Participants must be 40 to 75 years of age inclusive.
2. Participants who are current or ex-smokers with a tobacco history of ≥ 10 pack-years.
3. Participants who are up-to-date on pneumococcus and influenza vaccines as per local treatment guidelines.
4. Participants who have a documented history of COPD for at least 1 year.
5. Participants who have a post-BD FEV1/FVC < 0.70 and a post-BD FEV1 > 30% and < 80% predicted normal value at screening. Centralized spirometry will be used for this criteria assessment.
6. Participants who have a physician confirmed participant history of chronic bronchitis as defined as presence of cough and sputum on most days for ≥ 3 mos/yr in at least the 2 year period immediately prior to SV1 (screening).
7. Participants who have an average BCSS score of ≥ 2 in cough and ≥ 2 in sputum domains assessed over the 14 days preceding SV3.
8. Participants who have a documented stable regimen of dual therapy or triple therapy for ≥ 3 months prior to enrolment; there should have been no change in treatment after the previous exacerbation prior to entering into the study. Where dual therapy consists of ICS + LABA or LABA + LAMA, and triple therapy consists of ICS + LABA + LAMA. Both dual and triple therapy may be in the form of separate inhalers of fixed dose combination inhalers but may not be in nebulized form.
9. Participants who have a documented history of ≥ 1 moderate or severe AECOPD requiring systemic corticosteroids and/or antibiotics for at least 3 days duration (or 1 injection of depot formulation), or hospitalization for reason of AECOPD in the previous 12 months prior to screening.
10. Participants who are clinically stable and free from an exacerbation of COPD for 1 month prior to SV1 (screening) and prior to Day 1.
11. Body mass index within the range 19 to 35 kg/m2 (inclusive).

**Randomisation and Administration**

Randomisation will occur at study visit 3 (SV3 - Day 1). Participants who continue to meet eligibility criteria will be randomised into treatment groups as described above. Blood samples, urine samples, efficacy assessments and safety assessments will be performed in order to establish baseline.

The randomization will be stratified by baseline blood eosinophils (< 300 cells/μL vs ≥ 300 cells/μL) and background medication (includes ICS vs does not include ICS).

The first investigational product (IP) administration will occur at study visit 3 (Day 1), and will comprise administering the first dose of investigational product during the treatment window. Administering 600 mg MEDI3506 will require $2 \times 2$ mL SC injections per dose. Placebo groups will be injection volume matched to the MEDI3506 groups.

At study visit 4 (Day 2), participants will return for assessment of their adherence to self-assessment efficacy reporting procedures, and safety assessments. Procedures are outlined in Table 4.

The second investigational product administration will occur at study visit 6 (Day 29 ± 3).

The third investigational product administration will occur at study visit 7 (Day 57 ± 3).

The fourth investigational product administration will occur at study visit 8 (Day 85 ± 3).

The fifth investigational product administration will occur at study visit 9 (Day 113 ± 3).

The sixth investigational product administration will occur at study visit 10 (Day 141 ± 3).

The seventh and final investigational product administration will occur at study visit 11 (Day 169 ± 3).

**Endpoints**

The primary endpoint visit occurs at week 12, as assessed at study visit 10 (day 113 $\pm$ 4).

The primary endpoint is improvement is change from baseline to week 12 in clinic pre-BD $FEV_1$. Forced expiratory volume in 1 second is a validated and clinically important endpoint in COPD studies, and has been used extensively in trials used to support registration of add on therapy to current standard of care (dual/triple therapy) in a similar chronic bronchitis patient population (Martinez et al 2015).

Based on available data, the improvement in $FEV_1$ assumed in the sample size determination is expected to be achieved by Week 12. However, improvement in $FEV_1$ is considered important but not sufficient to meet the unmet medical need in COPD. To enable evaluation of the secondary endpoint of COPDCompEx, the treatment is continued after collection of primary endpoint data, to collect further events. The longer intervention period duration also allows the exploratory assessment of treatment effect on $FEV_1$ beyond Week 12.

The secondary endpoint is COPDCompEx at week 28. Changes in clinic pre-BD $FEV_1$ will also be assessed at week 28.

Blood samples will be collected from subjects for the assessment of biomarkers that are relevant to disease pathology and/or the mechanism of action of MEDI3506.

**Results**

The highest dose of MEDI3506 administered to subjects in this Phase 2 clinical study will be 600 mg by SC injection Q4W. This dose is predicted to have a lower exposure, in terms of maximum concentration at steady-state ($C_{max,ss}$; approximately 2.5-fold) and AUC (approximately 1.6-fold), compared with the highest dose administered (ie, single dose of 300 mg IV MEDI3506) in the Phase 1 clinical study (Study D9180C00001). A dose of 600 mg by SC injection Q4W is predicted to have a higher $C_{max,ss}$, but the same AUC compared with the highest multiple dose administered (ie, 300 mg SC Q2W) in the same study (Table 6).

The nature and severity of disease in the study population are not expected to significantly impact the overall exposure nor clearance. The published PK data for a monoclonal antibody licensed for use in AD indicate that the disease status (ie, healthy subjects vs subjects with AD) had no significant impact on exposure or clearance (Kovalenko et al, 2016). Hence, we anticipate that MEDI3506 exhibits similar PK profiles for both healthy subjects and subjects with COPD.

***Example 3 - Dose selection criteria for MEDI3506 in the treatment of COPD***

In order to select target dose a PK/PD model was generated using target engagement data from the Ph1 study (NCT03096795). More specifically, the PK/PD model was based on:

- Quantitative information of MEDI3506:Il,33 and IL33:ST2 complex in systemic circulation from phase 1. Both complex concentrations were measured using proprietary IL-33 detection reagents that specifically bind to the reduced form of IL-33 (redIL-33).
- MEDI3506 PK data from phase 1 (linear PK, half-life ($t_{1/2}$) of 17 days)

Additional preclinical information that informed dose selection includes:

- *redIL-33:ST2 signalling pathway*
- *oxIL-33:RAGE:EGFR signalling pathway*

*redIL-33:ST2 signalling pathway*

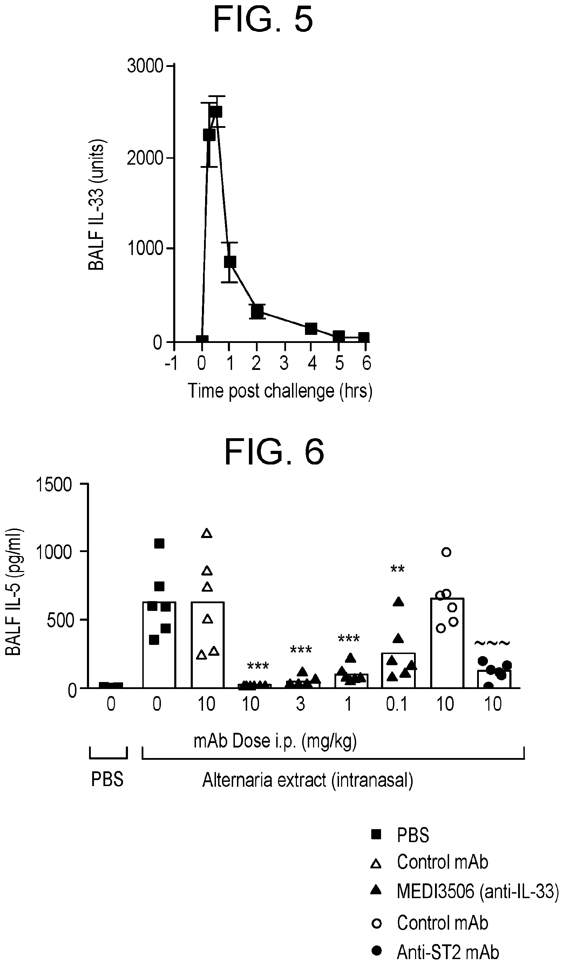

Models of Alternaria alternata (ALT) induced airway inflammation in mice have been previously described (Kouzaki et al. J. Immunol. 2011, 186: 4375-4387; Bartemes et al J Immunol, 2012, 188: 1503-1513). Endogenous IL-33 is released rapidly following ALT exposure and drives IL-33-dependent IL-5 production in the lung. Male or female wildtype or humanized IL-33 mice (6-10 weeks) were anaesthetized briefly with isofluorane and administered either 25 $\mu$g of ALT extract (Greer, Lenoir, NC) or vehicle intranasally in a total volume of 50 $\mu$l. Mice were treated intraperitoneally with MEDI3506 (0.1, 1, 2 or 10 mg/kg), isotype control IgG (NIP228) or vehicle (PBS, 10 ml/kg) at 24 hours prior to intranasal challenge with ALT. At 24 hours after challenge, mice were terminally anaesthetised with pentobarbital sodium prior to exsanguination and bronchoalveolar lavage fluid (BALF) collection. BALF was collected by lavage via tracheal cannula. BALF was centrifuged, cells counted (total cells by FACS (FacsCALIBER, BD)) and supernatant was analysed for cytokines by ELISA (Meso Scale Discovery, Rockville, MD). Differential cell counts (200 cells/slide) were performed on cytospin preparations stained with Diff-Quik (Fisher Scientific, UK). All work was carried out to UK Home Office ethical and husbandry standards under the authority of an appropriate project licence. A dose dependent inhibition of IL5 by MEDI3506 was observed in BALF with significant suppression achieved at the lowest dose tested in this study 0.1 mg/kg. At 3 mg/kg 90% of inhibition was achieved corresponding to a mean serum systemic exposure of 20 $\mu$g/mL in mouse. The results are shown in Figures 5 and 6.

*oxIL-33:RAGE:EGFR signalling pathway*

It has recently been discovered that the oxidised form of IL-33 (oxIL-33, IL-33ox or IL-33DSB) directly impairs epithelium repair responses, decreases epithelial goblet cell differentiation and proliferation, and increased mucus productions and production of mucin-associated genes, such as MUC5AC. It was found that oxIL-33 mediates pathological effects on the epithelium by binding to and signalling through a complex of RAGE and EGFR (as described in WO2021/089563).

The following MEDI3506 concentrations with respect to the oxIL-33 signalling pathway were used to inform dose selection:

- Threshold to reverse oxIL-3 3-mediated dysfunctional scratch wound closure

*Scratch Wound Closure*

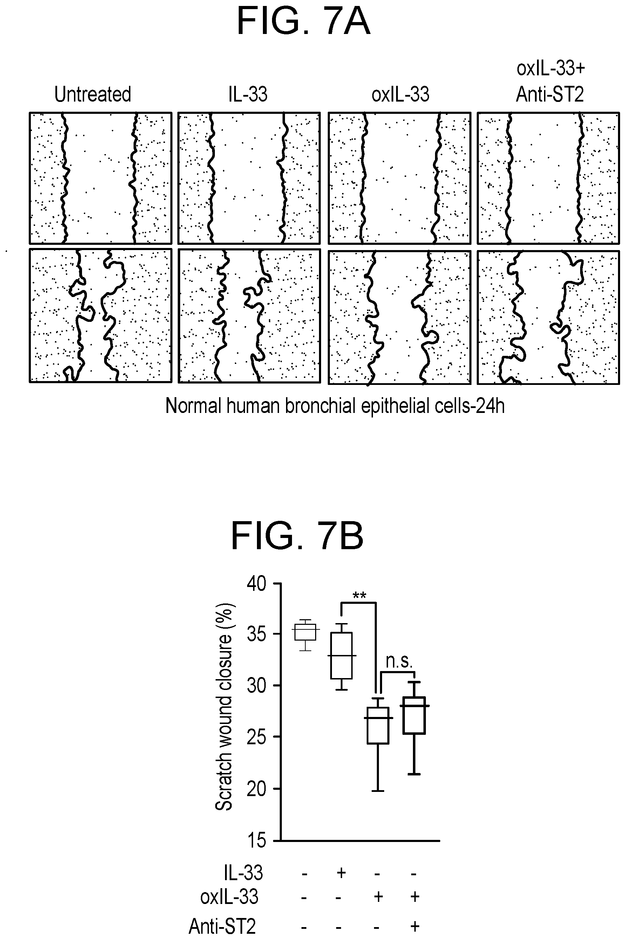

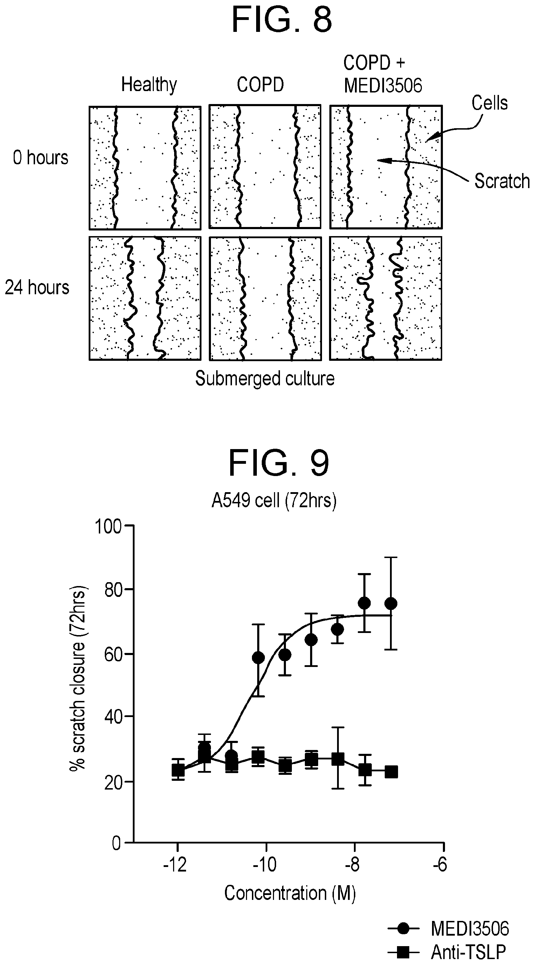

Previous experiments have shown that oxIL-33 impairs epithelial scratch wound closure in healthy human bronchial epithelial cells (Figure 7A and 7B). The impaired wound closure was not reversed with anti-ST2 antibody treatment, indicating that the pathological effect is mediated via the oxIL-33-RAGE/EGFR signalling axis. Scratch wound impairment was also seen in bronchial epithelial cells obtained from COPD subjects (Figure 8).

The concentration of MEDI3506 needed to reverse oxIL-33-mediated scratch wound closure dysfunction was calculated in A549 cells cultures.

A549s were obtained from ATCC and cultured in RPMI GlutaMax medium supplemented with 1% Penicillin/Streptomycin and 10% FBS. Cells were harvested with accutase (PAA, #L1 1-007) and seeded into 96 well plates at $5\times10^5/100$ $\mu$l and incubated at 37°C, 5% $CO_2$ for 6-8 hours. The wells were then washed twice with 100 $\mu$l of PBS before addition of 100 $\mu$l of starve media (RPMI GlutaMax medium supplemented with 1% Penicillin/Streptomycin) and incubated at 37°C, 5% $CO_2$ for 18-24 hours. Using a WoundMakerTM (Essen Bioscience), cells were scratched and then wells were washed 2x with 200 $\mu$l of PBS before addition of RPMI GlutaMax medium supplemented with 0.1% FBS (v/v) and 1% (v/v) Penicillin/Streptomycin containing the indicated stimulations; media alone (unstimulated control), different concentrations of MEDI3506 or anti-TSLP antibody, returned to 37°C, 5% $CO_2$. Plates were placed into an IncucyteZoom for wound healing imaging and analysis over a 72 hour period. Relative Wound Density was calculated through the wound healing algorithm within the Incucyte Zoom software. Figure 9 shows the MEDI3506 dose-dependent improvement in scratch wound closure in A549 cells. Concentrations of MEDI3506 greater than 50.4 pM (or 7.26 ng/ml) were needed to achieve full response. This has been assumed to be equivalent to a concentration in the blood of 0.15 $\mu$g/mL (assuming 5 % distribution to epithelial lining fluid following sub-cutaneous administration). No effect was seen for anti-TSLP antibody.

*Integrative PK/PD model for target engagement*

An integrated popPK /PD model was established based on SAD/MAD/IV MEDI3506 systemic exposure and target engagement (TE) clinical data from the MEDI3506 Ph1 study. TE information used were systemic II,33-MEDI3506 complex formation and IL33-ST2 reduced levels from FTIM.

Population and doses covered in the model include:

- healthy subjects with mild atopy after single sub-cutaneous (SC) doses (1 to 300 mg SC) and 300 mg intravenous (IV)
- Mild COPD patients receiving multiple doses of 30, 100 and 300 mg SC

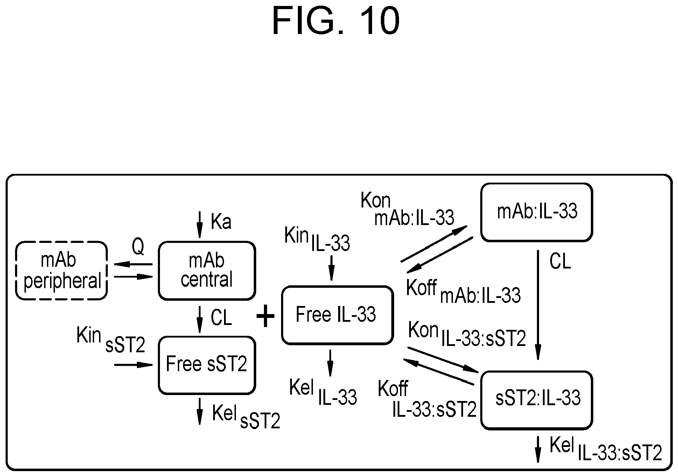

The model structure has four compartments defined, and is shown in Figure 10.

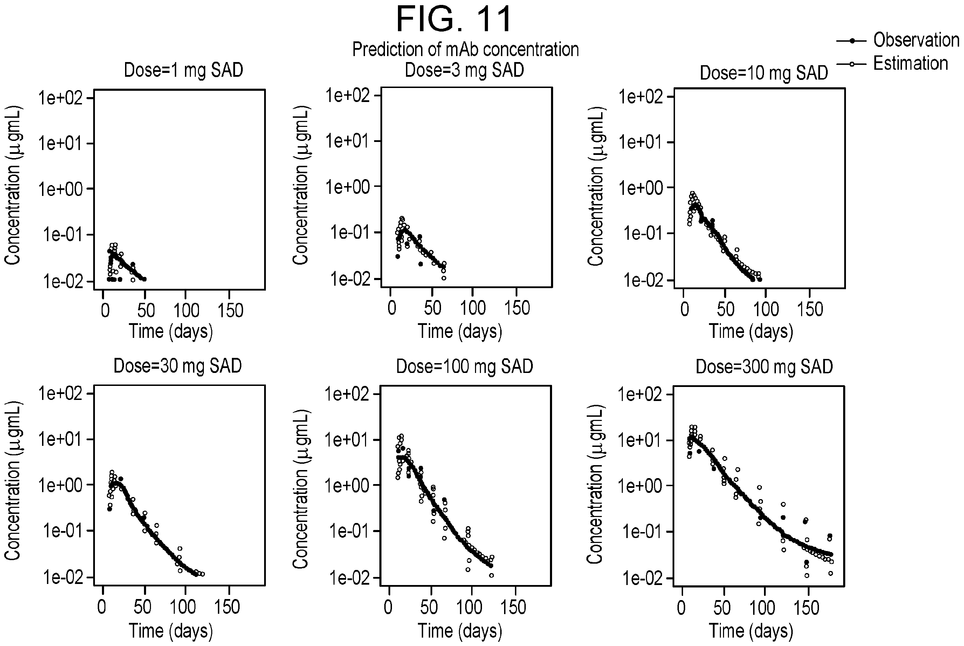

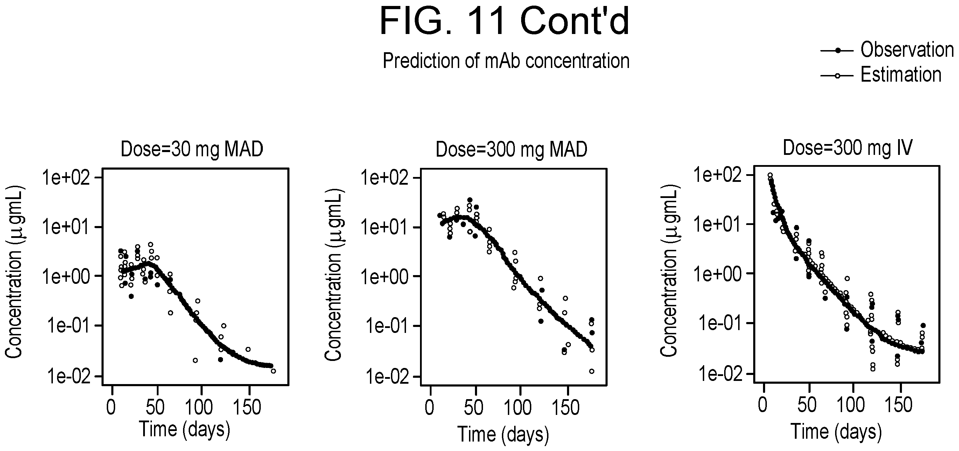

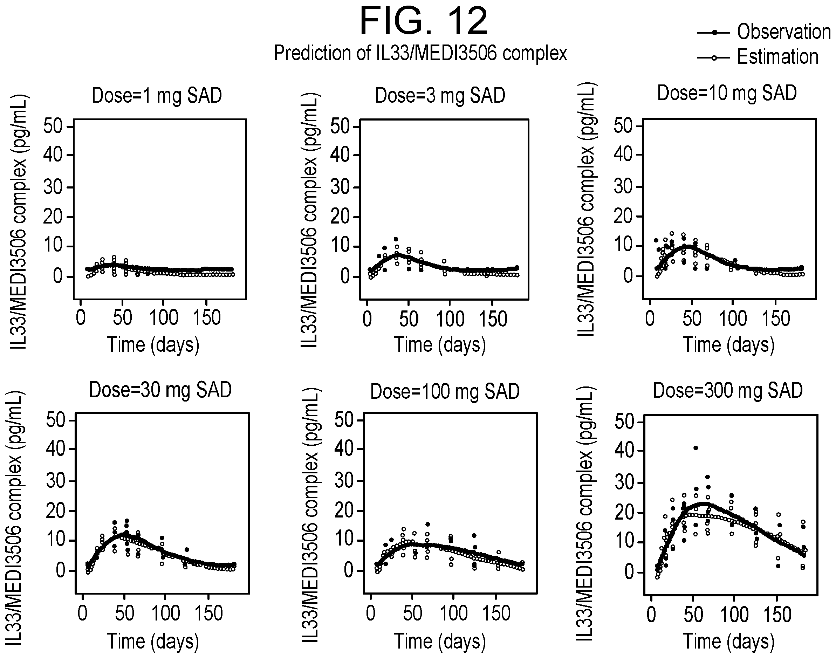

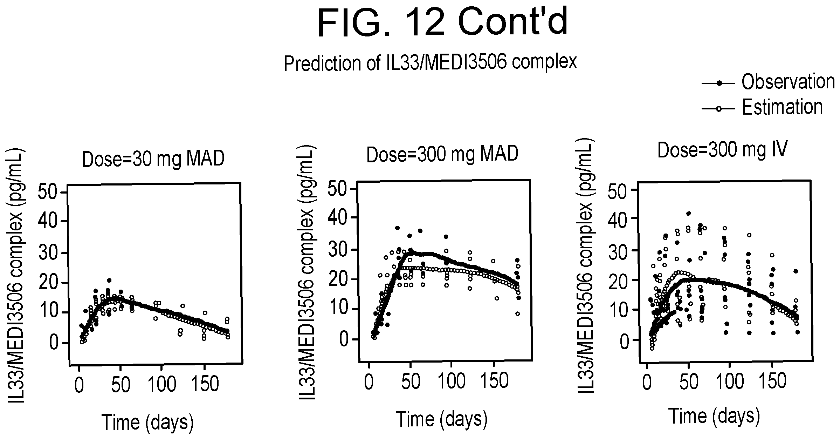

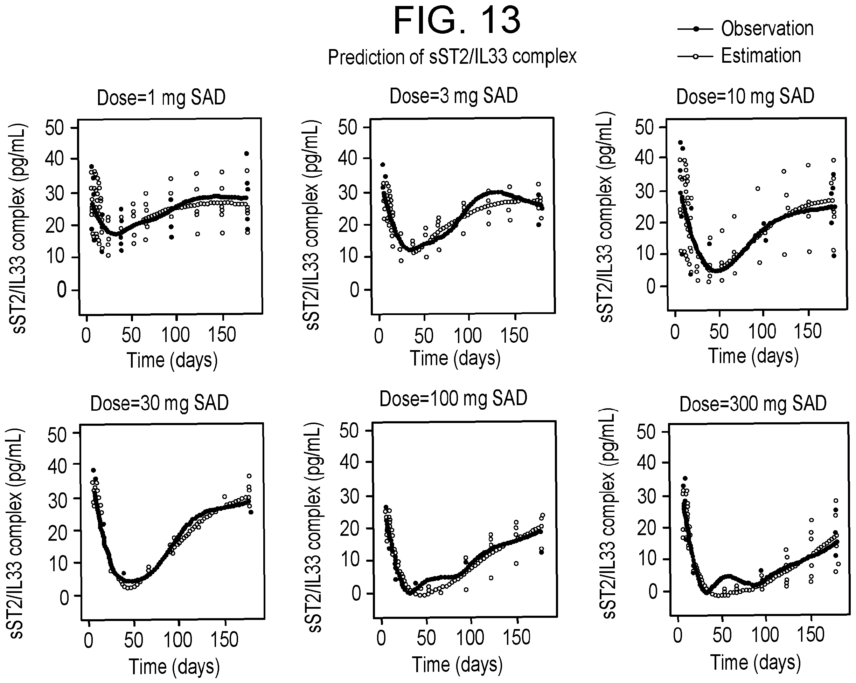

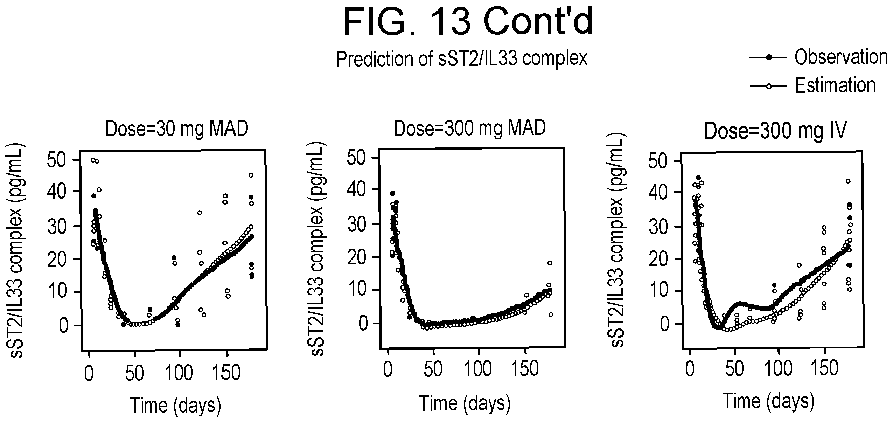

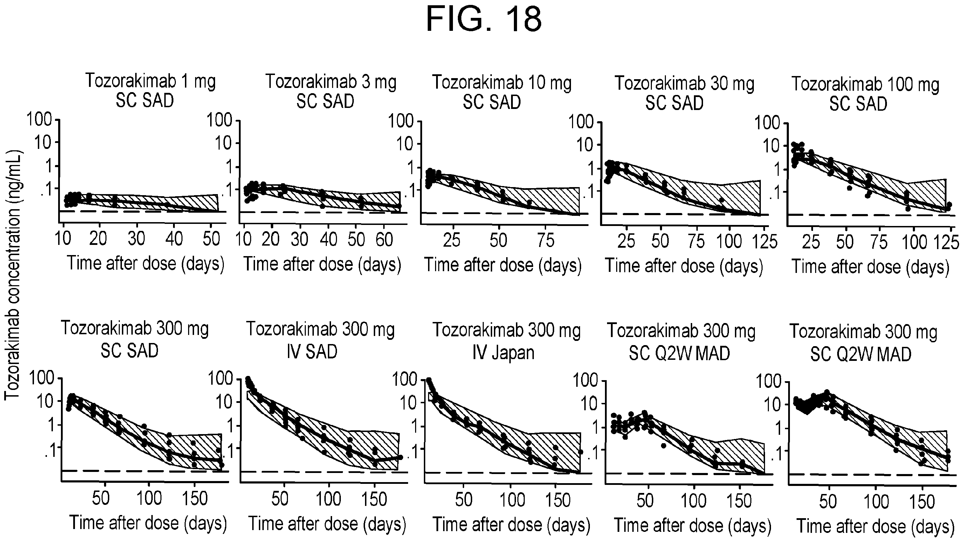

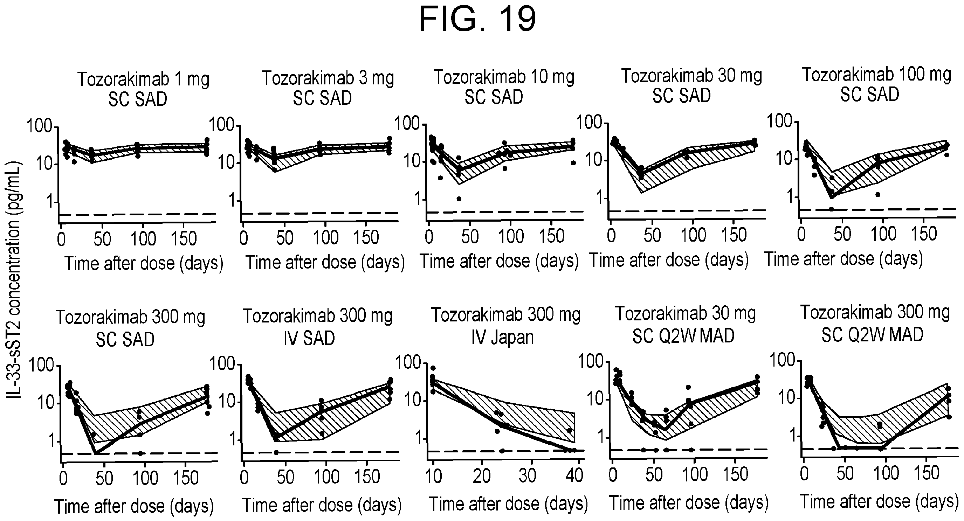

The PKPD model reliably describes observed PK profiles of MEDI3506, the MEDI3506:IL33 complex formation and IL33:ST2 dose dependent inhibition in the blood (Figures 11, 12 and 13). Figures 18 and 19 show a different presentation of Figures 11 and 13, respectively. The solid lines represent the medians of the observations. The shaded areas represent the 95% confidence intervals of the medians predicted by the model. The dashed lines represent the LLOQ values for tozorakimab (0.01 ng/ml) and IL-33:sST2 (0.5 pg/ml), respectively.

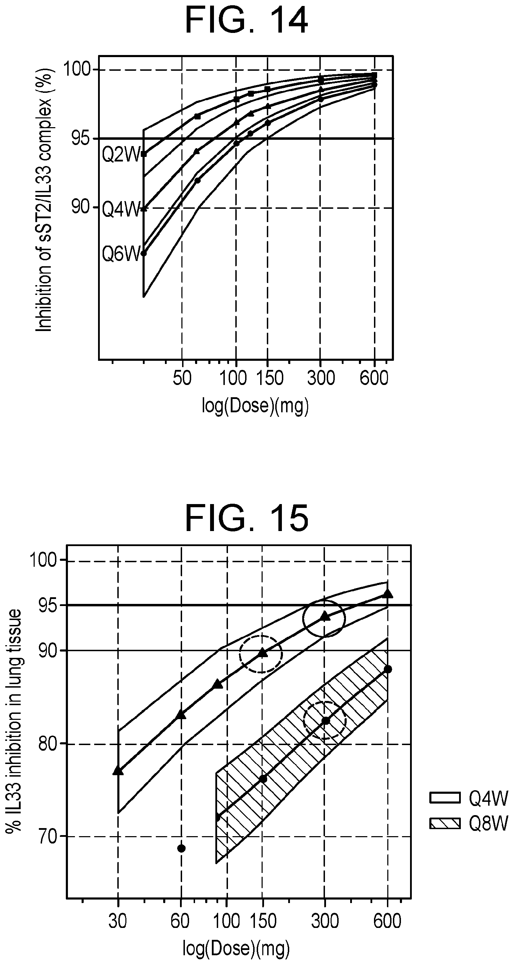

The dose response in ST2:IL33 complex inhibition for MEDI3506 in blood at trough for Q2W, Q4W and Q6W dosing regiments is shown in Figure 14.

The PK/PD model for IL-33/sST2 complex inhibition in blood has been further transformed to predict the IL-33 inhibition in the lung tissue (assumptions: blood:tissue partition coefficient of 14%, and that IL-33 levels in the lung are two-fold higher than in blood).

The %IL-33 inhibition in lung tissue at trough for the Q4W and Q8W dosing frequencies is shown in Figure 15.

Dose selection:

Almost 95% target inhibition in lung tissue at trough is predicted for 300mg dose Q4W dosing frequency. MEDI3506 300mg Q8W is predicted to achieve TE in the lung of greater than 80%, which means that sustained inhibition of IL-33 in the lungs of patients may be achievable using a longer but more convenient dosing interval for patients (Figure 15).

Based on Ph1 PK data and other input parameters, MEDI3506 serum concentrations were modelled for 300 mg Q4W and 300 mg Q8W. Both of these regimens are predicted to have a trough concentration higher than the amount predicted from the *Alternaria* humanised IL-33 mouse model required to achieve 60% inhibition (Figure 16). The *Alternaria* model is more representative of an acute effect caused in exacerbations or viral infections. As such, the dose projection using this cut off is likely to be sufficient to achieve an efficacious dose for human chronic IL-33-mediated diseases, such as COPD. Both regimens are also predicted to have trough concentrations higher than the threshold amount identified by the scratch wound model to inhibit pathological signalling via the oxIL-33:RAGE/EGFR signalling axis (Figure 17).

***Example** 4 - A Phase **III,** Multicentre, Randomised, Double-blind, Chronic-dosing, Parallel-group, Placebo-controlled Study to Evaluate the Efficacy and Safety of Two Dose Regimens of **MEDI3506** in Participants with **Symptomatic** Chronic Obstructive **Pulmonary** Disease (COPD) with **a** History **of COPD Exacerbations***

*Overall Design*

The purpose of this Phase III study is to evaluate the efficacy and safety of MEDI3506 300 mg every 8 weeks (Q8W) and 300 mg every 4 weeks (Q4W) dose regimens administered subcutaneously (SC) in adult participants with symptomatic COPD and history of $\geq 2$ moderate or $\geq 1$ severe exacerbation of COPD in the previous 12 months. Participants should be receiving optimised treatment with maintenance inhaled therapy (ICS/LABA/LAMA triple therapy, or dual therapy if triple is not indicated or contraindicated) in stable doses throughout at least 3 months prior to enrolment.

The study will randomise approximately 1272 participants, stratified by region, maintenance inhaled therapy (dual vs triple), and smoking status (current vs former). The study will include former and current smokers. Participants will continue the same COPD maintenance therapy throughout the duration of the study.

The study will consist of an at least 2-week screening period, a 52-week treatment period (with the site visits and IP administrations every 4 weeks), and an 8 week post treatment follow-up period.

Key Primary and Secondary objectives and endpoints are described in the following table:

| Objective | Endpoint/Estimand |
|---|---|
| **Primary** | |
| **Objective** | **Endpoint/Estimand** |
| To evaluate the effect of 2 dose regimens of MEDI3506 as add on to SoC compared with SoC plus placebo on the rate of moderate to severe COPD exacerbations in former smokers. | Endpoint: annualised rate of moderate to severe COPD exacerbations. Summary measure: rate ratio. Population: full analysis set, former smokers. Participants who are former smokers with symptomatic COPD and a history of exacerbations, on optimised treatment with maintenance inhaled therapy. Intercurrent events: treatment policy. All data up to Week 52 will be included regardless of IP discontinuation or changes in maintenance inhaled therapy. Supportive analysis: while-on-treatment [c]. |

(continued)

| Key secondary | |
|---|---|
| To evaluate the effect of 2 dose regimens of MEDI3506 as add on to SoC compared with SoC plus placebo on the rate of moderate to severe COPD exacerbation in former and current smokers. | Endpoint: annualised rate of moderate to severe COPD exacerbations.<br>Summary measure: rate ratio.<br>Population: full analysis set, former or current smokers. Participants who are former smokers or current smokers with symptomatic COPD and history of exacerbations, on optimised treatment with maintenance inhaled therapy.<br>Intercurrent events: treatment policy. |
| To evaluate the effect of 2 dose regimens of MEDI3506 as add on to SoC compared with SoC plus placebo on time to moderate to severe COPD exacerbations. | Endpoint: time to first moderate to severe COPD exacerbation.<br>Summary measure: hazard ratio.<br>Population: full analysis set, former smokers.<br>Intercurrent events: treatment policy. |
| To evaluate the effect of 2 dose regimens of MEDI3506 as add on to SoC compared with SoC plus placebo on change in pre-BD lung function. | US key secondary<br>Endpoint: change from baseline in pre-BD, pre-dose trough $FEV_1$ (mL) at Week 52.<br>Summary measure: difference in mean change from baseline.<br>Population: full analysis set, former smokers. Intercurrent events: treatment policy.<br>Ex-US key secondary<br>Endpoint: change from baseline in pre-BD, pre-dose trough $FEV_1$ (mL) over 52 weeks.<br>Summary measure: difference in mean change from baseline.<br>Population: full analysis set, former smokers. Intercurrent events: treatment policy. |
| To evaluate the effect of 2 dose regimens of MEDI3506 as add on to SoC compared with SoC plus placebo on respiratory symptoms. | US key secondary<br>Endpoint: proportion of participants achieving MCID in E-RS: COPD total score (percentage of participants with a decrease in E-RS:COPD total score of $\geq$ 2 points from baseline at Week 52).<br>Summary measure: odds ratio.<br>Population: full analysis set, former smokers. Intercurrent events: composite. |
| | Ex-US key secondary<br>Endpoint: change from baseline in E-RS:COPD total score over 52 weeks.<br>Summary measure: Difference in mean change from baseline.<br>Population: full analysis set, former smokers. Intercurrent events: treatment policy. |

(continued)

| Key secondary | |
|---|---|
| To evaluate the effect of 2 dose regimens of MEDI3506 as add on to SoC compared with SoC plus placebo on respiratory health status/health-related quality of life. | US key secondary<br>Endpoint: proportion of participants achieving MCID in SGRQ score (percentage of participants with a decrease in SGRQ total score of $\geq$ 4 points from baseline at Week 52).<br>Summary measure: odds ratio.<br>Population: full analysis set, former smokers. Intercurrent events: composite. |
| | Ex-US key secondary<br>Endpoint: change from baseline in SGRQ total score over 52 weeks.<br>Summary measure: difference in mean change from baseline.<br>Population: full analysis set, former smokers. Intercurrent events: treatment policy. |
| **Secondary** | |
| To evaluate the effect of 2 dose regimens of MEDI3506 as add on to SoC compared with SoC plus placebo on severe COPD exacerbations. | Time to first severe COPD exacerbation [b].<br><br>Annualised rate of severe COPD exacerbations. |
| To further evaluate the effect of 2 dose regimens of MEDI3506 as add on to SoC compared with SoC plus placebo on COPD health status/health-related quality of life. | Change from baseline in CAT total score at Week 52.<br>Proportion of participants achieving MCID in CAT score (percentage of participants with a decrease in CAT total score of > 2 points from baseline at Week 52). |
| To evaluate the effect of 2 dose regimens of MEDI3506 as add on to SoC compared with SoC plus placebo on overall and COPD-related HRU. | Proportion of participants having $\geq$ 1 HRU type.<br>Annualised rate of hospitalisations.<br>Annualised rate of ER/ED visits.<br>Annualised rate of hospitalisations and ER/ED visits combined.<br>Number of days in hospital, measured by total number of days in hospital, general ward, ICU, CCU, and any other HRU, if there is sufficient data.<br>Annualised rate of unscheduled visits/tests/procedures, including unscheduled visits to study sites due to COPD.<br>Rate of hospital readmissions. |
| To evaluate the effect of 2 dose regimens of MEDI3506 as add on to SoC compared with SoC plus placebo on daily rescue medication use. | Change from baseline (difference in mean number of puffs/day) in rescue medication use over 52 weeks. |
| AE = adverse event; BD = bronchodilator; CAT = COPD Assessment Test; CCU= critical care unit; COPD = chronic obstructive pulmonary disease; ECG = electrocardiogram; ED = emergency department; ER = emergency room; E-RS:COPD = Evaluating Respiratory Symptoms in COPD; $FEV_1$ = forced expiratory volume in one second; HRU = healthcare resource utilisation; ICU = intensive care unit; IP = investigational product; MCID = minimal clinically important difference; SGRQ = St. George's Respiratory Questionnaire; SoC = standard of care. | |

*Type of Participants and Disease Characteristics*

1 Documented diagnosis of COPD for at least one year prior to enrolment.

2 Post-BD $FEV_1$/FVC < 0.70 and post-BD $FEV_1$ >20% of predicted normal value (as assessed by central spirometry at screening).

3 Documented history of ≥ 2 moderate or ≥ 1 severe COPD exacerbations within 12 months prior to enrolment:

(a) An exacerbation is considered moderate if it required treatment with systemic corticosteroids and/or antibiotics, and severe if it required hospitalisation. Note: hospitalisation is defined as an inpatient admission ≥ 24 hours in the hospital, in an observation area, emergency department, or other equivalent healthcare facility depending on the country and healthcare system.

(b) At least one qualifying exacerbation should have been treated with systemic corticosteroids.

(c) Events treated with antibiotics alone qualify as a moderate exacerbation only when antibiotic was specifically prescribed for worsening of COPD symptoms.

(d) Previous exacerbations should be confirmed to have occurred while the participant was on stable dual or triple (ICS/LABA/LAMA) maintenance inhaled therapy for COPD and not as a result of a gap or step down in the treatment.

(e) At least one qualifying exacerbation should have occurred while on the most recent stable uninterrupted therapy prior to enrolment.

4 Documented optimised treatment with COPD maintenance therapy (ICS/LABA/LAMA triple therapy, or dual therapy if triple is not indicated or contraindicated) and at a stable dose for at least 3 months prior to enrolment.

5 Smoking history of ≥ 10 pack-years:

(a) Former smokers will be defined as participants who are currently not smoking and with smoking cessation ≥ 6 months prior to screening with an intention to quit permanently.

(b) Current smokers will be defined as participants who are currently smoking tobacco (at least one cigarette per day on average during the past 7 days) and are not currently participating in smoking cessation.

(c) Electronic cigarette (e-cigarette) use does not contribute to the pack-year count for eligibility.

6 CAT total score ≥ 10, with each of the phlegm (sputum) and cough items with a score ≥ 2, at both screening and randomisation.

7 At least 70% daily PRO completion during the entire screening period, with at least 50% daily PRO completion in the 14-day period prior to randomisation.

8 At least 70% compliance with COPD maintenance inhaled therapy (defined as taking COPD maintenance inhaled medication as scheduled for the day) during the entire screening period.

9 Able to read and use electronic devices.

*Study Intervention*

**Investigational Products (IPs)**

| Arm Name | MEDI3506 300 mg Q8W | MEDI3506 300 mg Q4W | Placebo |
|---|---|---|---|
| Intervention Name | MEDI3506 | MEDI3506 | Placebo |
| Type | Biologic | Biologic | Placebo |
| Dosage Form | Vial | Vial | Vial |

(continued)

| Unit Dose Strength(s) | 150 mg/mL | 150 mg/mL | Not applicable |
|---|---|---|---|
| Dosage Level (s) | Dosing Q4W alternately with 300 mg MEDI3506 and placebo | 300 mg Q4W | Equivalent volume to MEDI3506 Q4W |
| Route of Administration | SC injection | SC injection | SC injection |
| IMP and NIMP | IMP | IMP | NIMP |
| Sourcing | Provided centrally by the sponsor | Provided centrally by the sponsor | Provided centrally by the sponsor |
| Packaging and Labelling | Single vial kits with a unique number that is printed on all labels within the kit (ie, the outer carton label and the label of the vial within the carton). Labels will be prepared in accordance with GMP and local regulatory requirements. | Single vial kits with a unique number that is printed on all labels within the kit (ie, the outer carton label and the label of the vial within the carton). Labels will be prepared in accordance with GMP and local regulatory requirements. | Single vial kits with a unique number that is printed on all labels within the kit (ie, the outer carton label and the label of the vial within the carton). Labels will be prepared in accordance with GMP and local regulatory requirements. |

**Rescue Medicine**

Short-acting β2-agonists (SABAs, eg, salbutamol, albuterol, terbutaline, levalbuterol), short acting muscarinic antagonists (SAMA), SABA/SAMA combination or alternative rescue medication, as per local standard of care, may be used during the study in the event of worsening of COPD symptoms.

**Maintenance Therapy**

Stable optimised maintenance inhaled therapy (ICS/LABA/LAMA triple therapy, or dual therapy if triple is not indicated or contraindicated) Any other COPD maintenance therapy (eg, xanthines, antibiotics, PDE4 inhibitors etc) doses and regimen should be stable for 3 months prior to the study and throughout the study period.

*Efficacy Assessments*

**Assessment of COPD Exacerbations**

For the purpose of the protocol, a COPD exacerbation will be defined as a worsening in the participant's usual COPD symptoms (eg, dyspnoea, sputum volume, sputum purulence, cough, wheezing, and other COPD-related symptoms and/or findings) that is beyond normal day-to-day variation, is acute in onset, lasts 2 or more days (or less if the worsening is so rapid and profound that the treating physician judges that intensification of treatment cannot be delayed), and may warrant a change in regular medication and leads to any of the following:

Use of systemic corticosteroids for at least 3 days; a single depot injectable (IM) dose of corticosteroids will be considered equivalent to a 3-day course of systemic corticosteroids.
Use of antibiotics.
An inpatient hospitalisation due to COPD (defined as an inpatient admission ≥ 24 hours in the hospital, an observation area, the emergency department, or other equivalent healthcare facility depending on the country and healthcare system).
Results in death.

An exacerbation will be considered **moderate** if it requires treatment with systemic corticosteroids and/or antibiotics and does not meet severe event criteria. An exacerbation will be considered **severe** if it results in hospitalisation or death due to COPD.
The start of an exacerbation is defined as the start date of systemic corticosteroids or antibiotic treatment or hospital admission, whichever occurs earlier, and the end date is defined as the last day of systemic corticosteroids or antibiotic treatment or hospital discharge, whichever occurs later. A single depot injectable dose of corticosteroids will be considered equivalent to a 3-day course of systemic corticosteroids. The corresponding stop date for this treatment will consequently be determined as the date of administration plus 2 days.

**Spirometry (Pre- and Post-bronchodilator Assessments)**

All spirometry is performed pre-dose.

Lung function ($FEV_1$ and FVC) will be measured by spirometry using equipment provided by a central vendor. Spirometry will be performed by the investigator or authorised delegate according to American Thoracic Society (ATS)/European Respiratory Society (ERS) guidelines (Graham et al 2019).

**Spirometry references**

The Global Lung Function Initiative equations will be used to determine the predicted normal value (PNV) and are pre-programmed into the spirometer (Quanjer et al 2012).

Forced expiratory volume in one second expressed as percent of the PNV, will be calculated as follows:

$$FEV_1\% \; of \; PNV = (FEV_1 \; measured/FEV_1PNV) \times 100$$

$FEF_{25\text{-}75\%}$ will be calculated using a similar method to $FEV_1$.

**Post-BD Spirometry**

Endpoint maximal BD will be induced using albuterol (90 μg metered dose) or salbutamol (100 μg metered dose) with or without a spacer device up to a maximum of 4 inhalations within 30 minutes ± 15 minutes of the final pre-BD spirometry measurement. Post-BD spirometry will be performed 15 to 30 minutes later. If a participant cannot tolerate 4 puffs of albuterol or salbutamol, a lower number of inhalations may be considered at the investigator's clinical judgement.

**Patient-Reported Outcomes (PRO)**

Participants will complete the following non-daily PROs in the following order: SGRQ, CAT, 5-level EuroQol-5 Dimension (EQ-5D-5L), Work Productivity and Activity Impairment-General Health (WPAI-GH), PGIS, and Patient Global Impression of Change (PGIC). Refer to the SoA (Section 1.3) for frequency of assessments.

***Exacerbations of Chronic Pulmonary Disease Tool-Patient-reported Outcome (EXACT-PRO)***

The EXACT-PRO is a 14-item PRO instrument developed to assess the frequency, severity and duration of COPD exacerbations (Jones et al 2011, Leidy et al 2011). The instrument was developed for daily, at home, administration using a handheld electronic device. Respondents are instructed to complete the diary each evening just prior to bedtime and to answer the questions while considering their experiences "today". The daily EXACT-PRO total score has a range of 0 to 100 with higher scores indicative of greater severity. Total score changes are used to identify the onset and recovery from an EXACT-PRO defined exacerbation event. In identifying event onset and recovery, the EXACT-PRO can provide information on event frequency and duration as well as event severity.

***Evaluating Respiratory Symptoms in COPD (E-RS)***

The E-RS:COPD is an 11-item PRO developed to evaluate the severity of respiratory symptoms of COPD (Leidy et al 2014a, Leidy et al 2014b). The E-RS:COPD is a subset of items from the EXACT-PRO. The E-RS:COPD was designed to be captured as part of the daily EXACT-PRO assessment. Summation of E-RS:COPD item responses produces a total score ranging from 0 to 40, with higher scores indicating greater severity. In addition to the total score, symptom domain scores can be calculated for breathlessness (5 items; score range: 0 to 17), cough and sputum (3 items; score range: 0 to 11) and chest symptoms (3 items; score range: 0 to 12) by summing the responses of items within a respective domain. As with the total score, higher domain scores indicate greater severity. Individual score decrease of at least 2 points in the E-RS:COPD total score is considered meaningful and will be used as the responder definition (Leidy et al 2014a).

*Breathlessness, Cough and Sputum Scale (BCSS)*

The BCSS is a 3-item PRO (Leidy et al 2003a, Leidy et al 2003b) that assesses the severity of breathlessness, cough, and sputum on a scale of 0 to 4. Item scores are summed to yield a total score, with higher scores indicating more severe symptoms.

*St George's Respiratory Questionnaire (SGRQ)*

The SGRQ is a 50-item PRO instrument developed to measure the health status of participants with airway obstruction diseases (Jones et al 1991, Jones and Forde 2009). The questionnaire is divided into 2 parts: Part 1 consists of 8 items pertaining to the severity of respiratory symptoms in the preceding 4 weeks; Part 2 consists of 42 items related to the daily activity and psychosocial impacts of the individual's respiratory condition. The SGRQ yields a total score and 3 component scores (symptoms, activity, and impacts). The total score indicates the impact of disease on overall health status. This total score is expressed as a percentage of overall impairment, in which 100 represents the worst possible health status and 0 indicates the best possible health status. Likewise, the component scores range from 0 to 100, with higher scores indicative of greater impairment. Individual score decrease of at least 4 points in the SGRQ total score is considered meaningful and will be used to support the responder definition. Specific details on the scoring algorithms are provided by the developer in a user manual (Jones and Forde 2009).

*COPD Assessment Test (CAT)*

The CAT is an 8-item PRO developed to measure the impact of COPD on health status (Jones et al 2009, Kon et al 2014). The instrument uses semantic differential 6-point response scales which are defined by contrasting adjectives to capture the impact of COPD. Content includes items related to cough, phlegm, chest tightness, breathlessness going up hills/stairs, activity limitation at home, confidence leaving home, sleep, and energy. Each item response ranges from 0 to 5 with 0 having the least impact and 5 having the greatest impact on health status. The CAT total score is the sum of item responses with score range from 0 to 40 with higher scores indicative of greater COPD impact on health status. Individual score decrease of at least 2 points in the CAT total score is considered meaningful and will be used to support the responder definition (Kon et al 2014).

*5-level EuroQol-5 Dimension (EQ-5D-5L)*

The EQ-5D-5L is a 5-level standardised instrument for use as a measure of health outcome. Applicable to a wide range of health conditions and treatment, it provides a simple descriptive profile and a single index value for health status. The EQ-5D-5L consists of 2 assessments, a descriptive system, and a visual analogue scale (VAS). The descriptive system comprises of the following 5 dimensions: mobility, self-care, usual activities, pain/discomfort, and anxiety/depression. Each dimension has 5 severity levels: no problems, slight problems, moderate problems, severe problems, and extreme problems. The EQ-5D-5L index score can be calculated based upon participants' responses to the 5 dimensions and using an appropriate value set, which will be further described in the statistical analysis plan (SAP).

The EQ-5D VAS records the respondent's self-rated health on a 20 cm, 0 to 100 vertical scale with endpoints labelled "the best health you can imagine" and "the worst health you can imagine", with higher scores corresponding to a better health state. This information is used as a quantitative measure of health as judged by the individual respondents.

*Work Productivity and Activity Impairment Questionnaire (WPAI-GH)*

The WPAI-GH (version 2.0) is a self-administered tool comprised of 6 questions which address absenteeism, presenteeism (reduced effectiveness while working), overall work productivity loss (absenteeism plus presenteeism), and activity impairment. This validated tool captures data from the past 7 days. The WPAI-GH outcomes are scored as impairment percentages, with a higher percentage indicating greater impairment and less productivity (Reilly et al 1993).

*Patient Global Impression of Severity (PGIS)*

The PGIS is a single item designed to capture the participant's perception of overall COPD symptom severity at the time of completion using a 6-point scale (0 - no symptoms to 5 - very severe).

*Patient Global Impression of Change (PGIC)*

The PGIC is a single item designed to capture the participant's perception in change in overall COPD symptoms since first dose of IP using a 7-point scale (1 - much better to 7 - much worse).

### Composite Endpoint for Exacerbations of COPD (COPDCompEx)

The composite endpoint for exacerbations of COPD (COPDCompEx) is an endpoint based on combining exacerbations with daily PRO defined events and study drop out (Vogelmeier et al 2020). The definitions for COPDCompEx components are as follows:

Exacerbation: episodes leading to one or more of the following: hospitalisation, emergency room visit, treatment with systemic corticosteroids, or treatment with antibiotics.
Daily PRO events: defined by threshold and slope criteria using the following PRO variables: individual items of the BCSS and rescue medication use.

*Statistical Considerations*

### Primary Endpoint

The primary endpoint is the annualised rate of moderate-to-severe exacerbations. This will be assessed for each dose of MEDI3506 vs placebo first in the primary population (former smokers) and then in the overall population of current and former smokers.

Moderate-to-severe exacerbations rate in each MEDI3506 dose regimen group will be compared with moderate-to-severe exacerbation rate in the placebo group using a negative binomial model. The response variable in the model will be the number of COPD exacerbations experienced by a participant over the full double-blind 52-week treatment period. The model will include covariates of treatment group, region, maintenance inhaled therapy (triple or dual), and the number of exacerbations in the previous year (1 vs $\geq$ 2) as categorical factors, and post-BD FEV1% predicted at screening and log screening blood eosinophil count as continuous covariates. The logarithm of the participant's corresponding follow-up time will be used as an offset variable in the model. For the analysis in the overall population, smoking status will also be included as a covariate.

The estimated treatment effect (ie, the rate ratio of each dose of MEDI3506 versus placebo), corresponding 95% confidence interval (CI), and 2-sided p-value for the rate ratio will be presented. In addition, the model adjusted exacerbation rate in each treatment group will be presented.

A course of treatment with systemic corticosteroids or antibiotics started within 7 days of finishing the previous treatment course will be considered as treatments for the same single exacerbation.

### Secondary Endpoints

Analysis for all secondary endpoints will be conducted in the primary population (former smokers). A similar analysis will be conducted in the overall population (former and current smokers).

*Time to first moderate or severe COPD Exacerbations*

Time to first moderate or severe COPD exacerbation will be analysed as a key secondary efficacy variable to the primary objective to explore the extent to which treatment with each dose of MEDI3 506 delays the time to first exacerbation compared with placebo. A Cox proportional hazard model will be fitted with the covariates of treatment group, region, maintenance inhaled therapy, the number of exacerbations in the previous year, post-BD FEV1% predicted at screening, and log screening blood eosinophil count. Hazard ratios, 95% CI and p-values will be reported as well as the proportion of participants with an event.

*St George's Respiratory Questionnaire*

Change from baseline in SGRQ total score over 52 weeks will be compared between MEDI3506 and placebo using a repeated measures linear model. The dependent variable will be the change from baseline in SGRQ total score at post-baseline protocol-specified visits up to the Week 52 visit. Treatment, visit, treatment-by-visit interaction, region, maintenance inhaled therapy and the number of exacerbations in the previous year will be fitted as categorical covariates with baseline SGRQ total score, post-BD FEV1% predicted, and log screening blood eosinophil count as continuous covariates. An unstructured variance covariance matrix will be used to model correlation within a participant.

Contrasts will be used to produce treatment effect estimates at each visit (including at Week 24 and Week 52) and over 52 weeks. This will be reported alongside the 2-sided 95% CI and p-value.

Responder analyses will be performed for SGRQ total score at Week 52. Responders are defined as participants with a ≥ 4.0 points improvement (decrease) over baseline. Participants who discontinue from the study for any reason or have missing data at Week 52 will be classified as non responders. Logistic regression will be used to compare the treatment groups with treatment, region, maintenance inhaled therapy, and the number of exacerbations in the previous year as categorical covariates and post-BD FEV1% predicted, log screening blood eosinophil count, and baseline SGRQ total score as continuous covariates. P-values and odds ratios with 95% CI will be produced for each treatment comparison.

*Change from Baseline in E-RS: COPD Total Score*

Change from baseline in E-RS:COPD total score over 52 weeks will be analysed using a similar model to change from baseline in SGRQ total score. Responder analyses for E RS: COPD total score at Week 52 based upon a ≥ 2 point improvement (decrease) from baseline will also be produced similarly to SGRQ responder analysis.

*Change from Baseline in Pre-dose FEV1*

Change from baseline in pre-dose/pre-BD FEV1 will be analysed using a similar repeated measures analysis model as for change from baseline in SGRQ score, but with treatment, visit, treatment-by-visit interaction, region, maintenance inhaled therapy and the number of exacerbations in the previous year fitted as categorical covariates, and baseline FEV1 and log screening blood eosinophil count as continuous covariates. Contrasts will be used to produce treatment effect estimates at each visit (including at Week 24 and Week 52) and over 52 weeks. This will be reported alongside the 2-sided 95% CI and p-value.

*Other Secondary Endpoints*

Time to first severe exacerbation and annualised rate of severe exacerbations will be analysed in a similar manner to moderate or severe exacerbations as described above.

Analyses of change from baseline in CAT total score and the proportion of participants with ≥ 2 point decrease (improvement) in CAT total score will be conducted using similar methods as SGRQ total score.

**References**

A number of publications are cited above in order to more fully describe and disclose the invention and the state of the art to which the invention pertains. Full citations for these references are provided below.

Adeloye D, Chua S, Lee C, Basquill C, Papana A, Theodoratou E et al. Global and regional estimates of COPD prevalence: Systematic review and meta-analysis. J Glob Health. 2015;5(2):020415.

Allinne J, Scott G, Lim WK, Birchard D, Erjefält JS, Sandén C et al. IL-33 blockade affects mediators of persistence and exacerbation in a model of chronic airway inflammation. J Allergy Clin Immunol. 2019;144(6):1624-37. e10.

Byers DE, Alexander-Brett J, Patel AC, Agapov E, Dang-Vu G, Jin X et al. Long-term IL-33-producing epithelial progenitor cells in chronic obstructive lung disease. J Clin Invest. 2013;123(9):3967-82.

Celli BR, MacNee W, Force AET. Standards for the diagnosis and treatment of patients with COPD: a summary of the ATS/ERS position paper. Eur Respir J. 2004;23(6):932-46.

Donaldson GC, Seemungal TA, Bhowmik A, Wedzicha JA. Relationship between exacerbation frequency and lung function decline in chronic obstructive pulmonary disease. Thorax. 2002;57(10):847-52.

EMA. Guideline on Clinical Investigation of Medicinal Products in the Treatment of Chronic Obstructive Pulmonary Disease 2012 [16 July 2021]. Available from: https://www.ema.europa.eu/en/documents/scientific-guideline/guideline-clinical-investigation-medicinal-products-treatment-chronic-obstructive-pulmonary-disease_en.pdf   Accessed on: 16 July 2021

GOLD. Global strategy for the diagnosis, management, and prevention of chronic obstructive pulmonary disease. 2020 Report 2020 [04 August 2020]. Available from: https://goldcopd.org/wp-content/uploads/2019/12/GOLD-2020-FINAL-ver1.2-03Dec19_WMV.pdf. Accessed on: 04 August 2020

Graham BL, Steenbruggen I, Miller MR, Barjaktarevic IZ, Cooper BG, Hall GL et al. Standardization of Spirometry 2019 Update. An Official American Thoracic Society and European Respiratory Society Technical Statement. Am J Respir Crit Care Med. 2019;200(8):e70-e88.

Guarascio AJ, Ray SM, Finch CK, Self TH. The clinical and economic burden of chronic obstructive pulmonary disease in the USA. Clinicoecon Outcomes Res. 2013;5235-45.

Hochberg Y. A sharper Bonferroni procedure for multiple tests of significance. Biometrika. 1988;75(4):800-2.

Jones PW, Quirk FH, Baveystock CM. The St George's Respiratory Questionnaire. Respir Med. 1991;85 Suppl B25-31; discussion 3-7.

Jones PW, Forde Y. St George's Respiratory Questionnaire Manual. 2009;Version 2.3.

Jones PW, Harding G, Berry P, Wiklund I, Chen WH, Kline Leidy N. Development and first validation of the COPD Assessment Test. Eur Respir J. 2009;34(3):648-54.

Jones PW, Chen WH, Wilcox TK, Sethi S, Leidy NK, Group E-PS. Characterizing and quantifying the symptomatic features of COPD exacerbations. Chest. 2011;139(6):1388-94.

Kearley J, Silver JS, Sanden C, Liu Z, Berlin AA, White N et al. Cigarette smoke silences innate lymphoid cell function and facilitates an exacerbated type I interleukin-33-dependent response to infection. Immunity. 2015;42(3):566-79.

Kemp SF, Lockey RF, Simons FE. World Allergy Organization ad hoc Committee on Epinephrine in A. Epinephrine: the drug of choice for anaphylaxis. A statement of the World Allergy Organization. Allergy. 2008;63(8):1061-70.

Kim V, Crapo J, Zhao H, Jones PW, Silverman EK, Cornelias A et al. Comparison between an alternative and the classic definition of chronic bronchitis in COPDGene. Ann Am Thorac Soc. 2015;12(3):332-9.

Kim V, Zhao H, Regan E, Han MK, Make BJ, Crapo JD et al. The St. George's Respiratory Questionnaire Definition of Chronic Bronchitis May Be a Better Predictor of COPD Exacerbations Compared With the Classic Definition. Chest. 2019;156(4):685-95.

Kon SS, Canavan JL, Jones SE, Nolan CM, Clark AL, Dickson MJ et al. Minimum clinically important difference for the COPD Assessment Test: a prospective analysis. Lancet Respir Med. 2014;2(3):195-203.

Lahousse L, Seys LJM, Joos GF, Franco OH, Stricker BH, Brusselle GG. Epidemiology and impact of chronic bronchitis in chronic obstructive pulmonary disease. Eur Respir J. 2017;50(2).

Leidy NK, Rennard SI, Schmier J, Jones MK, Goldman M. The breathlessness, cough, and sputum scale: the development of empirically based guidelines for interpretation. Chest. 2003a;124(6):2182-91.

Leidy NK, Schmier JK, Jones MK, Lloyd J, Rocchiccioli K. Evaluating symptoms in chronic obstructive pulmonary disease: validation of the Breathlessness, Cough and Sputum Scale. Respir Med. 2003b;97 Suppl AS59-70.

Leidy NK, Wilcox TK, Jones PW, Roberts L, Powers JH, Sethi S et al. Standardizing measurement of chronic obstructive pulmonary disease exacerbations. Reliability and validity of a patient-reported diary. Am J Respir Crit Care Med. 2011;183(3):323-9.

Leidy NK, Murray LT, Monz BU, Nelsen L, Goldman M, Jones PW et al. Measuring respiratory symptoms of COPD: performance of the EXACT- Respiratory Symptoms Tool (E-RS) in three clinical trials. RespirRes. 2014a; 15124.

Leidy NK, Sexton CC, Jones PW, Notte SM, Monz BU, Nelsen L et al. Measuring respiratory symptoms in clinical trials of COPD: reliability and validity of a daily diary. Thorax. 2014b;69(5):443-9.

Leuppi JD, Schuetz P, Bingisser R, Bodmer M, Briel M, Drescher T et al. Short-term vs conventional glucocorticoid therapy in acute exacerbations of chronic obstructive pulmonary disease: the REDUCE randomized clinical trial. JAMA. 2013;309(21):2223-31.

Mannino DM, Braman S. The epidemiology and economics of chronic obstructive pulmonary disease. Proc Am Thorac Soc. 2007;4(7):502-6.

Miravitlles M, Ribera A. Understanding the impact of symptoms on the burden of COPD. Respir Res. 2017;18(1):67.

Müllerová H, Maskell J, Meeraus WH, Galkin D, Albers FC, Gait C. Characterization of COPD Patients Treated with Inhaled Triple Therapy Containing Inhaled Corticosteroids [ICS], Long-Acting Beta2-Agonists [LABA], and Long-Acting Muscarinic Antagonists [LAMA] in the UK. Am J Respir Crit Care Med. 2017;195:A4986.

Mütze T, Glimm E, Schmidli H, Friede T. Group sequential designs with robust semiparametric recurrent event models. Stat Methods Med Res. 2019;28(8):2385-403.

Okajima Y, Come CE, Nardelli P, Sonavane SK, Yen A, Nath HP et al. Luminal Plugging on Chest CT Scan: Association With Lung Function, Quality of Life, and COPD Clinical Phenotypes. Chest. 2020;158(1):121-30.

Parikh RH, Seliger SL, Christenson R, Gottdiener JS, Psaty BM, deFilippi CR. Soluble ST2 for Prediction of Heart Failure and Cardiovascular Death in an Elderly, Community-Dwelling Population. J Am Heart Assoc. 2016;5(8).

Quanjer PH, Stanojevic S, Cole TJ, Baur X, Hall GL, Culver BH et al. Multi-ethnic reference values for spirometry for the 3-95-yr age range: the global lung function 2012 equations. Eur Respir J. 2012;40(6): 1324-43.

Rabe KF, Martinez FJ, Ferguson GT, Wang C, Singh D, Wedzicha JA et al. Triple Inhaled Therapy at Two Glucocorticoid Doses in Moderate-to-Very-Severe COPD. N Engl J Med. 2020;383(1):35-48.

Rabe KF, Celli BR, Wechsler ME, Abdulai RM, Luo X, Boomsma MM et al. Safety and efficacy of itepekimab in patients with moderate-to-severe COPD: a genetic association study and randomised, double-blind, phase 2a trial. Lancet Respir Med. 2021.

Radicioni G, Ceppe A, Ford AA, Alexis NE, Barr RG, Bleecker ER et al. Airway mucin MUC5AC and MUC5B concentrations and the initiation and progression of chronic obstructive pulmonary disease: an analysis of the SPIROMICS cohort. Lancet Respir Med. 2021.

Reilly MC, Zbrozek AS, Dukes EM. The validity and reproducibility of a work productivity and activity impairment instrument. Pharmacoeconomics. 1993;4(5):353-65.

Ritchie AI, Wedzicha JA. Definition, Causes, Pathogenesis, and Consequences of Chronic Obstructive Pulmonary Disease Exacerbations. Clin Chest Med. 2020;41(3):421-38.

Sampson HA, Munoz-Furlong A, Campbell RL, Adkinson NF, Jr., Bock SA, Branum A et al. Second symposium on the definition and management of anaphylaxis: summary report--Second National Institute of Allergy and Infectious Disease/Food Allergy and Anaphylaxis Network symposium. J Allergy Clin Immunol. 2006;117(2):391-7.

Schmitz J, Owyang A, Oldham E, Song Y, Murphy E, McClanahan TK et al. IL-33, an interleukin-1-like cytokine that signals via the IL-1 receptor-related protein ST2 and induces T helper type 2-associated cytokines. Immunity. 2005;23(5):479-90.

Seemungal TA, Donaldson GC, Paul EA, Bestall JC, Jeffries DJ, Wedzicha JA. Effect of exacerbation on quality of life in patients with chronic obstructive pulmonary disease. Am J Respir Crit Care Med. 1998;157(5 Pt 1):1418-22.

Smith D, Helgason H, Sulem P, Bjomsdottir US, Lim AC, Sveinbjomsson G et al. A rare IL33 loss-of-function mutation reduces blood eosinophil counts and protects from asthma. PLoS Genet. 2017;13(3):el006659.

Soler-Cataluna JJ, Martinez-Garcia MA, Roman Sanchez P, Salcedo E, Navarro M, Ochando R. Severe acute exacerbations and mortality in patients with chronic obstructive pulmonary disease. Thorax. 2005;60(11):925-31.

Stanczak MA, Sanin DE, Apostolova P, Nerz G, Lampaki D, Hofmann M et al. IL-33 expression in response to SARS-CoV-2 correlates with seropositivity in COVID-19 convalescent individuals. Nat Commun. 2021;12(1):2133.

Stone A. The application of bespoke spending functions in group-sequential designs and the effect of delayed treatment switching in survival trials. Pharm Stat. 2010;9(2): 151-61.

Stott-Miller M, Mullerova H, Miller B, Tabberer M, El Baou C, Keeley T et al. Defining Chronic Mucus Hypersecretion Using the CAT in the SPIROMICS Cohort. Int J Chron Obstruct Pulmon Dis. 2020;152467-76.

Toy EL, Gallagher KF, Stanley EL, Swensen AR, Duh MS. The economic impact of exacerbations of chronic obstructive pulmonary disease and exacerbation definition: a review. COPD. 2010;7(3):214-28.

van Manen JG, Bindels PJ, Dekker FW, Bottema BJ, van der Zee JS, Ijzermans CJ et al. The influence of COPD on health-related quality of life independent of the influence of comorbidity. J clin epidemiol. 2003;56(12):1177-84.

Vestbo J, Papi A, Corradi M, Blazhko V, Montagna I, Francisco C et al. Single inhaler extrafine triple therapy versus long-acting muscarinic antagonist therapy for chronic obstructive pulmonary disease (TRINITY): a double-blind, parallel group, randomised controlled trial. Lancet. 2017;389(10082):1919-29.

Vogelmeier CF, Fuhlbrigge A, Jauhiainen A, Scheepers L, Bengtsson T, Peterson S et al. COPDCompEx: A novel composite endpoint for COPD exacerbations to enable faster clinical development. Respir Med. 2020;173106175.

Wedzicha JA, Seemungal TA. COPD exacerbations: defining their cause and prevention. Lancet. 2007;370(9589):786-96.

Corhay JL, Vincken W, Schlesser M, Bossuyt P and Imschoot J. Chronic bronchitis in COPD patients is associated with increased risk of exacerbations: a cross-sectional multicentre study. Int J Clin Pract 2013;67(12):1294-301.

Crawford B, Monz B, Hohlfeld J, Roche N, Rubin B, Magnussen H, et al. Development and validation of a cough and sputum assessment questionnaire. Respir Med2008;102(11):1545-55.

de Oca MM, Halbert RJ, Lopez MV, Perez-Padilla R, Tálamo C, Moreno D, et al. The chronic bronchitis phenotype in subjects with and without COPD: the PLATINO study. Eur Respir J 2012;40(1):28-36.

Jones PW and Forde Y. St George's respiratory questionnaire manual. 2009. Version 2.3.

Kim V, Han MK, Vance GB, Make BJ, Newell JD, Hokanson JE, et al. The chronic bronchitic phenotype of COPD: an analysis of the COPDGene Study. Chest 2011;140(3):626-33.

Kim V and Criner GJ. Chronic bronchitis and chronic obstructive pulmonary disease. Am J Respir Crit Care Med 2013;187(3):228-37.

Martinez FJ, Calverley PM, Goehring U-M, Brose M, Fabbri LM, Rabe KF. Effect of roflumilast on exacerbations in patients with severe chronic obstructive pulmonary disease uncontrolled by combination therapy (REACT): a multicentre randomised controlled trial. Lancet 2015;358(9971):857-66.

Monz BU, Sachs P, McDonald J, Crawford B, Nivens MC and Tetzlaff K. Responsiveness of the cough and sputum assessment questionnaire in exacerbations of COPD and chronic bronchitis. Respir Med 2010;104(4):534-41.

NIH. Guidelines for the prevention and treatment of opportunistic infections in adults and adolescents with HIV. Available from URL: https://aidsinfo.nih.gov/contentfiles/lvguidelines/adult_oi.pdf. Published 2019. Accessed 17Mar2020.

Smith J, Owen E, Earis J and Woodcock A. Cough in COPD: correlation of objective monitoring with cough challenge and subjective assessments. Chest 2006;130(2):379-85.

Woodruff PG, Barr RG, Bleecker E, Christenson SA, Couper D, Curtis JL, et al. Clinical Significance of Symptoms in Smokers with Preserved Pulmonary Function. N Engl J Med 2016;374(19): 1811-21.

For standard molecular biology techniques, see Sambrook, J., Russel, D.W. Molecular Cloning, A Laboratory Manual. 3 ed. 2001, Cold Spring Harbor, New York: Cold Spring Harbor Laboratory Press

**Claims**

1. An IL-33 antibody or an antibody variant thereof for use in a method of treating chronic obstructive pulmonary disease (COPD) in a subject, wherein the method comprises administering a therapeutically effective amount of an anti-IL-33 antibody or antibody variant thereof in a dose of about 300 mg at an interval of every 2 weeks (Q2W), 4 weeks (Q4W) or 8 weeks (Q8W), or in a dose of about 600 mg at an interval of every 4 weeks (Q4W), wherein the anti-IL-33 antibody or variant thereof comprises a VH domain having the sequence set forth in SEQ ID NO: 4 and a VL domain having the sequence set forth in SEQ ID NO: 8.

2. The anti-IL-33 antibody or antibody variant thereof for use according to any preceding claim, wherein the COPD is associated with chronic bronchitis in the subject.

3. The anti-IL-33 antibody or antibody variant thereof for use of any preceding claim, wherein the COPD is moderate COPD, moderate-to-severe COPD or severe COPD.

4. The anti-IL-33 antibody or antibody variant thereof for use of any preceding claim, wherein the subject has a history of at least one or at least two moderate, or at least one severe, acute exacerbations of COPD (aeCOPD) in the 12 months prior to treatment.

5. The anti-IL-33 antibody or antibody variant thereof for use of any preceding claim, wherein, prior to treatment, the subject has a post bronchodilator forced expiratory volume in 1 second ($FEV_1$) to forced vital capacity (FVC) ratio (post-BD-$FEV_1$/FVC) of less than (<) 0.70 and/or a post-BD FEV1 > 20% of predicted normal value.

6. The anti-IL-33 antibody or antibody variant thereof for use of any preceding claim, wherein the subject is a current smoker or a former smoker and optionally wherein the subject has a smoking history of at least 10 pack-years.

7. The anti-IL-33 antibody or antibody variant thereof for use of any preceding claim, wherein the subject is receiving COPD inhaled maintenance therapy comprising a long acting Beta 2 agonist (LABA), a long activating muscarinic receptor antagonist (LAMA), and/or a inhaled corticosteroid (ICS) and wherein the inhaled maintenance therapy optionally comprises LABA and LAMA, ICS and LABA, or ICS, LABA and LAMA.

8. The anti-IL-33 antibody or antibody variant thereof for use of any preceding claim, wherein the annualised rate of moderate to severe or severe COPD exacerbations is reduced in the subject and/or the time to first moderate to severe or severe COPD exacerbation is increased.

9. The anti-IL-33 antibody or antibody variant thereof for use of any preceding claim, wherein the $FEV_1$, pre-bronchodilator $FEV_1$ and/or $FEV_1$ to Forced Vital Capacity (FVC) ratio (FEVi/FVC), is improved in the subject.

10. The anti-IL-33 antibody or antibody variant thereof for use of any preceding claim, wherein the score is improved in the subject in one or more questionnaires selected from Evaluating Respiratory Symptoms in COPD (E-RS), St George's Respiratory Questionnaire (SGRQ), COPD Assessment Test (CAT), Exacerbations of Chronic Pulmonary Disease Tool-Patient-reported Outcome (EXACT-PRO), Breathlessness, Cough and Sputum Scale (BCSS), 5-level EuroQol-5 Dimension (EQ-5D-5L), Work Productivity and Activity Impairment Questionnaire (WPAI-GH), Patient Global Impression of Severity (PGIS) or Patient Global Impression of Change (PGIC).

11. The anti-IL-33 antibody or antibody variant thereof for use of claim 10, wherein the method achieves the minimum clinically important difference in E-RS: COPD score, SGRQ score and/or CAT score.

12. The anti-IL-33 antibody or antibody variant thereof for use of any preceding claim, wherein the dose is effective to achieve a $C_{max.ss}$ of from about 10 to 35 $\mu$g/ml during the dosing period.

13. The anti-IL-33 antibody or antibody variant thereof for use of any preceding claim, wherein the anti-IL-33 antibody or antibody variant thereof is selected from: human antibody, a humanized antibody, a chimeric antibody, a monoclonal antibody, a recombinant antibody, an antigen-binding antibody fragment, a single chain antibody, a monomeric antibody, a diabody, a triabody, tetrabody, a Fab fragment, an IgG1 antibody, an IgG2 antibody, an IgG3 antibody, and an IgG4 antibody.

14. The IL-33 antibody or antibody variant thereof for use of any preceding claim, wherein the anti-IL-33 antibody or antibody variant thereof is an IgG1.

15. The IL-33 antibody or antibody variant thereof for use of any preceding claim, wherein the anti-IL-33 antibody comprises a light chain sequence as set forth in SEQ ID NO:9 and a heavy chain sequence as set forth in SEQ ID NO: 10.

16. The anti-IL-33 antibody or antibody variant thereof for use of any preceding claim, wherein the anti-IL-33 antibody is 33-670087_7B (MEDI3506).

17. The anti-IL-33 antibody or antibody variant thereof for use according to any preceding claim, wherein the administration is subcutaneous.

**Patentansprüche**

1. IL-33-Antikörper oder Antikörpervariante davon zur Verwendung in einem Verfahren zur Behandlung einer chronisch obstruktiven Lungenerkrankung (COPD) bei einem Subjekt, wobei das Verfahren das Verabreichen einer therapeutisch wirksamen Menge eines Anti-IL-33-Antikörpers oder einer Antikörpervariante davon in einer Dosis von etwa 300 mg in einem Intervall von jeweils 2 Wochen (Q2W), 4 Wochen (Q4W) oder 8 Wochen (Q8W) oder in einer Dosis von etwa 600 mg in einem Intervall von jeweils 4 Wochen (Q4W) umfasst, wobei der Anti-IL-33-Antikörper oder die Variante davon eine VH-Domäne mit der in SEQ ID NO: 4 dargelegten Sequenz und eine VL-Domäne mit der in SEQ ID NO: 8 dargelegten Sequenz umfasst.

2. Anti-IL-33-Antikörper oder Antiköurpervariante davon zur Verwendung nach einem der vorhergehenden Ansprüche, wobei die COPD mit einer chronischen Bronchitis des Subjekts verbunden ist.

3. Anti-IL-33-Antikörper oder Antikörpervariante davon zur Verwendung nach einem der vorhergehenden Ansprüche, wobei es sich bei der COPD um eine mittelschwere COPD, um eine mittelschwere bis schwere COPD oder um eine schwere COPD handelt.

4. Anti-IL-33-Antikörper oder Antikörpervariante davon zur Verwendung nach einem der vorhergehenden Ansprüche, wobei das Subjekt in den 12 Monaten vor der Behandlung mindestens eine oder mindestens zwei mittelschwere oder mindestens eine schwere akute COPD-Exazerbation (aeCOPD) in der Anamnese hatte.

5. Anti-IL-33-Antikörper oder Antikörpervariante davon zur Verwendung nach einem der vorhergehenden Ansprüche, wobei das Subjekt vor der Behandlung nach der Gabe eines Bronchodilatators ein Verhältnis von forciertem exspiratorischem Volumen in 1 Sekunde ($FEV_1$) zu der forcierten Vitalkapazität (FVC) (post-BD-$FEV_1$/FVC) von weniger als (<) 0,70 und/oder ein post-BD-$FEV_1$ von > 20 % des vorhergesagten Normalwerts aufweist.

6. Anti-IL-33-Antikörper oder Antikörpervariante davon zur Verwendung nach einem der vorhergehenden Ansprüche, wobei das Subjekt ein gegenwärtiger oder ehemaliger Raucher ist und wobei das Subjekt optional eine Raucherhistorie von mindestens 10 Packungsjahren aufweist.

7. Anti-IL-33-Antikörper oder Antikörpervariante davon zur Verwendung nach einem der vorhergehenden Ansprüche, wobei das Subjekt eine inhalative Erhaltungstherapie bei COPD erhält, die einen lang wirkenden Beta-2-Agonisten (LABA), einen lang wirkenden Muskarinrezeptor-Antagonisten (LAMA) und/oder ein inhaliertes Kortikosteroid (ICS) umfasst, und wobei die inhalative Erhaltungstherapie optional LABA und LAMA, ICS und LABA oder ICS, LABA und LAMA umfasst.

8. Anti-IL-33-Antikörper oder Antikörpervariante davon zur Verwendung nach einem der vorhergehenden Ansprüche, wobei die jährliche Rate mittelschwerer bis schwerer oder schwerer COPD-Exazerbationen beim Patienten verringert ist und/oder die Zeit bis zu der ersten mittelschweren bis schweren oder schweren COPD-Exazerbation verlängert ist.

9. Anti-IL-33-Antikörper oder Antikörpervariante davon zur Verwendung nach einem der vorhergehenden Ansprüche, wobei das $FEV_1$, das $FEV_1$ vor der Anwendung des Bronchodilatators und/oder das Verhältnis von $FEV_1$ zu der forcierten Vitalkapazität (FVC) ($FEV_1$/FVC) beim Patienten verbessert ist.

10. Anti-IL-33-Antikörper oder Antikörpervariante davon zur Verwendung nach einem der vorhergehenden Ansprüche, wobei sich der Score des Subjekts in einem oder mehreren Fragebögen verbessert, ausgewählt aus Evaluating Respiratory Symptoms in COPD (E-RS), St George's Respiratory Questionnaire (SGRQ), COPD Assessment Test (CAT), Exacerbations of Chronic Pulmonary Disease Tool - Patient-reported Outcome (EXACT-PRO), Breathlessness, Cough and Sputum Scale (BCSS), 5-level EuroQol-5 Dimension (EQ-5D-5L), Work Productivity and Activity Impairment Questionnaire (WPAI-GH), Patient Global Impression of Severity (PGIS) oder Patient Global Impression of Change (PGIC).

11. Anti-IL-33-Antikörper oder Antikörpervariante davon zur Verwendung nach Anspruch 10, wobei das Verfahren den minimalen klinisch bedeutsamen Unterschied in E-RS: COPD-Score, SGRQ-Score und/oder CAT-Score erzielt.

12. Anti-IL-33-Antikörper oder Antikörpervariante davon zur Verwendung nach einem der vorhergehenden Ansprüche, wobei die Dosis dazu ausreicht, während des Dosierungszeitraums eine $C_{max.ss}$ von etwa 10 bis 35 $\mu$g/ml zu erzielen.

13. Anti-IL-33-Antikörper oder Antikörpervariante davon zur Verwendung nach einem der vorhergehenden Ansprüche, wobei der Anti-IL-33-Antikörper oder die Antikörpervariante davon ausgewählt ist aus: einem menschlichen Antikörper, einem humanisierten Antikörper, einem chimären Antikörper, einem monoklonalen Antikörper, einem rekombinanten Antikörper, einem antigenbindenden Antikörperfragment, einem Einzelkettenantikörper, einem monomeren Antikörper, einem Diabody, einem Triabody, einem Tetrabody, einem Fab-Fragment, einem IgG1-Antikörper, einem IgG2-Antikörper, einem IgG3-Antikörper und einem IgG4-Antikörper.

14. IL-33-Antikörper oder Antikörpervariante davon zur Verwendung nach einem der vorhergehenden Ansprüche, wobei der Anti-IL-33-Antikörper oder die Antikörpervariante davon ein IgG1 ist.

15. IL-33-Antikörper oder Antikörpervariante davon zur Verwendung nach einem der vorhergehenden Ansprüche, wobei der Anti-IL-33-Antikörper eine Leichtkettensequenz gemäß SEQ ID NO: 9 und eine Schwerkettensequenz gemäß SEQ ID NO: 10 umfasst.

16. Anti-IL-33-Antikörper oder Antikörpervariante davon zur Verwendung nach einem der vorhergehenden Ansprüche, wobei der Anti-IL-33-Antikörper 33-670087_7B (MEDI3506) ist.

17. Anti-IL-33-Antikörper oder Antikörpervariante davon zur Verwendung nach einem der vorhergehenden Ansprüche, wobei die Verabreichung subkutan erfolgt.


## Revendications

1. Anticorps d'IL-33 ou variant d'anticorps correspondant pour une utilisation dans un procédé de traitement d'une maladie pulmonaire obstructive chronique (COPD) chez un sujet, le procédé comprenant une administration d'une quantité thérapeutiquement efficace d'un anticorps anti-IL-33 ou d'un variant d'anticorps correspondant en une dose d'environ 300 mg à un intervalle de toutes les 2 semaines (Q2W), 4 semaines (Q4W) ou 8 semaines (Q8W), ou en une dose d'environ 600 mg à un intervalle de toutes les 4 semaines (Q4W), l'anticorps anti-IL-33 ou le variant correspondant comprenant un domaine VH ayant la séquence indiquée dans la SEQ ID NO: 4 et un domaine VL ayant la séquence indiquée dans la SEQ ID NO: 8.

2. Anticorps anti-IL-33 ou variant d'anticorps correspondant pour une utilisation selon une quelconque revendication précédente, la COPD étant associée à une bronchite chronique chez le sujet.

3. Anticorps anti-IL-33 ou variant d'anticorps correspondant pour une utilisation selon une quelconque revendication précédente, la COPD étant une COPD modérée, une COPD modérée à grave ou une COPD grave.

4. Anticorps anti-IL-33 ou variant d'anticorps correspondant pour une utilisation selon une quelconque revendication précédente, le sujet ayant un antécédent d'au moins une ou d'au moins deux exacerbations modérées, ou d'au moins une exacerbation grave, aiguë(s) de COPD (aeCOPD) dans les 12 mois avant le traitement.

5. Anticorps anti-IL-33 ou variant d'anticorps correspondant pour une utilisation selon une quelconque revendication précédente, avant le traitement, le sujet ayant un rapport de volume expiratoire forcé en 1 seconde ($FEV_1$) post-bronchodilatateur sur capacité vitale forcée (FVC) **(post-BD-$FEV_1$/FVC)** inférieur à (<) 0,70 et/ou un post-BD FEV1 > 20 % d'une valeur normale prédite.

**6.** Anticorps anti-IL-33 ou variant d'anticorps correspondant pour une utilisation selon une quelconque revendication précédente, le sujet étant un fumeur actuel ou un ancien fumeur et éventuellement, le sujet ayant un antécédent de tabagisme de 10 paquets-années.

**7.** Anticorps anti-IL-33 ou variant d'anticorps correspondant pour une utilisation selon une quelconque revendication précédente, le sujet recevant une thérapie de maintenance inhalée de COPD comprenant un agoniste de bêta 2 à longue durée d'action (LABA), un antagoniste de récepteur muscarinique à longue durée d'activation (LAMA) et/ou un corticostéroïde inhalé (ICS) et la thérapie de maintenance inhalée comprenant éventuellement LABA et LAMA, ICS et LABA, ou ICS, LABA et LAMA.

**8.** Anticorps anti-IL-33 ou variant d'anticorps correspondant pour une utilisation selon une quelconque revendication précédente, le taux annualisé d'exacerbations de COPD modérée à grave ou grave étant réduit chez le sujet et/ou la durée jusqu'à la première exacerbation de COPD modérée à grave ou grave étant augmentée.

**9.** Anticorps anti-IL-33 ou variant d'anticorps correspondant pour une utilisation selon une quelconque revendication précédente, le $FEV_1$, le $FEV_1$ pré-bronchodilatateur et/ou le rapport de $FEV_1$ sur capacité vitale forcée (FVC) ($FEV_1$/FVC), étant amélioré(s) chez le sujet.

**10.** Anticorps anti-IL-33 ou variant d'anticorps correspondant pour une utilisation selon une quelconque revendication précédente, le score d'un ou plusieurs questionnaires choisis parmi l'évaluation des symptômes respiratoires de la COPD (E-RS), le questionnaire respiratoire de St George (SGRQ), le test d'évaluation de la COPD (CAT), les résultats rapportés par le patient de l'outil d'exacerbations de la maladie pulmonaire chronique(EXACT-PRO), l'échelle d'essoufflement, de toux et d'expectorations (BCSS), la dimension EuroQol-5 à 5 niveaux (EQ-5D-5L), le questionnaire sur la productivité au travail et les déficiences d'activité (WPAI-GH), l'impression globale de gravité du patient (PGIS) et l'impression globale de changement du patient (PGIC) étant amélioré chez le sujet.

**11.** Anticorps anti-IL-33 ou variant d'anticorps correspondant pour une utilisation selon la revendication 10, le procédé atteignant la différence importante cliniquement minimale dans E-RS : score de COPD, score de SGRQ et/ou score de CAT.

**12.** Anticorps anti-IL-33 ou variant d'anticorps correspondant pour une utilisation selon une quelconque revendication précédente, la dose étant efficace pour atteindre un $C_{max.ss}$ allant d'environ 10 à 35 $\mu$g/ml pendant la période de dosage.

**13.** Anticorps anti-IL-33 ou variant d'anticorps correspondant pour une utilisation selon une quelconque revendication précédente, l'anticorps anti-IL-33 ou le variant d'anticorps correspondant étant choisi parmi : un anticorps humain, un anticorps humanisé, un anticorps chimérique, un anticorps monoclonal, un anticorps recombinant, un fragment d'anticorps de liaison à un antigène, un anticorps monocaténaire, un anticorps monomérique, un dianticorps, un trianticorps, un tétraanticorps, un fragment Fab, un anticorps IgG1, un anticorps dIgG2, un anticorps IgG3 et un anticorps IgG4.

**14.** Anticorps d'IL-33 ou variant d'anticorps correspondant pour une utilisation selon une quelconque revendication précédente, l'anticorps anti-IL-33 ou le variant d'anticorps correspondant étant une IgG1.

**15.** Anticorps d'IL-33 ou variant d'anticorps correspondant pour une utilisation selon une quelconque revendication précédente, l'anticorps anti-IL-33 comprenant une séquence de chaîne légère telle qu'indiquée dans la SEQ ID NO: 9 et une séquence de chaîne lourde telle qu'indiquée dans la SEQ ID NO: 10.

**16.** Anticorps anti-IL-33 ou variant d'anticorps correspondant pour une utilisation selon une quelconque revendication précédente, l'anticorps anti-IL-33 étant **33-670087_7B (MEDI3506).**

**17.** Anticorps anti-IL-33 ou variant d'anticorps correspondant pour une utilisation selon une quelconque revendication précédente, l'administration étant sous-cutanée.

# FIG. 1A

# FIG. 1B

# FIG. 1C

# FIG. 1D

FIG. 2A

FIG. 2B

FIG. 2C

# FIG. 3

# FIG. 4A

# FIG. 4B

# FIG. 4C

# FIG. 5

# FIG. 6

# FIG. 7A

Normal human bronchial epithelial cells-24h

# FIG. 7B

# FIG. 8

Healthy    COPD    COPD +
                   MEDI3506

0 hours

Cells

Scratch

24 hours

Submerged culture

# FIG. 9

A549 cell (72hrs)

● MEDI3506
■ Anti-TSLP

# FIG. 10

FIG. 11

# FIG. 11 Cont'd

Prediction of mAb concentration

—•— Observation
—○— Estimation

EP 4 244 252 B1

Dose=30 mg MAD

Dose=300 mg MAD

Dose=300 mg IV

# FIG. 12

Prediction of IL33/MEDI3506 complex

Observation ●
Estimation ○

Dose=1 mg SAD

Dose=3 mg SAD

Dose=10 mg SAD

Dose=30 mg SAD

Dose=100 mg SAD

Dose=300 mg SAD

# FIG. 12 Cont'd

Prediction of IL33/MEDI3506 complex

— Observation
— Estimation

EP 4 244 252 B1

# FIG. 13

Prediction of sST2/IL33 complex

—●— Observation
—○— Estimation

# FIG. 13 Cont'd

Prediction of sST2/IL33 complex

Observation
Estimation

Dose=30 mg MAD

Dose=300 mg MAD

Dose=300 mg IV

# FIG. 14

# FIG. 15

# FIG. 16

# FIG. 17

FIG. 18

FIG. 19

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2021089563 A
- US 20210000949 A
- US 4816567 A
- US 5585089 A
- US 5693762 A
- US 9605928 W
- US 9306926 W
- US 5545807 A
- US 91245 W
- GB 8901207 W
- EP 54607381 A
- EP 546073 A1
- WO 2016156440 A

### Non-patent literature cited in the description

- Minimum Clinically Important Difference'') was established as clinically relevant after patient and clinician testing. *Jones COPD,* 2005, vol. 2 (1), 75-9
- KAKKAR et al. *Nat. Rev. Drug Disc.,* 2008, vol. 40 7, 827-840
- LINGEL et al. *Structure,* 2009, vol. 17 (10), 1398-1410
- LIU et al. *Proc. Nat. Acad. Sci.,* 2013, vol. 11 0 (37), 14918-14924
- MORRISON et al. *Proc. Natl. Acad. Sci.,* 1985, vol. 81, 6851-55
- JONES et al. *Nature,* 1986, vol. 321, 522-25
- RIECHMANN et al. *Nature,* 1998, vol. 332, 323-27
- VERHOEYEN et al. *Science,* 1988, vol. 239, 1534-36
- JAKOBOVITS et al. *Proc. Natl. Acad. Sci.,* 1993, vol. 90, 2551-55
- JAKOBOVITS et al. *Nature,* 1993, vol. 362, 255-58
- BRUGGERMANN et al. *Year in Immuno.,* 1993, vol. 7, 33
- SCOTT IC et al. *Sci Rep,* 2018, vol. 8, 3363
- COHEN E et al. *Nat Commun,* 2015, vol. 6, 8327
- MURDACA G et al. *Int J Mol Sci,* 2019, vol. 20, 5856
- ALLINNE J et al. *J Allergy Clin Immunol,* 2019, vol. 144, 1624-37.e10
- SCHMITZ J et al. *Immunity,* 2005, vol. 23, 479-90
- KOUZAKI et al. *J. Immunol.,* 2011, vol. 186, 4375-4387
- BARTEMES et al. *J Immunol,* 2012, vol. 188, 1503-1513
- ADELOYE D ; CHUA S ; LEE C ; BASQUILL C ; PAPANA A ; THEODORATOU E et al. Global and regional estimates of COPD prevalence: Systematic review and meta-analysis. *J Glob Health.,* 2015, vol. 5 (2), 020415
- ALLINNE J ; SCOTT G ; LIM WK ; BIRCHARD D ; ERJEFÄLT JS ; SANDÉN C et al. IL-33 blockade affects mediators of persistence and exacerbation in a model of chronic airway inflammation. *J Allergy Clin Immunol.,* 2019, vol. 144 (6), 1624-37. e10
- BYERS DE ; ALEXANDER-BRETT J ; PATEL AC ; AGAPOV E ; DANG-VU G ; JIN X et al. Long-term IL-33-producing epithelial progenitor cells in chronic obstructive lung disease. *J Clin Invest.,* 2013, vol. 123 (9), 3967-82
- CELLI BR ; MACNEE W ; FORCE AET. Standards for the diagnosis and treatment of patients with COPD: a summary of the ATS/ERS position paper. *Eur Respir J.,* 2004, vol. 23 (6), 932-46
- DONALDSON GC ; SEEMUNGAL TA ; BHOWMIK A ; WEDZICHA JA. Relationship between exacerbation frequency and lung function decline in chronic obstructive pulmonary disease. *Thorax,* 2002, vol. 57 (10), 847-52
- *Guideline on Clinical Investigation of Medicinal Products in the Treatment of Chronic Obstructive Pulmonary Disease 2012,* 16 July 2021, https://www.ema.europa.eu/en/documents/scientific-guideline/guideline-clinical-investigation-medicinal-products-treatment-chronic-obstructive-pulmonary-disease_en.pdf
- *Global strategy for the diagnosis, management, and prevention of chronic obstructive pulmonary disease. 2020 Report 2020,* 04 August 2020, https://goldcopd.org/wp-content/uploads/2019/12/GOLD-2020-FINAL-ver1.2-03Dec19_WMV.pdf

- **GRAHAM BL ; STEENBRUGGEN I ; MILLER MR ; BARJAKTAREVIC IZ ; COOPER BG ; HALL GL et al.** Standardization of Spirometry 2019 Update. An Official American Thoracic Society and European Respiratory Society Technical Statement. *Am J Respir Crit Care Med.,* 2019, vol. 200 (8), e70-e88
- **GUARASCIO AJ ; RAY SM ; FINCH CK ; SELF TH.** The clinical and economic burden of chronic obstructive pulmonary disease in the USA. *Clinicoecon Outcomes Res.,* 2013, 5235-45
- **HOCHBERG Y.** A sharper Bonferroni procedure for multiple tests of significance. *Biometrika,* 1988, vol. 75 (4), 800-2
- **JONES PW ; QUIRK FH ; BAVEYSTOCK CM.** The St George's Respiratory Questionnaire. *Respir Med.,* 1991, vol. 85 (B25-31), 3-7
- **JONES PW ; FORDE Y.** St George's Respiratory Questionnaire Manual. 2009
- **JONES PW ; HARDING G ; BERRY P ; WIKLUND I ; CHEN WH ; KLINE LEIDY N.** Development and first validation of the COPD Assessment Test. *Eur Respir J.,* 2009, vol. 34 (3), 648-54
- **JONES PW ; CHEN WH ; WILCOX TK ; SETHI S ; LEIDY NK.** Characterizing and quantifying the symptomatic features of COPD exacerbations. *Chest,* 2011, vol. 139 (6), 1388-94
- **KEARLEY J ; SILVER JS ; SANDEN C ; LIU Z ; BERLIN AA ; WHITE N et al.** Cigarette smoke silences innate lymphoid cell function and facilitates an exacerbated type I interleukin-33-dependent response to infection. *Immunity,* 2015, vol. 42 (3), 566-79
- **KEMP SF ; LOCKEY RF ; SIMONS FE.** World Allergy Organization ad hoc Committee on Epinephrine in A. Epinephrine: the drug of choice for anaphylaxis. A statement of the World Allergy Organization. *Allergy,* 2008, vol. 63 (8), 1061-70
- **KIM V ; CRAPO J ; ZHAO H ; JONES PW ; SILVERMAN EK ; CORNELIAS A et al.** Comparison between an alternative and the classic definition of chronic bronchitis in COPDGene. *Ann Am Thorac Soc.,* 2015, vol. 12 (3), 332-9
- **KIM V ; ZHAO H ; REGAN E ; HAN MK ; MAKE BJ ; CRAPO JD et al.** The St. George's Respiratory Questionnaire Definition of Chronic Bronchitis May Be a Better Predictor of COPD Exacerbations Compared With the Classic Definition. *Chest,* 2019, vol. 156 (4), 685-95
- **KON SS ; CANAVAN JL ; JONES SE ; NOLAN CM ; CLARK AL ; DICKSON MJ et al.** Minimum clinically important difference for the COPD Assessment Test: a prospective analysis. *Lancet Respir Med.,* 2014, vol. 2 (3), 195-203
- **LAHOUSSE L ; SEYS LJM ; JOOS GF ; FRANCO OH ; STRICKER BH ; BRUSSELLE GG.** Epidemiology and impact of chronic bronchitis in chronic obstructive pulmonary disease. *Eur Respir J.,* 2017, vol. 50 (2
- **LEIDY NK ; RENNARD SI ; SCHMIER J ; JONES MK ; GOLDMAN M.** The breathlessness, cough, and sputum scale: the development of empirically based guidelines for interpretation. *Chest,* 2003, vol. 124 (6), 2182-91
- **LEIDY NK ; SCHMIER JK ; JONES MK ; LLOYD J ; ROCCHICCIOLI K.** Evaluating symptoms in chronic obstructive pulmonary disease: validation of the Breathlessness, Cough and Sputum Scale. *Respir Med.,* 2003, vol. 97 (A), S59-70
- **LEIDY NK ; WILCOX TK ; JONES PW ; ROBERTS L ; POWERS JH ; SETHI S et al.** Standardizing measurement of chronic obstructive pulmonary disease exacerbations. Reliability and validity of a patient-reported diary. *Am J Respir Crit Care Med.,* 2011, vol. 183 (3), 323-9
- **LEIDY NK ; MURRAY LT ; MONZ BU ; NELSEN L ; GOLDMAN M ; JONES PW et al.** Measuring respiratory symptoms of COPD: performance of the EXACT- Respiratory Symptoms Tool (E-RS) in three clinical trials. *RespirRes.,* 2014, 15124
- **LEIDY NK ; SEXTON CC ; JONES PW ; NOTTE SM ; MONZ BU ; NELSEN L et al.** Measuring respiratory symptoms in clinical trials of COPD: reliability and validity of a daily diary. *Thorax,* 2014, vol. 69 (5), 443-9
- **LEUPPI JD ; SCHUETZ P ; BINGISSER R ; BODMER M ; BRIEL M ; DRESCHER T et al.** Short-term vs conventional glucocorticoid therapy in acute exacerbations of chronic obstructive pulmonary disease: the REDUCE randomized clinical trial. *JAMA,* 2013, vol. 309 (21), 2223-31
- **MANNINO DM ; BRAMAN S.** The epidemiology and economics of chronic obstructive pulmonary disease. *Proc Am Thorac Soc.,* 2007, vol. 4 (7), 502-6
- **MIRAVITLLES M ; RIBERA A.** Understanding the impact of symptoms on the burden of COPD. *Respir Res.,* 2017, vol. 18 (1), 67
- **MÜLLEROVÁ H ; MASKELL J ; MEERAUS WH ; GALKIN D ; ALBERS FC ; GAIT C.** Characterization of COPD Patients Treated with Inhaled Triple Therapy Containing Inhaled Corticosteroids [ICS], Long-Acting Beta2-Agonists [LABA], and Long-Acting Muscarinic Antagonists [LAMA] in the UK. *Am J Respir Crit Care Med.,* 2017, vol. 195, A4986
- **MÜTZE T ; GLIMM E ; SCHMIDLI H ; FRIEDE T.** Group sequential designs with robust semiparametric recurrent event models. *Stat Methods Med Res.,* 2019, vol. 28 (8), 2385-403

- **OKAJIMA Y ; COME CE ; NARDELLI P ; SONA-VANE SK ; YEN A ; NATH HP et al.** Luminal Plugging on Chest CT Scan: Association With Lung Function, Quality of Life, and COPD Clinical Phenotypes. *Chest,* 2020, vol. 158 (1), 121-30
- **PARIKH RH ; SELIGER SL ; CHRISTENSON R ; GOTTDIENER JS ; PSATY BM ; DEFILIPPI CR.** Soluble ST2 for Prediction of Heart Failure and Cardiovascular Death in an Elderly, Community-Dwelling Population. *J Am Heart Assoc.,* 2016, vol. 5 (8
- **QUANJER PH ; STANOJEVIC S ; COLE TJ ; BAUR X ; HALL GL ; CULVER BH et al.** Multi-ethnic reference values for spirometry for the 3-95-yr age range: the global lung function 2012 equations. *Eur Respir J.,* 2012, vol. 40 (6), 1324-43
- **RABE KF ; MARTINEZ FJ ; FERGUSON GT ; WANG C ; SINGH D ; WEDZICHA JA et al.** Triple Inhaled Therapy at Two Glucocorticoid Doses in Moderate-to-Very-Severe COPD. *N Engl J Med.,* 2020, vol. 383 (1), 35-48
- **RABE KF ; CELLI BR ; WECHSLER ME ; ABDULAI RM ; LUO X ; BOOMSMA MM et al.** Safety and efficacy of itepekimab in patients with moderate-to-severe COPD: a genetic association study and randomised, double-blind, phase 2a trial. *Lancet Respir Med.,* 2021
- **RADICIONI G ; CEPPE A ; FORD AA ; ALEXIS NE ; BARR RG ; BLEECKER ER et al.** Airway mucin MUC5AC and MUC5B concentrations and the initiation and progression of chronic obstructive pulmonary disease: an analysis of the SPIROMICS cohort. *Lancet Respir Med.,* 2021
- **REILLY MC ; ZBROZEK AS ; DUKES EM.** The validity and reproducibility of a work productivity and activity impairment instrument. *Pharmacoeconomics,* 1993, vol. 4 (5), 353-65
- **RITCHIE AI ; WEDZICHA JA.** Definition, Causes, Pathogenesis, and Consequences of Chronic Obstructive Pulmonary Disease Exacerbations. *Clin Chest Med.,* 2020, vol. 41 (3), 421-38
- **SAMPSON HA ; MUNOZ-FURLONG A ; CAMPBELL RL ; ADKINSON NF, JR. ; BOCK SA ; BRANUM A et al.** Second symposium on the definition and management of anaphylaxis: summary report--Second National Institute of Allergy and Infectious Disease/Food Allergy and Anaphylaxis Network symposium. *J Allergy Clin Immunol.,* 2006, vol. 117 (2), 391-7
- **SCHMITZ J ; OWYANG A ; OLDHAM E ; SONG Y ; MURPHY E ; MCCLANAHAN TK et al.** IL-33, an interleukin-1-like cytokine that signals via the IL-1 receptor-related protein ST2 and induces T helper type 2-associated cytokines. *Immunity,* 2005, vol. 23 (5), 479-90
- **SEEMUNGAL TA ; DONALDSON GC ; PAUL EA ; BESTALL JC ; JEFFRIES DJ ; WEDZICHA JA.** Effect of exacerbation on quality of life in patients with chronic obstructive pulmonary disease. *Am J Respir Crit Care Med.,* 1998, vol. 157 (5), 1418-22
- **SMITH D ; HELGASON H ; SULEM P ; BJOMS-DOTTIR US ; LIM AC ; SVEINBJOMSSON G et al.** A rare IL33 loss-of-function mutation reduces blood eosinophil counts and protects from asthma. *PLoS Genet.,* 2017, vol. 13 (3), el006659
- **SOLER-CATALUNA JJ ; MARTINEZ-GARCIA MA ; ROMAN SANCHEZ P ; SALCEDO E ; NAVARRO M ; OCHANDO R.** Severe acute exacerbations and mortality in patients with chronic obstructive pulmonary disease. *Thorax,* 2005, vol. 60 (11), 925-31
- **STANCZAK MA ; SANIN DE ; APOSTOLOVA P ; NERZ G ; LAMPAKI D ; HOFMANN M et al.** IL-33 expression in response to SARS-CoV-2 correlates with seropositivity in COVID-19 convalescent individuals. *Nat Commun.,* 2021, vol. 12 (1), 2133
- **STONE A.** The application of bespoke spending functions in group-sequential designs and the effect of delayed treatment switching in survival trials. *Pharm Stat.,* 2010, vol. 9 (2), 151-61
- **STOTT-MILLER M ; MULLEROVA H ; MILLER B ; TABBERER M ; EL BAOU C ; KEELEY T et al.** Defining Chronic Mucus Hypersecretion Using the CAT in the SPIROMICS Cohort. *Int J Chron Obstruct Pulmon Dis.,* 2020, 152467-76
- **TOY EL ; GALLAGHER KF ; STANLEY EL ; SWENSEN AR ; DUH MS.** The economic impact of exacerbations of chronic obstructive pulmonary disease and exacerbation definition: a review. *COPD,* 2010, vol. 7 (3), 214-28
- **VAN MANEN JG ; BINDELS PJ ; DEKKER FW ; BOTTEMA BJ ; VAN DER ZEE JS ; IJZERMANS CJ et al.** The influence of COPD on health-related quality of life independent of the influence of comorbidity. *J clin epidemiol.,* 2003, vol. 56 (12), 1177-84
- **VESTBO J ; PAPI A ; CORRADI M ; BLAZHKO V ; MONTAGNA I ; FRANCISCO C et al.** Single inhaler extrafine triple therapy versus long-acting muscarinic antagonist therapy for chronic obstructive pulmonary disease (TRINITY): a double-blind, parallel group, randomised controlled trial. *Lancet,* 2017, vol. 389 (10082), 1919-29
- **VOGELMEIER CF ; FUHLBRIGGE A ; JAUHIAINEN A ; SCHEEPERS L ; BENGTSSON T ; PETERSON S et al.** COPDCompEx: A novel composite endpoint for COPD exacerbations to enable faster clinical development. *Respir Med.,* 2020, 173106175
- **WEDZICHA JA ; SEEMUNGAL TA.** COPD exacerbations: defining their cause and prevention. *Lancet,* 2007, vol. 370 (9589), 786-96

- **CORHAY JL ; VINCKEN W ; SCHLESSER M ; BOSSUYT P ; IMSCHOOT J.** Chronic bronchitis in COPD patients is associated with increased risk of exacerbations: a cross-sectional multicentre study. *Int J Clin Pract,* 2013, vol. 67 (12), 1294-301
- **CRAWFORD B ; MONZ B ; HOHLFELD J ; ROCHE N ; RUBIN B ; MAGNUSSEN H et al.** Development and validation of a cough and sputum assessment questionnaire. *Respir Med,* 2008, vol. 102 (11), 1545-55
- **DE OCA MM ; HALBERT RJ ; LOPEZ MV ; PE-REZ-PADILLA R ; TÁLAMO C ; MORENO D et al.** The chronic bronchitis phenotype in subjects with and without COPD: the PLATINO study. *Eur Respir J,* 2012, vol. 40 (1), 28-36
- **JONES PW ; FORDE Y.** St George's respiratory questionnaire manual. 2009
- **KIM V ; HAN MK ; VANCE GB ; MAKE BJ ; NEW-ELL JD ; HOKANSON JE et al.** The chronic bronchitic phenotype of COPD: an analysis of the COP-DGene Study. *Chest,* 2011, vol. 140 (3), 626-33
- **KIM V ; CRINER GJ.** Chronic bronchitis and chronic obstructive pulmonary disease. *Am J Respir Crit Care Med,* 2013, vol. 187 (3), 228-37
- **MARTINEZ FJ ; CALVERLEY PM ; GOEHRING U-M ; BROSE M ; FABBRI LM ; RABE KF.** Effect of roflumilast on exacerbations in patients with severe chronic obstructive pulmonary disease uncontrolled by combination therapy (REACT): a multicentre randomised controlled trial. *Lancet,* 2015, vol. 358 (9971), 857-66
- **MONZ BU ; SACHS P ; MCDONALD J ; CRAW-FORD B ; NIVENS MC ; TETZLAFF K.** Responsiveness of the cough and sputum assessment questionnaire in exacerbations of COPD and chronic bronchitis. *Respir Med,* 2010, vol. 104 (4), 534-41
- *Guidelines for the prevention and treatment of opportunistic infections in adults and adolescents with HIV,* 2019, https://aidsinfo.nih.gov/contentfiles/lvguidelines/adult_oi.pdf
- **SMITH J ; OWEN E ; EARIS J ; WOODCOCK A.** Cough in COPD: correlation of objective monitoring with cough challenge and subjective assessments. *Chest,* 2006, vol. 130 (2), 379-85
- **WOODRUFF PG ; BARR RG ; BLEECKER E ; CHRISTENSON SA ; COUPER D ; CURTIS JL et al.** Clinical Significance of Symptoms in Smokers with Preserved Pulmonary Function. *N Engl J Med,* 2016, vol. 374 (19), 1811-21
- **SAMBROOK, J. ; RUSSEL, D.W.** Molecular Cloning, A Laboratory Manual. Cold Spring Harbor Laboratory Press, 2001